(19) Europäisches Patentamt
European Patent Office
Office européen des brevets

(11) EP 0 768 302 B1

(12) **EUROPÄISCHE PATENTSCHRIFT**

(45) Veröffentlichungstag und Bekanntmachung des Hinweises auf die Patenterteilung:
**06.07.2005 Patentblatt 2005/27**

(21) Anmeldenummer: **96810622.9**

(22) Anmeldetag: **20.09.1996**

(51) Int Cl.7: **C07D 213/69**, C07D 405/06, C07D 409/06, C07D 498/04, A61K 31/44, A61K 31/535 // (C07D498/04, 265:00, 221:00)

(54) **Hydroxypyridinone**

Hydroxypyridinones

Hydroxypyridinones

(84) Benannte Vertragsstaaten:
**AT BE CH DE DK ES FI FR GB GR IE IT LI LU NL PT SE**

(30) Priorität: **29.09.1995 CH 275995**

(43) Veröffentlichungstag der Anmeldung:
**16.04.1997 Patentblatt 1997/16**

(73) Patentinhaber:
- **Novartis AG**
  **4056 Basel (CH)**
  Benannte Vertragsstaaten:
  **BE CH LI DE DK ES FI FR GB GR IE IT LU NL PT SE**
- **Novartis Pharma GmbH**
  **1230 Wien (AT)**
  Benannte Vertragsstaaten:
  **AT**

(72) Erfinder: **Zbinden, Paul**
**4108 Witterswil (CH)**

(56) Entgegenhaltungen:

| | |
|---|---|
| **EP-A- 0 325 559** | **EP-A- 0 335 745** |
| **EP-A- 0 494 754** | **EP-A- 0 579 221** |
| **WO-A-94/03169** | **WO-A-96/22021** |
| **GB-A- 1 057 446** | **GB-A- 2 118 176** |

- **H. BICKEL ET AL.: HELVETICA CHIMICA ACTA, Bd. XLVI, Nr. IV, 1963, Seiten 1385-9, XP002049220**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 768 302 B1

Printed by Jouve, 75001 PARIS (FR)

## Beschreibung

Die Erfindung betrifft Verbindungen der Formel I

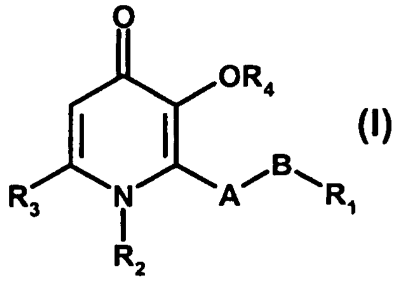

(I)

worin:

$R_1$ Wasserstoff, Halogen, Niederalkyl, Niederalkoxy, unsubstituiertes Niederalkoxycarbonyl, Amino, Niederalkylamino, Diniederalkylamino, Aminocarbonyl, N-Niederalkylaminocarbonyl, N,N-Diniederalkylaminocarbonyl, Carboxyl, unsubstituiertes Niederalkylsulfonyl, Aminosulfonyl, Cyano, Hydroxy, Nitro, Tetrazolyl, oder Niederalkylendioxy, das mit der Gruppe B ein heterocyclisches sauerstoffhaltiges Ringsystem bildet; $R_2$ Wasserstoff, unsubstituiertes oder mit Halogen, Hydroxy oder Trifluoromethyl substituiertes Niederalkyl, Niederalkylenhydroxy, Niederalkylen-niederalkoxy, unsubstituiertes Niederalkylencarboxy,unsubstituiertes Niederalkylencarbonyl-niederalkoxy, Niederalkylenamin, N-Niederalkanoylniederalkylenamin, unsubstituiertes Niederalkanoyloxyniederalkylen oder Formylniederalkylen; $R_3$ Wasserstoff, Niederalkyl, Carboxyl, Niederalkanoyloxyniederalkylen, Aminocarbonyl, N-Niederalkylaminocarbonyl oder unsubstituiertes N,N-Diniederalkylaminocarbonyl; $R_4$ Wasserstoff; A unsubstituiertes oder mit Niederalkyl, Halogen oder Hydroxy substituiertes Methylen, Carbonyl oder zusammen mit $R_2$ und den mit ihnen verbunden Atomen einen sauerstoffhaltigen heterocyclischen Ring; und B einfach oder mehrfach substituiertes oder unsubstituiertes Aryl oder einfach oder mehrfach substituiertes oder unsubstituiertes Heteroaryl; bedeuten; und deren Stereoisomere, Tautomere und Salze, insbesondere pharmazeutisch verträgliche Salze; Verfahren zur Herstellung dieser Verbindungen, pharmazeutische Präparate, die diese Verbindungen enthalten; und die Verwendung dieser Verbindungen zur Herstellung pharmazeutischer Präparate.

Aus dem Stand der Technik sind folgende Dokumente zu erwähnen:

In EP-A-0 494 754, WO 94 03169 A, EP-A-0 335 745 und EP-A-0 325 559 sind Hydroxypyridin-Derivate beschrieben, welche sich von den Verbindungen der Formel I durch die Gruppe B der Formel I unterscheiden.

WO 96 22021 A beschreibt 3-Hydroxypyridin-4-on Derivate als Protein Hydroxylierungs-Inhibitoren, welche zur Behandlung von fibrotischen oder fibroproliferativen Erkrankungen eingesetzt werden können.

Die vor- und nachstehend verwendeten Allgemeinbegriffe haben im Rahmen der vorliegenden Anmeldung vorzugsweise die folgenden Bedeutungen:

Halogen ist z. B. Chlor, Brom oder Fluor, kann aber auch Iod bedeuten.

Das Präfix "Nieder" bezeichnet einen Rest bis und mit 7 und insbesondere bis und mit 4 Kohlenstoffatomen.

Niederalkyl und Niederalkylen sind geradkettig oder verzweigt. Für sich, wie beispielsweise Niederalkyl, oder als Bestandteil anderer Gruppen, wie z. B. Niederalkoxy, Niederalkylamin, Niederalkylaminocarbonyl, Niederalkylenhydroxy, können sie unsubstituiert oder substituiert sein mit Halogen, Hydroxy, oder Trifluoromethyl, vorzugsweise sind sie unsubstituiert oder mit Hydroxy substituiert.

Methylen kann unsubstituiert oder substituiert sein mit Niederalkyl, Halogen oder Hydroxy, vorzugsweise ist es unsubstituiert oder mit Hydroxy substituiert.

Niederalkyl ist z. B. *n*-Propyl, Isopropyl, *n*-Butyl, Isobutyl, *sek*-Butyl, *tert*-Butyl, *n*-Pentyl, Neopentyl, *n*-Hexyl oder *n*-Heptyl, vorzugsweise Methyl, Ethyl und *n*-Propyl.

Niederalkoxy ist z. B. *n*-Propoxy, Isopropoxy, *n*-Butoxy, Isobutoxy, *sek*-Butoxy, *tert*-Butoxy, *n*-Amyloxy, Isoamyloxy, vorzugsweise Methoxy und Ethoxy.

Niederalkoxycarbonyl ist z. B. Niederalkyl-O-C(O)-, z. B. *n*-Propoxycarbonyl, Isopropoxycarbonyl, *n*-Butoxycarbonyl, *sek*-Butoxycarbonyl, *tert*-Butoxycarbonyl, vorzugsweise Methoxycarbonyl und Ethoxycarbonyl.

Niederalkylamino ist beispielsweise *n*-Propylamino, *n*-Butylamino, *i*-Propylamino, *i*-Butylamino, vorzugsweise Methylamino und Ethylamino

Diniederalkylamino ist beispielsweise Dimethylamino, Diethylamino, Di-*n*-Propylamino, *n*-Butylamino, Di-*n*-butylamino, *n*-Propyl-*n*-butylamino, vorzugsweise Dimethylamino, Diethylamino und Methylethylamino.

Aminocarbonyl bezeichnet den Carbamoyl-Rest

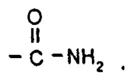

N-Niederalkylaminocarbonyl ist z. B. N-Methylcarbamoyl, N-Ethylcarbamoyl, N-n-Propylcarbamoyl, N-Isopropylcarbamoyl, vorzugsweise N-Methylcarbamoyl und N-Ethylcarbamoyl.

N,N-Diniederalkylaminocarbonyl ist beispielsweise N,N-Dimethylcarbamoyl, N,N-Diethylcarbamoyl, N-Methyl-N-ethylcarbamoyl, N,N-Di-*n*-Propylcarbamoyl, N-Methyl-N-isopropylcarbamoyl, vorzugsweise N,N-Dimethylcarbamoyl, N,N-Diethylcarbamoyl und N-Methyl-N-ethylcarbamoyl.

Niederalkylen für sich oder als Bestandteil anderer Gruppen wie z. B. Niederalkylendioxy, Niederalkylenhydroxy, Niederalkylenamin, Niederalkylencarboxy, Niederalkanoyloxyniederalkylen steht für die Gruppe $-(CH_2)_n-$, wobei n eine natürliche Zahl von und mit 1 bis und mit 7, vorzugsweise von und mit 1 bis und mit 4, ist, und ist beispielsweise Methylen, 1,2-Ethylen, 1,3-Propylen, 1,4-Butylen.

Niederalkylendioxy bezeichnet die Gruppe $-O-(CH_2)_n-O-$, wobei n eine natürliche Zahl von und mit 1 bis und mit 7 ist, und ist z. B. Methylendioxy, 1,2-Ethylendioxy, 1,3-*n*-Propylendioxy, vorzugsweise Methylendioxy und 1,2-Ethylendioxy.

Niederalkylenhydroxy steht für die Gruppe $-(CH_2)_n-OH$, wobei n eine natürliche Zahl von und mit 1 bis und mit 7, vorzugsweise von und mit 1 bis und mit 4, ist, und ist beispielsweise Methylenhydroxy, Ethylenhydroxy, Propylenhydroxy, insbesondere jedoch Ethylenhydroxy und Propylenhydroxy.

Niederalkylen-niederalkoxy ist mit Niederalkyl verethertes Niederalkylenhydroxy, z. B Methoxymethylen, Methoxyethylen, Ethoxymethylen, Ethoxyethylen, insbesondere Ethoxyethylen, während Niederalkanoyloxyniederalkylen Niederalkylenhydroxy ist, das mit Niederalkanoyl verestert ist, z. B. Acetoxyethylen, Acetoxypropylen, vorzugsweise Acetoxyethylen.

Niederalkylencarboxy steht für die Gruppe

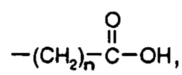

wobei n eine natürliche Zahl von und mit 1 bis und mit 7, vorzugsweise von und mit 1 bis und mit 4, ist, und ist beispielsweise Methylencarboxy, Ethylencarboxy, Propylencarboxy, Butylencarboxy, in erster Linie Methylencarboxy, Ethylencarboxy oder Propylencarboxy, während Niederalkylencarbonyl-niederalkoxy mit Niederalkyl verestertes Niederalkylencarboxy ist, z. B. Ethylencarbonsäuremethylester, Ethylencarbonsäureethylester, Propylencarbonsäuremethylester, Propylencarbonsäureethylester, Butylencarbonsäuremethylester, Butylencarbonsäureethylester, insbesondere Ethylencarbonsäureethylester, Propylencarbonsäureethylester oder Butylencarbonsäureethylester.

Niederalkylenamin steht für die Gruppe $-(CH_2)_n-NH_2$, wobei n eine natürliche Zahl von und mit 1 bis und mit 7, vorzugsweise von und mit 1 bis und mit 4, ist, und ist beispielsweise Methylenamin, Ethylenamin, Propylenamin, Butylenamin, aber auch für Reste, bei denen ein oder zwei Wasserstoffe am Stickstoff durch Niederalkyl ersetzt sind, vorzugsweise für N-Methylniederalkylenamin, N,N-Dimethylniederalkylenamin, N-Ethylniederalkylenamin, N,N-Diniederalkylenamin, N-Methyl-N-ethylniederalkylenamin.

Bei dem Rest N-Niederalkanoyl-niederalkylenamin ist einer der am Stickstoff gebundenen Wasserstoffe des Niederalkylenamins durch einen Niederalkanoyl-Rest ersetzt, z. B. Acetamidoethylen, Acetamidopropylen, insbesondere Acetamidoethylen.

Niederalkanoyl ist z. B. Acetyl, Propanoyl, Butanoyl aber auch Formyl.

Formylniederalkylen steht für die Gruppe

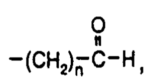

wobei n eine natürliche Zahl von und mit 1 bis und mit 7, vorzugsweise von und mit 1 bis und mit 4, ist, und ist beispielsweise Ethylenaldehyd, Propylenaldehyd, Butylenaldehyd, insbesondere Ethylenaldehyd.

Niederalkoxycarbonyl bedeutet die Gruppe

$$-\overset{\overset{\displaystyle O}{\|}}{C}-O-(CH_2)_m-CH_3,$$

wobei m eine natürliche Zahl von und mit 0 bis und mit 6, vorzugsweise von und mit 0 bis und mit 3, ist, und ist insbesondere Methyloxycarbonyl.

Sauerstoffhaltige heterocyclische Ringe sind gesattigte oder ungesättigte Ringe mit fünf bis und mit sieben Ringgliedern, von denen mindestens eines Sauerstoff ist und in denen gebenenfalls ein oder mehrere weitere Heteroatome, beispielsweise Stickstoff, vorhanden sind, so z. B. Dioxolan, Dioxan, Oxazol, Oxazin, gegebenenfalls können auch ein oder mehrere Ringkohlenstoffatome zu Carbonyl oxidiert sein, wie z. B. bei Dioxanon, Oxazolon, Oxazinon. Sie können unsubstituiert oder substituiert sein, insbesondere unsubstituiert oder substituiert mit Niederalkyl, Niederalkoxy oder Hydroxy.

Aryl ist Phenyl oder Naphthyl, das substituiert oder unsubstituiert ist. Aryl ist bevorzugt Phenyl, das unsubstituiert oder durch einen oder mehrere, insbesondere einen oder zwei, Substituenten, beispielsweise Niederalkyl, Niederalkoxy, Hydroxy, Nitro, Amino, Halogen, Trifluoromethyl, Carboxy, Amino oder Cyano, substituiert ist. In erster Linie bedeutet Aryl unsubstituiertes Phenyl oder Naphthyl, oder Phenyl, das durch Niederalkyl, Niederalkoxy, Hydroxy, Halogen oder Trifluoromethyl substituiert ist.

Heteroaryl ist ein aromatischer Rest mit 5 bis und mit 7 Ringatomen, worin mindestens eines der Ringatome ein Heteroatom ist, z. B. Pyrrolyl, Furanyl, Thiophenyl, Pyridyl, Pyranyl, Pyrimidinyl. Bevorzugt ist Azaheteroaryl, dass heisst, dass mindestens eines der Ringatome ein Stickstoffatom ist. Azaheteroaryl kann noch weitere Ringheteroatome enthalten, z. B. Stickstoff, Sauerstoff oder Schwefel; es steht z. B. Pyrrolyl, Pyridyl, Pyrimidinyl oder Pyrazinyl. Heteroaryl kann substituiert oder unsubstituiert sein. Bevorzugt ist Heteroaryl, das unsubstituiert oder durch einen oder mehrere insbesondere einen oder zwei, Substituenten Niederalkyl, Halogen oder Trifluoromethyl substituiert ist. In erster Linie bedeutet Heteroaryl unsubstituiertes Pyridyl.

Reste wie Pyrrolyl, Pyridyl, Pyrimidinyl und Pyrazinyl können über ein Ringstickstoffatom oder ein Ringkohlenstoffatom gebunden sein, Reste wie Pyridyl oder Pyrimidinyl sind vorzugsweise über ein C-Atom gebunden.

Salze von Verbindungen der Formel I sind insbesondere pharmazeutisch verwendbare Salze, vor allem Salze mit Basen, wie entsprechend Alkalimetall- oder Erdalkalimetallsalze, z. B. Natrium-, Kalium- oder Magnesiumsalze, pharmazeutisch verwendbare Uebergangsmetallsalze wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B Mono-, Di- oder Trihydroxyniederalkylaminen, Hydroxyniederalkyl-niederalkyl-aminen oder Polyhydroxyniederalkylaminen. Cyclische Amine sind z. B. Morpholin, Thiomorpholin, Piperidin oder Pyrrolidin. Als Mononiederalkylamine kommen beispielsweise Ethyl- und *tert*-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z. B. Mono-, Di- und Triethanolamin; Hydroxyniederalkyl-niederalkyl-amine sind z. B. N,N-Dimethylamino- und N,N-Diethylaminoethanol, als Polyhydroxyniederalkylamin kommt z. B. Glucosamin in Frage. In anderen Fällen können auch Säureadditionssalze, beispielsweise mit starken anorganischen Säuren, wie Mineralsäuren, z. B. Schwefelsäure, einer Phosphorsäure oder einer Halogenwasserstoffsäure, mit starken organischen Carbonsäuren, wie Niederalkancarbonsäuren, z. B. Essigsäure, wie gegebenenfalls ungesättigten Dicarbonsäuren, z. B. Malon-, Malein- oder Fumarsäure, oder wie Hydroxycarbonsäuren, z. B. Wein- oder Zitronensäure, oder mit Sulfonsäuren, wie Niederalkan- oder gegebenenfalls substituierten Benzolsulfonsäuren, z. B. Methan- oder *p*-Toluolsulfonsäure, gebildet werden. Verbindungen der Formel I mit einer sauren Gruppe, z. B. Carboxy, und einer basischen Gruppe, z. B. Amino, können auch in Form von inneren Salzen, d. h. in zwitterionischer Form vorliegen, oder es kann ein Teil des Moleküls als inneres Salz, und ein anderer Teil als normales Salz vorliegen. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Die Verbindungen der Formel I weisen wertvolle pharmakologische Eigenschaften, in erster Linie eine ausgeprägte Bindung dreiwertiger Metallionen, insbesondere derjenigen von Eisen (A. E. Martell und R. J. Motekaitis, "Determination and Use of Stability Constants",VCH Publishers, New York 1992), auf. Sie vermögen, wie z. B. im Tiermodell unter der Verwendung der nicht-eisenüberladenen Gallenfistelratte (R. J. Bergeron et al., *J. Med. Chem.* **34**, 2072-2078 (1991); G. F. Smith, W. H. McCurdy und H. Diehl, *Analyst* **77**, 418-422 (1952)) oder des eisenuberladenen Affen (R. J Bergeron et al., *Blood* **81**, 2166-2173 (1993)) in Dosen ab etwa 50 µmol/kg gezeigt werden kann, u. a. die Ablagerung eisenhaltiger Pigmente zu verhindern und bei bestehenden Eisenablagerungen im Organismus eine Ausscheidung des Eisens bewirken.

Bei verschiedenen Erkrankungen von Warmblütern, insbesondere des Menschen, kommt es zu einem Ueberschuss von Eisen(III)-Ionen im Blut und zu Ablagerung eisenhaltiger Pigmente im Gewebe, z. B. bei Hämochromatose, Hämosiderose, Lebercirrhose und Vergiftungen mit Verbindungen des Eisens Weitere Krankheiten und krankhafte Zustände des menschlichen Körpers (sowie des Körpers andere Warmblüter), die mit übermässiger Belastung des Organismus mit Eisen(III)-Ionen ($Fe^{3+}$-Ionen) einhergehen, sind beispielsweise Thalassämien, Sichelzellen-Anämie, sideroachrestische Anämie, aplastische Anämie und weitere anämische Formen, in welchen Hämosiderose (d. h. eine lokale oder allgemeine Erhöhung der Eisenvorräte in sonst unbeschädigten Körpergeweben) eine Rolle spielen. Zu diesem Typus gehören auch krankhafte Zustände, die sich in Patienten nach mehrmaligen Bluttransfusionen oder mehrfach wiederholter Dialysebehandlung bei fehlender oder geschädigter Nierenfunktion entwickeln. Eine Reduzierung der Eisen(III)-Konzentration ist auch für die Behandlung von Erkrankungen durch Eisen(III)-abhängige Mikroorganismen und Parasiten von Interesse, die nicht nur in humaner Medizin, wie insbesondere bei Malaria, sondern auch in Tiermedizin von prinzipieller Wichtigkeit ist. Auch die Komplexbildung mit anderen dreiwertigen Metallen kann zu deren Ausscheidung aus dem Organismus verwendet werden, z. B. zur Entfernung von Aluminium bei der Dialyse-Encephalopathie und Osteomalacia, sowie bei der Alzheimer Krankheit

Bereits seit langem bekannt und therapeutisch für diese Zwecke genutzt ist das Desferrioxamin B (H. Bickel, H. Keberle und E. Vischer, *Helv. Chim. Acta* **46**, 1385-9 [1963]). Als ein Nachteil dieses Präparates erweist sich jedoch die Tatsache, dass Desferrioxamin und seine Salze bei oraler Gabe nur eine geringe, unzureichende Wirksamkeit aufweisen und bei allen oben genannten Anwendungsmöglichkeiten eine parenterale Verabreichungsform benötigen. So ist z. B. als eine besonders wirksame Methode empfohlen, die Wirksubstanz mittels einer langsamen (8- bis 12-stündigen) subkutanen Infusion zu verabreichen, was aber die Anwendung einer tragbaren mechanischen Vornchtung, wie einer durch elektrischen Antrieb betätigten Infusionsspritze, bedingt. Abgesehen von ihrer Umständlichkeit sind solche Lösungen mit hohem Behandlungsaufwand behaftet, was Ihre Anwendung stark einschränkt, insbesondere wird eine umfassende Behandlung der Thalassämien in den Ländern des Mittelmeerraums, des Mittleren Ostens, Indiens und Südostasiens, der Malaria weltweit und der Sichelzellenanämie in den afrikanischen Ländern verunmöglicht. Diese weit verbreiteten Krankheiten stellen weiterhin ein schwerwiegendes Problem für das Gesundheitswesen in diesen Ländern dar und machen die Suche nach einer einfachere und billigeren Therapie, vorzugsweise mittels eines oral wirksamen Präparates, zur vordringlichen Aufgabe auf diesem Gebiet.

Aus GB 2 118 176 ist bekannt, dass orale Dosen von 1,2-Dimethyl-3-hydroxy-pyrid-4-on und seinen Alkylderivaten den Eisenüberschuss im Gewebe zu senken vermögen. Jedoch gibt es eindeutige Indizien, dass beispielsweise erstere Substanz, die auch unter dem Namen Deferripron bekannt ist, eine deutliche Toxizität aufweist und so zu ernsten Nebenwirkungen führen kann. Darüberhinaus sind derartige Substanzen in der Lage bei Verabreichung die Blut/Hirnschranke zu überwinden und im Gehim unerwünschte Nebenwirkungen auszulösen. Verglichen mit den bekannten Verbindungen erweisen sich die neuen Verbindungen der vorliegenden Erfindung nicht nur als oral wirksamer sondern auch als gut verträglich.

Die vorliegende Erfindung stellt somit Verbindungen der Formel I zur Verfügung, die sich sowohl durch ihre hervorragende orale Wirksamkeit als auch durch ihre Verträglichkeit selbst bei hoher Dosierung auszeichnen.

Ganz besonders betrifft die Erfindung die Verbindungen der Formel I, worin

$R_1$ Wasserstoff, Halogen, Niederalkyl, unsubstituiertes Niederalkoxycarbonyl, Amino, unsubstituiertes Niederalkylamino, unsubstituiertes Diniederalkylamino, Aminocarbonyl, N-Niederalkylaminocarbonyl, N,N-Diniederalkylaminocarbonyl, Carboxyl, Cyano, Hydroxy, Nitro oder Niederalkylendioxy, das mit der Gruppe B ein heterocyclisches sauerstoffhaltiges Ringsystem bildet; $R_2$ Wasserstoff, unsubstituiertes oder mit Halogen, Hydroxy oder Trifluoromethyl substituiertes Niederalkyl, Niederalkylenhydroxy, Niederalkylen-niederalkoxy,unsubstituiertes Niederalkylencarboxy, unsubstituiertes Niederalkylencarbonyl-niederalkoxy, Niederalkylenamin, oder N-Niederalkanoylniederalkylenamin; $R_3$ Wasserstoff, Niederalkyl, Carboxyl, Niederalkanoyloxyniederalkylen oder N-Niederalkylaminocarbonyl; $R_4$ Wasserstoff; A unsubstituiertes oder mit Niederalkyl, Halogen oder Hydroxy substituiertes Methylen, Carbonyl oder zusammen mit $R_2$ und den mit ihnen verbundenen Atomen einen sauerstoffhaltigen heterocyclischen Ring; und B einfach oder mehrfach substituiertes oder unsubstituiertes Phenyl oder Naphthyl oder einfach oder mehrfach substituiertes oder unsubstituiertes Heteroaryl; bedeuten; und deren Stereoisomere, Tautomere und pharmazeutisch verträgliche Salze, wobei, falls nicht anders angegeben, Niederalkyl sowie Niederalkylen für sich oder als Bestandteil anderer Gruppen alternativ substituiert sein können mit Halogen, Hydroxy oder Trifluoromethyl.

In erster Linie betrifft die Erfindung die Verbindungen der Formel I, worin

$R_1$ Wasserstoff, Halogen, Niederalkyl, Niederalkoxy, Cyano, Hydroxy oder Nitro; $R_2$ unsubstituiertes oder mit Halogen, Hydroxy oder Trifluoromethyl substituiertes Niederalkyl, Niederalkylenhydroxy oder Niederalkylenamin; $R_3$ und $R_4$ Wasserstoff; A unsubstituiertes oder mit Niederalkyl, Halogen oder Hydroxy substituiertes Methylen, Carbonyl oder zusammen mit $R_2$ und den mit ihnen verbundenen Atomen einen unsubstituierten sauerstoffhaltigen heterocyclischen Ring; und B einfach substituiertes oder unsubstituiertes Phenyl oder Naphthyl oder einfach substituiertes oder unsubstituiertes Heteroaryl; bedeuten; und deren Stereoisomere, Tautomere und pharmazeutisch verträgliche Salze, wobei, falls nicht anders angegeben, Niederalkyl sowie Niederalkylen für sich oder als Bestandteil anderer Gruppen alternativ substituiert sein können mit Halogen, Hydroxy oder Trifluoromethyl.

Vor allen Dingen betrifft die Erfindung die Verbindungen der Formel I, worin

$R_1$ Wasserstoff, Halogen oder unsubstituiertes Niederalkyl; $R_2$ unsubstituiertes oder mit Halogen, Hydroxy oder Trifluoromethyl substituiertes Niederalkyl oder Niederalkylenhydroxy; $R_3$ und $R_4$ Wasserstoff; A unsubstituiertes oder mit Niederalkyl, Halogen oder Hydroxy substituiertes Methylen oder zusammen mit $R_2$ und den mit ihnen verbundenen Atomen einen unsubstituierten sauerstoffhaltigen heterocyclischen Ring; und B einfach substituiertes oder unsubstituiertes Phenyl oder Naphthyl oder einfach substituiertes oder unsubstituiertes Heteroaryl; bedeuten; und deren Stereoisomere, Tautomere und pharmazeutisch verträgliche Salze, wobei, falls nicht anders angegeben, Niederalkyl sowie Niederalkylen für sich oder als Bestandteil anderer Gruppen alternativ substituiert sein können mit Halogen, Hydroxy oder Trifluoromethyl.

Die Erfindung betrifft vor allem die in den Beispielen beschriebenen spezifischen Verbindungen und Salze davon.

Ganz besonders betrifft die Erfindung 3-Hydroxy-1-(2-hydroxy-ethyl)-2-(hydroxy-phenylmethyl)-1*H*-pyridin-4-on, 3-Hydroxy-1-(2-hydroxy-ethyl)-2-(4-methyl-benzyl)-1*H*-pyridin-4-on; 3-Hydroxy-1-(2-hydroxy-ethyl)-2-(hydroxy-4-fluorphenyl-methyl)-1*H*-pyridin-4-on; 3-Hydroxy-1-(2-hydroxy-ethyl)-2-(4-fluorbenzyl)-*1H*-pyridin-4-on; 3-Hydroxy-1-(2-hydroxy-ethyl)-2-(hydroxy-4-chlorphenyl-methyl)-1*H*-pyridin-4-on; 3-Hydroxy-1-(2-hydroxy-ethyl)-2-(4-chlorbenzyl)-*1H*-pyridin-4-on; und deren Stereoisomere, Tautomere und pharmazeutisch verträgliche Salze davon.

Die Verbindungen können in an sich bekannter Weise hergestellt werden, indem man z. B. eine Verbindung der Formel II

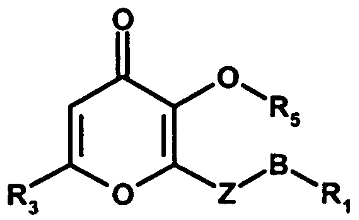

**(II)**

worin $R_1$, $R_3$, und B wie unter Formel I definiert sind, Z unsubsituiertes oder substituiertes Methylen, wobei gegebenfalls Substituenten in geschützter Form vorliegen können, oder Carbonyl bedeutet und $R_5$ gleich $R_4$ wie unter Formel I definiert ist oder gegebenenfalls eine geeignete Schutzgruppe darstellt, mit einer Verbindung der Formel III

$$H_2N\text{-}R_2 \qquad \textbf{(III)} \qquad\qquad (III)$$

worin $R_2$ wie unter Formel I definiert ist, umsetzt zu einer Verbindung der Formel IV

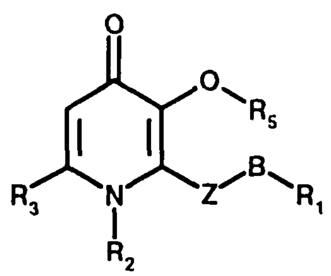

**(IV)**

worin $R_1$, $R_2$, $R_3$, $R_5$, B und Z wie unter Formeln I und II definiert sind, und diese Verbindung

a) wenn erforderlich, durch gleichzeitige Abspaltung einer Schutzgruppe $R_5$ und einer gegebenfalls an der Gruppe Z vorhanden Schutzgruppe in eine Verbindung der Formel I umwandelt, und, wenn erwünscht, in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, oder

b) wenn erforderlich, nach Abspaltung einer Schutzgruppe $R_5$ oder einer gegebenfalls an der Gruppe Z vorhanden Schutzgruppe zunächst in eine andere geschützte Form einer Verbindung der Formel I umwandelt und, wenn

erwünscht, in eine geschützte Form einer anderen Verbindung der Formel I umwandelt, und anschliessend durch Abspaltung der verbleibenden Schutzgruppen in eine Verbindung der Formel I umwandelt, und, wenn erwünscht, in eine andere Verbindung der Formel umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

In der folgenden näheren Beschreibung des Verfahrens haben die Symbole $R_1$-$R_5$, A, B und Z jeweils die unter Formeln I und II angegebene Bedeutung, sofern nichts anderes angegeben ist.

Das Verfahren entspricht der an sich bekannten Umsetzung von 3-Hydroxypyran-4-onen mit Ammoniak oder primären Aminen.

Schutzgruppen, ihre Einführung und Abspaltung sind beispielsweise beschrieben in J F W. McOmie, "Protective Groups in Organic Chemistry", Plenum Press, London, New York 1973, und in "Methoden der organischen Chemie", Houben-Weyl, 4 Auflage, Bd. 15/1, Georg-Thieme-Verlag, Stuttgart 1974, sowie in Theodora W. Greene, "Protective Groups in Organic Synthesis", John Wiley & Sons, New York 1981. Charakteristisch für Schutzgruppen ist, dass sie leicht, d. h. ohne dass unerwünschte Nebenreaktionen stattfinden, z. B. solvolytisch, reduktiv, photolytisch oder auch unter physiologischen Bedingungen abspaltbar sind.

Hydroxygruppen können beispielsweise in Form einer leicht spaltbaren Ester- oder Ethergruppe, vorzugsweise einer Alkanoyl- oder Aralkanoylestergruppe oder einer Cycloheteroalkyl-, Aralkyl- oder Alkoxyalkylethergruppe, aber auch einer Silylester- oder Silylethergruppe vorliegen, insbesondere als Acetyl- oder Benzoylester oder als Tetrahydropyranyl-, Benzyl- oder Methoxymethylether.

Die Umsetzung zwischen dem Pyranon der Formel II und dem Amin der Formel III findet ohne Lösungsmittel oder in geeigneten, inerten polaren Lösungsmitteln statt, insbesondere ein- oder mehrwertigen Alkoholen, z. B. Niederalkanolen oder Niederalkanpolyolen, wie Methanol, Propanol, Isopropanol, Glykol, Propandiol, oder vor allem Ethanol, Ethylenglykol oder Diethylenglykol. Mitunter ist die Zugabe einer Base, z. B. eines tertiären Amins, von Vorteil.

Die Reaktion findet bei Raumtemperatur oder bei erhöhten Temperaturen statt, vorzugsweise zwischen Raumtemperatur und der Rückflusstemperatur des Reaktionsgemisches. Die Temperatur kann auch im Reaktionszeitraum erhöht oder gesenkt werden.

Die Ausgangsmaterialien der Formel II sind neu und ebenfalls Gegenstand der vorliegenden Erfindung. Sie lassen sich nach an sich bekannten Verfahren durch Umsetzung von Pyromeconsäure *[CAS-Registry-Nr. : 496-63-9*] oder geeigneter Derivate mit den entsprechenden Aryl- oder Heteroarylaldehyden und, erforderlichenfalls, anschliessender Einführung geeigneter Schutzgruppen, und, gegebenenfalls, durch weitere Derivatisierung nach an sich bekannten Methoden herstellen.

So erhält man beispielsweise Verbindungen der Formel II, indem man Pyromeconsäure mit einem Aldehyd der Formel V

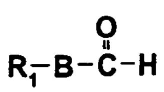 (V)

worin $R_1$ und B die unter Formel I angegebene Bedeutung haben, in an sich bekannter Weise in ethanolischer Natronlauge umsetzt und anschliessend, wenn erwünscht, geeignete Schutzgruppen einführt, so z. B. die 3-Hydroxyfunktion mit einem Aralkylhalogenid in an sich bekannter Weise verethert und die exocyclische Hydroxyfunktion mit einem Cycloheteroalkylether schützt.

Die Abspaltung der Schutzgruppen, die nicht Bestandteil des gewünschten Endprodukts der Formel I sind, erfolgt in an sich bekannter Weise, z. B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig.

Verbindungen der Formel I können auch in andere Verbindungen der Formel I überführt werden.

So kann z. B. eine Verbindung der Formel I, worin A für Hydroxymethyliden steht, zur entsprechenden Carbonylverbindung oxydiert werden, wobei man eine Verbindung der Formel I erhält, worin A Carbonyl bedeutet. Die Umsetzung wird beispielsweise in einem inerten unpolaren Lösungsmittel, wie einem Halogenniederalkan, unter Zusatz eines Bisalkansulfoxids und eines Pyridin-$SO_3$-Komplexes durchgeführt.

Eine Verbindung der Formel I, worin A für Hydroxymethyliden steht, kann z. B. auch zum entsprechenden Alkan reduziert werden, wobei man eine Verbindung der Formel I erhält, worin A Methylen bedeutet. Dazu wird beispielsweise die Verbindung zunächst acyliert und dann in Gegenwart eines Katalysators, z. B. Palladium, mit Wasserstoff umgesetzt.

Eine Verbindung der Formel I, worin $R_2$ für Hydroxyalkylen und A für Hydroxymethyliden steht, kann in an sich bekannter Weise, z. B. in Gegenwart einer Säure, zu einem inneren Ether umgesetzt werden, so dass $R_2$ und A zusammen mit den Atomen, mit denen sie verbunden sind, einen sauerstoffhaltigen Azaheterocyclus bilden. setzt werden, so dass $R_2$ und $R_3$ zusammen mit den Atomen, mit denen sie verbunden sind, einen sauerstoffhaltigen Azaheterocyclus bilden.

Eine Verbindung der Formel I, worin $R_2$ für Niederalkylencarboxy und A für Hydroxymethyliden steht, kann in an sich bekannter Weise, z. B. in Gegenwart einer Säure, zu einem inneren Ester umgesetzt werden, so dass $R_2$ und A zusammen mit den Atomen, mit denen sie verbunden sind, einen sauerstoffhaltigen Azaheterocyclus bilden.

Wenn Ausgangsverbindungen der Formel I oder irgendwelche Zwischenprodukte störende reaktionsfähige Gruppen, z. B. Carboxy-, Hydroxy- oder Aminogruppen enthalten, können diese vorübergehend durch leicht abspaltbare Schutzgruppen geschützt werden. Vorteilhafterweise kann man jedoch für die Umsetzung auch ein geeignetes Zwischenprodukt der Formel IV verwenden.

Zur Aufarbeitung der erhältlichen Verbindungen der Formel I oder ihrer Salze und erforderlichenfalls der Zwischenprodukte finden übliche Verfahren Verwendung, beispielsweise Solvolyse von überschüssigen Reagenzien; Umkristallisieren, Chromatographieren, z. B. Verteilungs-, Ionen- oder Gelchromatographie, Verteilung zwischen anorganischer und organischer Lösungsmittelphase; ein- oder mehrfache Extraktion, insbesondere nach Ansäuern oder Erhöhung der Basizität oder des Salzgehaltes, Trocknen über hygroskopischen Salzen oder bei erhöhter Temperatur, gegebenenfalls unter Durch- oder Vorbeileiten eines Gasstromes; Digerieren; Filtrieren; Waschen; Auflösen; Eindampfen (erforderlichenfalls im Vakuum oder Hochvakuum); Destillation; Fällung; Zentrifugation; Kristallisation, beispielsweise von erhaltenen Verbindungen in Oelform oder aus der Mutterlauge, wobei auch mit einem Kristall des Endproduktes angeimpft werden kann; Lyophilisation; oder eine Kombination zweier oder mehrerer der genannten Aufarbeitungsschritte, die auch wiederholt eingesetzt werden können; etc..

Ausgangsmaterialien und Zwischenprodukte können in reiner Form, beispielsweise nach Aufarbeitung, wie zuletzt erwähnt, in teilweise gereinigter Form oder auch beispielsweise direkt als Rohprodukte verwendet werden

Die Verbindungen, einschliesslich ihrer Salze, können auch in Form von Hydraten oder Solvaten erhalten werden, oder ihre Kristalle können z. B. das zur Kristallisation verwendete Lösungsmittel einschliessen.

Lösungs- und Verdünnungsmittel sind beispielsweise Wasser, Alkohole, z. B. Niederalkanole, wie Methanol, Ethanol, Propanol oder Butanol, Diole, wie Ethylenglykol, Tri- oder Polyole, wie Glycenn oder Diethylenglykol, oder Arlyalkohole, wie Phenol oder Benzylalkohol, Saureamide, z. B. Carbonsäureamide, wie N,N-Dimethylformamid, oder N,N-Dimethylacetamid, Amide anorganischer Säuren, wie Hexamethylphosphorsäuretriamid, Ether, z. B. cyclische Ether, wie Tetrahydrofuran oder Dioxan, oder acyclische Ether, wie Diethylether oder Ethylenglykoldimethlyether, halogenierte Kohlenwasserstoffe, wie Halogenniederalkane, z. B. Methylenchlorid oder Chloroform, Ketone, wie Aceton, Nitrile, wie Acetonitril, Ester, wie Essigsaureethylester, Bisalkansulfoxide, wie Dimethylsulfoxid, Stickstoffheterocyclen, wie N-Methylpyrrolidon oder Pyridin, Kohlenwasserstoffe, z. B. Niederalkane, wie Hexan oder Heptan, oder Aromaten, wie Benzol, Toluol oder Xylol(e), oder Gemische dieser Lösungsmittel. wobei die jeweils geeigneten Lösungsmittel für die oben genannten Reaktionen und Aufarbeitungsschritte ausgewählt werden können.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen. Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z. B eines Salzes davon, verwendet wird.

Salze von Verbindungen I können in an sich bekannter Weise hergestellt werden. So erhält man beispielsweise Saureadditionssalze von Verbindungen I durch Behandeln mit einer geeigneten Säure oder einem geeigneten lonenaustauscherreagens und Salze mit Basen durch Behandeln mit einer geeigneten Base oder einem geeigneten lonenaustauscherreagens. Salze von Verbindungen der Formel I konnen in üblicher Weise in die freien Verbindungen I überführt werden, Säureadditionssalze z. B. durch Behandeln mit einem geeigneten basischen Mittel oder einem geeigneten lonenaustauscherreagens und Salze mit Basen z. B. durch Behandeln mit einer geeigneten Säure oder einem geeigneten lonenaustauscherreagens.

Salze von Verbindungen I können in an sich bekannter Weise in andere Salze von Verbindungen I umgewandelt werden, Säureadditionssalze beispielsweise in andere Säureadditionssalze, z. B. durch Behandeln eines Salzes einer organische Säure, wie eines Hydrochlorids, mit einem geeigneten Metallsalz, wie einem Natrium-, Barium-, oder Silbersalz, einer Säure, z. B. Silberacetat, in einem geeigneten Lösungsmittel, in welchem ein sich bildendes organisches Salz, z. B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren, beispielsweise Stereoisomere oder Tautomere, oder als Gemisch derselben vorliegen. Dabei sind als reine Isomere z. B reine Enantiomere, reine Diastereoisomere oder reine Tautomere erhältlich. Entsprechend konnen als Isomerengemische z. B. Racemate oder Diastereoisomerengemische vorliegen. Verfahrensgemäss oder anderweitig erhältliche Isomerengemische von Verbindungen I in freier Form oder in Salzform können in üblicher Weise in die Komponenten aufgetrennt werden, z. B. aufgrund der physikalisch chemischen Unterschiede der Bestandteile in bekannter Weise durch fraktionierte Kristallisation, Destillation und/oder Chromatographie. Vorteilhaft isoliert man das wirksamere Isomere.

Die Erfindung betrifft ebenfalls Verbindungen der Formel I und ihrer pharmazeutisch verwendbaren Salze zur Behandlung von Krankheiten, die einen Eisenüberschuss im menschlichen oder tierischen Körper verursachen oder durch ihn verursacht werden, vorzugsweise in Form von pharmazeutisch verwendbaren Zubereitungen, insbesondere in einem Verfahren zur therapeutischen Behandlung des menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, insbesondere oralen, ferner rektalen, Verabreichung und um solche zur parenteralen Verabreichung an Warmblüter, vor allem an Menschen, wobei der pharmakologische Wirkstoff allein oder zusammen mit üblichen pharmazeutischen Hilfsstoffen enthalten ist. Die pharmazeutischen Präparate enthalten (in Gewichtsprozenten) z. B. von etwa 0,001% bis 100%, vorzugsweise von etwa 0,1% bis etwa 100% des Wirkstoffs.

Pharmazeutische Präparate zur enteralen bzw. parenteralen Verabreichung sind z. B. solche in Dosiseinheitsformen, wie Dragees, Tabletten, dispergierbare Tabletten, Brausetabletten, Kapseln, suspendierbare Pulver, Suspensionen oder Suppositorien, oder Ampullen. Diese werden in an sich bekannter Weise, z. B. mittels konventioneller Dragier-, Misch-, Granulier-, oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe wie Zucker, z. B. Lactose, Saccharose, Mannit, oder Sorbit, Cellulosepraparate und/oder Calciumphosphate, z. B. Tricalciumphosphat oder Calciumhydrogenphosphat, ferner Bindemittel, wie Stärkekleister, unter Verwendung z. B. von Mais-, Weizen-, Reis- oder Kartoffelstärke, Gelatine, Tragant, Methylcellulose und oder Polyvinylpyrrolidon, und, wenn erwünscht, Sprengmittel, wie die oben genannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium oder Calciumstearat, und/oder Polyethylenglykol. Dragee-Kerne werden mit geeigneten, gegebenenfalls Magensaft-resistenten, Ueberzügen versehen, wobei man u. a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z. B. zur Identifizierung oder zur Kennzeichnung verschiedener Wirkstoffdosen, beigefügt werden.

Dispergierbare Tabletten sind in verhältnismässig geringer Flüssigkeitsmenge, z. B. Wasser, rasch zerfallende Tabletten, die gegebenfalls Aromen oder Stoffe zu Maskierung des Wirkstoffeigengeschmacks enthalten. Sie können vorteilhaft zur oralen Applikation grosser Einzeldosen eingesetzt werden, bei denen die zu applizierende Wirkstoffmenge so gross ist, dass sie bei Darreichung als unzerteilt oder unzerkaut zu schluckende Tabletten nicht mehr bequem aufgenommen werden kann, insbesondere von Kindern. Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls Stabillisatoren, enthalten. In weichen Kapseln ist der Wirkstoff vorzugsweise in geeigneten Flüssigkeiten, wie fetten Oelen,

Parafinöl oder flüssigen Polyethylenglykolen, gelöst oder suspendiert, wobei ebenfalls Stabilisatoren zugefügt sein konnen.

Darüber hinaus kommen für eine orale Darreichungsform auch suspendierbare Pulver, z B solche, die als "Powder in Bottle", kurz "PIB", bezeichnet werden, oder trinkfertige Suspensionen in Betracht. Für diese Form wird der Wirkstoff beispielsweise mit pharmazeutisch anwendbaren oberflächenaktiven Substanzen, wie beispielsweise Natriumlaurylsulfat oder Polysorbat, Suspendierungshilfsmitteln, z B. Hydroxypropylcellulose, Hydroxypropylmethylzellulose oder einem anderen aus dem Stand der Technik bekannten und beispielsweise in "Handbook of Pharmaceutical Excipients" vorbeschriebenen, pH-Regulatoren, wie Zitronenoder Weinsäure und deren Salzen oder einem USP-Puffer und, gegebenenfalls, Füllstoffen, z. B. Lactose, und weiteren Hilfsstoffen vermischt und in geeignete Gefasse, vorteilhafterweise Portionsfläschchen oder -ampullen, abgefüllt. Unmittelbar vor der Anwendung wird eine bestimmte Menge Wasser zugesetzt und die Suspension durch Schütteln hergestellt. Alternativ kann das Wasser auch bereits vor dem Abfüllen zugesetzt werden.

Als rektal anwendbare pharmazeutische Präparate kommen z. B. Suppositorien in Betracht, welche aus einer Kombination des Wirkstoffes mit einer Suppositonengrundmasse bestehen. Als Suppositoriengrundmasse eignen sich z. B. natürliche oder synthetische Triglyceride, Paraffinkohlenwasserstoff, Polyethylenglykole oder höhere Alkanole. Ferner können auch Gelatine-Rektalkapseln verwendet werden, die eine Kombination des Wirkstoffes mit einem Grundmassenstoff enthalten. Als Grundmassenstoffe kommen z. B. flüssige Tnglyceride, Polyethylenglykole oder Paraffinkohlenwasserstoffe in Frage

Zur parenteralen Verabreichung eignen sich in erster Linie wässerige Lösungen eines Wirkstoffes in wasserlöslicher Form, z. B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z. B. Sesamol, oder synthetische Fettsäureester, z. B. Ethyloleat oder Tnglycende, verwendet, oder wässerige Injektionssuspensionen, welche viskositätserhöhende Stoffe, z. B. Natriumcarboxymethylcellulose, Sorbit und/oder Dextran, und gegebenenfalls auch Stabilisatoren enthalten.

Die Dosierung des Wirkstoffs kann von verschiedenen Faktoren, wie Wirksamkeit und Wirkungsdauer des Wirkstoffs, Stärke der zu behandelnden Krankheit bzw ihrer Symptome, Applikationsweise, Warmblüter-Spezies, -Geschlecht, -Alter, -Gewicht und/oder individuellem Zustand des Warmblüters, abhängen. Die täglich zu verabreichenden Dosen liegen bei oraler Applikation zwischen 10 und etwa 120 mg/kg, insbesondere 20 und etwa 80 mg/kg und für einen Warmblüter mir einem Körpergewicht von etwa 40 kg vorzugsweise zwischen etwa 400 mg und etwa 4800 mg, insbesondere etwa 800 mg bis 3200 mg, die zweckmassigerweise auf 2 bis 12 Einzeldosen aufgeteilt wird.

Die vorstehend beschriebene Erfindung soll durch die nachfolgenden Beispiele illustriert werden, jedoch ohne sie auf diese einzuschranken. (Vor- und nachstehend haben folgende Abkürzungen - soweit nicht anders angegeben - die Bedeutungen: Dimethylformamid = N,N-Dimethylformamid; Essigester = Essigsaureethylester; Ether = Diethylether; HCl = Salzsäure, wässerige Lösung; NaOH = Natronlauge, wasserige Lösung; Fp = Schmelzpunkt)

<u>Beispiel 1:</u> <u>3-Hydroxy-1-(2-hydroxy-ethyl)-2-(hydroxy-phenyl-methyl)-1*H*-pyridin-4-on</u>

12,37 g 3-Benzyloxy-1-(2-hydroxy-ethyl)-2-(hydroxy-phenyl-methyl)-1 *H*-pyridin-4-on werden in 200 ml Methanol gelöst und bei Raumtemperatur über 1,2 g Palladiumkohle (5%) bis zur Aufnahme von 1 mol $H_2$ pro mol Edukt hydriert. Man filtriert vom Katalysator ab und dampft das Filtrat am Rotationsverdampfer zur Trockene ein. Nach Umkristallisation aus Methanol erhält man 3-Hydroxy-1-(2-hydroxy-ethyl)-2-(hydroxy-phenyl-methyl)-1 *H*-pyridin-4-on als farblose Kristalle. Fp:198-200°C

Das Ausgangsmaterial kann zum Beispiel wie folgt hergestellt werden:

a) <u>3-Benzyloxy-2-(hydroxy-phenyl-methyl)-pyran-4-on:</u> 29,5 g 3-Hydroxy-2-(hydroxy-phenylmethyl)-pyran-4-on (Beschrieben in: BE 651427 [*CAS-Nr.: 4940-15-2*]) und 40 g pulverisiertes Kaliumcarbonat werden in 210 ml Dimethylformamid unter Rühren vorgelegt. Man setzt 24,3 g Benzylbromid zu und rührt die Suspension während 18 Stunden bei Raumtemperatur. Zum Aufarbeiten giesst man auf 1000 ml Wasser und extrahiert zweimal mit je 300 ml Essigester. Die organischen Phasen werden viermal mit je 50 ml Wasser gewaschen, vereinigt und über Magnesiumsulfat getrocknet. Man filtriert vom Trockenmittel ab und dampft das Filtrat am Rotationsverdampfer ein. Der Rückstand wird mit Diethylether verrührt und abfiltriert. Nach dem Trocknen erhält man 3-Benzyloxy-2-(hydroxy-phenylmethyl)-pyran-4-on als blassgelbe Kristalle Fp 121-122°C

b) <u>3-Benzyloxy-2-[phenyl-(tetrahydropyran-2-yloxy)-methyl]-pyran-4-on</u> 23,1 g 3-Benzyloxy2-(hydroxy-phenylmethyl)-pyran-4-on werden mit 250 ml Dichlormethan und 10 ml 3,4-Dihydro-2*H*-pyran [*CAS-Nr.:*11*0-87-2*] versetzt. Nach Zugabe von 0,1 g *p*-Toluolsulfonsäure rührt man 5 Stunden bei Raumtemperatur. Man wäscht die Lösung einmal mit verdünnter Natriumbicarbonatlösung und zweimal mit Wasser. Anschliessend trocknet man über Magnesiumsulfat und filtriert. Nach dem Eindampfen erhält man 3-Benzyloxy-2-[phenyl-(tetrahydropyran-2-yloxy)-methyl]-pyran-4-on als gelbliches, kristallines Diastereoisomerengemisch. Fp:115-116,5°C

c) 3-Benzyloxy-1-(2-hydroxy-ethyl)-2-[phenyl-(tetrahydropyran-2-yloxy)-methyl]-1*H*-pyridin-4-on: 11,8 g 3-Benzyloxy-2-[phenyl-(tetrahydropyran-2-yloxy)-methyl]-pyran-4-on werden zusammen mit 10 ml Ethanolamin in 100 ml Ethanol während 26 Stunden unter Rückfluss gekocht. Zum Aufarbeiten entfernt man das Ethanol am Rotationsverdampfer. Der Rückstand wird in 200 ml Essigester aufgenommen und dreimal mit je 50 ml Wasser gewaschen. Die organische Phase wird über Magnesiumsulfat getrocknet, filtriert und am Rotationsverdampfer zur Trockene eingedampft. Als Rückstand verbleibt 3-Benzyloxy-1 -(2-hydroxy-ethyl)-2-[phenyl-(tetrahydropyran-2-yloxy)-methyl]-1 *H*-pyridin-4-on als braunes Harz. Diastereoisomerengemisch, $R_f$-Wert: 0,2 (Kieselgel 60; Essigester/Ethanol 9/1).

d) 3-Benzyloxy-1-(2-hydroxy-ethyl)-2-(hydroxy-phenyl-methyl)-1*H*-pyridin-4-on: 3,39 g 3-Benzyloxy-1-(2-hydroxy-ethyl)-2-[phenyl-(tetrahydropyran-2-yloxy)-methyl]-1 *H*-pyridin-4-on werden in 40 ml Ethanol und 10 ml HCl 2N während 3 Stunden unter Rückfluss gekocht. Zum Aufarbeiten befreit man am Rotationsverdampfer vom Ethanol. Der Rückstand wird mit 50 ml Wasser verdünnt und mit 10 ml Essigester überschichtet. Unter Rühren gibt man jetzt 25 ml gesättigte, wässerige Natriumbicarbonatlösung zu. Das ausfallende Produkt wird abfiltriert und mit Wasser und Essigester gewaschen. Nach dem Trocknen verbleibt 3-Benzyloxy-1-(2-hydroxy-ethyl)-2-(hydroxy-phenyl-methyl)-1 *H*-pyridin-4-on als farblose Kristalle. Fp: 191,5-192,5°C

Beispiel 2: 2-Benzyl-3-hydroxy-1-(2-hydroxy-ethyl)-1*H*-pyridin-4-on

2,14 g 3-Benzyloxy-1-(2-hydroxy-ethyl)-2-(hydroxy-phenyl-methyl)-1 *H*-pyridin-4-on (Beispiel 1d) werden in 200 ml Methanol über 0,5 g Palladiumkohle (5%) bei Normaldruck bei einer Temperatur von 50°C hydriert bis 2 Mol $H_2$ pro Mol Edukt aufgenommen worden sind Man filtriert vom Katalysator ab und dampft das Filtrat am Rotationsverdampfer ein. Der Ruckstand wird aus Ethanol umkristallisiert und man erhalt 2-Benzyl-3-hydroxy-1-(2-hydroxyethyl)-1 *H*-pyridin-4-on als farblose Kristalle. Fp: 228-230°C.

Das gleiche Produkt kann auch wie folgt hergestellt werden:

0,915 g 2-Benzyl-3-hydroxy-1-(2-acetoxy-ethyl)-1 *H*-pyridin-4-on (Beispiel 42)werden in einer Mischung aus 2 ml 2N NaOH und 10 ml Ethanol während 24 Stunden bei Raumtemperatur gerührt. Man gibt nun 2 ml 2N HCl zu und rührt eine Stunde bei Raumtemperatur. Man filtriert und wäscht mit kaltem Ethanol. Nach dem Trocknen erhält man 2-Benzyl-3-hydroxy-1-(2-hydroxy-ethyl)-1 *H*-pyridin-4-on.

Beispiel 3: 2-[(4-Fluorphenyl)-hydroxy-methyl]-3-hydroxy-1-(2-hydroxy-ethyl)-1*H*-pyridin-4-on

1,72 g 2-[(4-Fluorphenyl)-hydroxy-methyl]-3-benzyloxy-1-(2-hydroxy-ethyl)-1 *H*-pyridin-4-on werden analog Beispiel 1 hydriert und man erhält 2-[(4-Fluorphenyl)-hydroxy-methyl]-3-hydroxy-1-(2-hydroxy-ethyl)-1 *H*-pyridin-4-on. Farblose Kristalle, Fp: 180-185°C, Kristallumwandlung, dann Fp: 200-203°C

Das Ausgangsmaterial kann zum Beispiel wie folgt hergestellt werden:

a) 2-[(4-Fluorphenyl)-hydroxy-methyl]-3-hydroxy-pyran-4-on: 5,6 g Pyromeconsäure (3-Hydroxy-pyran-4-on) [*CAS-Nr.: 496-63-9*] werden in 10 ml Wasser und 24,5 ml 2N NaOH bei Raumtemperatur unter Rühren aufgelöst. Man gibt 6,33 g 4-Fluorbenzaldehyd und 25 ml Ethanol zu und rührt 18 Stunden bei Raumtemperatur. Zum Aufarbeiten wird am Rotationsverdampfer das Ethanol entfernt und die zurückbleibende wässerige Lösung mit 24,5 ml 2N HCl neutralisiert. Das auskristallisierte Produkt wird abfiltriert, mit Wasser gewaschen und getrocknet. Man erhält 2-[(4-Fluorphenyl)-hydroxy-methyl]-3-hydroxy-pyran-4-on als farblose Kristalle. Fp:154-156°C.

b) 2-[(4-Fluorphenyl)-hydroxy-methyl]-3-benzyloxy-pyran-4-on: Aus 9 g 2-[(4-Fluorphenyl)-hydroxy-methyl]-3-hydroxy-pyran-4-on erhält man durch Umsetzung mit Benzylbromid in Dimethylformamid und Kaliumcarbonat analog Beispiel 1a: 2-[(4-Fluorphenyl)-hydroxymethyl]-3-benzyloxy-pyran-4-on. Farblose Kristalle, Fp:117-117,5°C.

c) 3-Benzyloxy-2-[(4-fluorphenyl)-(tetrahydropyran-2-yloxy)-methyl]-pyran-4-on Aus 10,8 g 2-[(4-Fluorphenyl)-hydroxy-methyl]-3-benzyloxy-pyran-4-on erhält man durch Umsetzung mit 3,4-Dihydro-2*H*-pyran analog Beispiel 1 b: 3-Benzyloxy-2-[(4-fluorphenyl)-(tetrahydropyran-2-yloxy)-methyl]-pyran-4-on als kristallines Diastereoisomerengemisch. Fp:104-107°C.

d) 3-Benzyloxy-1-(2-hydroxy-ethyl)-2-[(4-fluorphenyl)-(tetrahydropyran-2-yloxy)-methyl]-1*H*-pyridin-4-on: 4,1 g 3-Benzyloxy-2-[(4-fluorphenyl)-(tetrahydropyran-2-yloxy)-methyl]-pyran-4-on werden in 40 ml Ethanol und 8 ml Ethanolamin während 24 Stunden unter Rückfluss gekocht. Nach Aufarbeitung analog Beispiel 1c erhält man

3-Benzyloxy-1-(2-hydroxy-ethyl)-2-[(4-fluorphenyl)-(tetrahydropyran-2-yloxy)-methyl]-1 *H*-pyridin-4-on als sprodes Harz $R_f$-Wert: 0,15 (Kieselgel 60, Essigester/Ethanol 9/1).

e) 2-[(4-Fluorphenyl)-hydroxy-methyl]-3-benzyloxy-1-(2-hydroxy-ethyl)-1*H*-pyridin-4-on: 13,7 g 3-Benzyloxy-1-(2-hydroxy-ethyl)-2-[(4-fluorphenyl)-(tetrahydropyran-2-yloxy)-methyl]-1 *H*-pyridin-4-on werden in 150 ml Ethanol und 50 ml 2N HCl während 3 Stunden unter Rückfluss gekocht. Zum Aufarbeiten befreit man am Rotationsverdampfer vom Ethanol. Der Rückstand wird mit 50 ml Wasser verdünnt und mit 50 ml Essigester überschichtet. Unter Rühren gibt man jetzt 50 ml 2N NaOH zu. Das ausfallende Produkt wird abfiltriert und mit Wasser und Essigester gewaschen. Nach dem Trocknen verbleibt 2-[(4-Fluorphenyl)-hydroxy-methyl]-3-benzyloxy-1-(2-hydroxy-ethyl)-1 *H*-pyridin-4-on als farblose Kristalle. Fp: 187-187,5°C.

Beispiel 4: 3-Hydroxy-1-methyl-2-(hydroxy-phenyl-methyl)-1*H*-pyridin-4-on:

0,643 g 3-Benzyloxy-1-methyl-2-(hydroxy-phenyl-methyl)-1*H*-pyridin-4-on werden in 25 ml Methanol über 0,1 g Palladiumkohle (5%) bei Normaldruck und Raumtemperatur hydriert bis zur Aufnahme von 1 mol $H_2$ pro mol Edukt. Nach Entfernen des Katalysators und Umkristallisation aus Methanol erhält man 3-Hydroxy-1-methyl-2-(hydroxy-phenyl-methyl)-1 *H*-pyridin-4-on. Fp: 210-213°C.

Das Ausgangsmaterial kann zum Beispiel wie folgt hergestellt werden:

a) 3-Benzyloxy-2-[phenyl-(tetrahydropyran-2-yloxy)-methyl]-1-methyl-1*H*-pyridin-4-on: 4,6 g 3-Benzyloxy-2-[phenyl-(tetrahydropyran-2-yloxy)-methyl]-pyran-4-on (Beispiel 1b) werden mit 50 ml einer 30 proz. Losung von Methylamin in Ethanol versetzt und 96 Stunden verschlossen bei Raumtemperatur stehen gelassen. Zum Aufarbeiten dampft man am Rotationsverdampfer bis zur Trockene ein. Man erhält rohes Diastereoisomerengemisch von 3-Benzyloxy-2-[phenyl-(tetrahydropyran-2-yloxy)-methyl]-1-methyl-1 *H*-pyridin-4-on. $R_f$-Wert: 0,2 (Kieselgel 60, Essigester/Ethanol 9/1).

b) 3-Benzyloxy-1-methyl-2-(hydroxy-phenyl-methyl)-1*H*-pyridin-4-on: Aus 4,1 g 3-Benzyloxy-2-[phenyl-(tetrahydropyran-2-yloxy)-methyl]-1-methyl-1*H*-pyridin-4-on erhält man analog Beispiel 1 d: 3-Benzyloxy-1-methyl-2-(hydroxy-phenyl-methyl)-1 *H*-pyridin-4-on als blassgelbe Kristalle. Fp: 181,5-183,5°C.

Beispiel 5: 2-Benzoyl-3-hydroxy-1-(2-hydroxy-ethyl)-1*H*-pyridin-4-on

0,95 g 2-Benzoyl-3-benzyloxy-1-(2-benzyloxy-ethyl)-1 *H*-pyridin-4-on werden in Methanol bei Raumtemperatur und Normaldruck hydriert bis zur Aufnahme von 2 mol $H_2$ pro mol Edukt. Nach Entfernen des Katalysators wird am Rotationsverdampfer eingedampft und der Rückstand über 20 g Kieselgel chromatographiert. Als Eluent dient ein Gemisch von Essigester und Ethanol im Verhältnis von 95:5. Bei einer Fraktionengrösse von 17 ml eluiert man das Produkt in den Fraktionen 6-20. Nach Umkristallisation aus Ethanol erhält man 2-Benzoyl-3-hydroxy-1-(2-hydroxyethyl)-1 *H*-pyridin-4-on als farblose Kristalle. Fp: 195-200°C

Das Ausgangsmaterial kann zum Beispiel wie folgt hergestellt werden:

a) 3-Benzyloxy-1-(2-benzyloxy-ethyl)-2-[phenyl-(tetrahydropyran-2-yloxy)-methyl]-1*H*-pyridin-4-on: 3,39 g 3-Benzyloxy-2-[phenyl-(tetrahydropyran-2-yloxy)-methyl]-pyran-4-on (Beispiel 1 b) werden zusammen mit 5 ml 2-Benzyloxyethylamin in 25 ml Diethylenglykol während 26 Stunden bei 120°C gerührt. Zum Aufarbeiten wird mit 300 ml Wasser verdünnt und zweimal mit je 100 ml Essigester extrahiert. Die organischen Phasen werden dreimal mit je 50 ml Wasser gewaschen. Man vereinigt die organischen Phasen, trocknet über Magnesiumsulfat filtriert und dampft das Filtrat am Rotationsverdampfer zur Trockene ein. Der Rückstand wird über 260 g Kieselgel chromatographiert. Als Eluent dient eine Mischung von Essigester und Ethanol im Verhältnis von 98/2. Bei einer Fraktionengrösse von 140 ml enthalten die Fraktionen 11-24 das Produkt. Man erhält 3-Benzyloxy-1-(2-benzyloxy-ethyl)-2-[phenyl-(tetrahydropyran-2-yloxy)-methyl]-1*H*-pyndin-4-on als gelbes, klares Harz. Diastereoisomerengemisch, $R_f$-Wert: 0,31 (Kieselgel 60, Essigester/Ethanol 95/5).

b) 3-Benzyloxy-1-(2-benzyloxy-ethyl)-2-(hydroxy-phenyl-methyl)-1*H*-pyridin-4-on: 2,65 g 3-Benzyloxy-1-(2-benzyloxy-ethyl)-2-[phenyl-(tetrahydropyran-2-yloxy)-methyl]-1 *H*-pyridin-4-on werden in 25 ml Ethanol und 10 ml HCl 2N während 3 Stunden unter Rückfluss gekocht Man entfernt das Ethanol am Rotationsverdampfer und neutralisiert die zurückbleibende wässenge Lösung mit überschüssiger wässeriger Natriumbicarbonatlosung. Man extrahiert das ausfallende Produkt zweimal mit je 50 ml Essigester. Die organischen Phasen werden zweimal mit je 20 ml Wasser gewaschen. Man vereinigt die organischen Phasen, trocknet über Magnesiumsulfat, filtriert und dampft das Filtrat am Rotationsverdampfer zur Trockene ein. Der Rückstand wird aus Essigester umkristallisiert. Man

erhält auf diese Weise 3-Benzyloxy-1-(2-benzyloxy-ethyl)-2-(hydroxy-phenyl-methyl)-1 *H*-pyridin-4-on. Gelbliche Kristalle, Fp: 154-155°C.

c) 2-Benzoyl-3-benzyloxy-1-(2-benzyloxy-ethyl)-1*H*-pyridin-4-on: 1,79 g 3-Benzyloxy-1-(2-benzyloxy-ethyl)-2-(hydroxy-phenyl-methyl)-1 *H*-pyridin-4-on werden in 20 ml Dichlorethan und 5 ml Dimethylsulfoxid vorgelegt. Man gibt 3,5 ml Triethylamin zu und kühlt mit einem Eisbad auf eine Innentemperatur von 3-5°C. Nun gibt man 2,58 g Pyridin·$SO_3$-Komplex *[CAS-Nr.: 26412-87-3*] zu und lässt das Gemisch wieder auf Raumtemperatur auftauen. Nach 18 Stunden Rühren bei Raumtemperatur schüttelt man zweimal mit Wasser aus, trocknet die organische Phase über Magnesiumsulfat, filtriert und dampft am Rotationsverdampfer zur Trockene ein. Der Rückstand wird über Kieselgel chromatographiert. Als Eluent dient eine Mischung von Essigester und Ethanol im Verhältnis von 95/5. Man erhält 2-Benzoyl-3-benzyloxy-1-(2-benzyloxy-ethyl)-1*H*-pyridin-4-on als gelbes dickes Oel $R_f$-Wert: 0,18 (Kieselgel 60, Essigester/Ethanol 95/5).

Beispiel 6: 3-Hyrdroxy-1-(2-hydroxy-ethyl)-2-(hydroxy-pyridin-3-yl-methyl)-1*H*-pyridin-4-on

0,6 g 3-Benzyloxy-1-(2-hydroxy-ethyl)-2-(hydroxy-pyridin-3-yl-methyl)-1 *H*-pyridin-4-on werden in 20 ml Methanol bei Raumtemperatur und Normaldruck hydriert bis zur Aufnahme von 1 mol $H_2$ pro mol Edukt. Man filtriert vom Katalysator ab und dampft das Filtrat zur Trockene ein. Der Rückstand wird aus Methanol umkristallisiert. Man erhält auf diese Weise 3-Hydroxy-1-(2-hydroxy-ethyl)-2-(hydroxy-pyridin-3-yl-methyl)-1*H*-pyridin-4-on als farblose Kristalle, Fp: 189-190,5°C.

Das Ausgangsmaterial kann zum Beispiel wie folgt hergestellt werden

a) 2-(Hydroxy-pyridin-3-yl-methyl)-3-hydroxy-pyran-4-on 5,6 g Pyromeconsäure werden in 11 ml Wasser und 23,7 ml 2N NaOH bei Raumtemperatur unter Rühren aufgelöst. Man gibt 5,52 g Pyridin-3-carbaldehyd [*CAS-Nr.: 500-22-*1] zu und rührt 18 Stunden bei Raumtemperatur. Man neutralisiert mit 23,7 ml 2N HCI und kühlt die entstehende Knstallsuspension 2 Stunden im Eisbad. Man filtriert und wäscht mit wenig kaltem Wasser. Nach dem Trocknen erhält man 2-(Hydroxy-pyridin-3-yl-methyl)-3-hydroxy-pyran-4-on. Blassgelbe Kristalle, Fp: 176-180°C unter Zersetzung.

b) 3-Benzyloxy-2-(hydroxy-pyridin-3-yl-methyl)-pyran-4-on: 10,6 g 2-(Hydroxy-pyridin-3-yl-methyl)-3-hydroxy-pyran-4-on und 14 g Kaliumcarbonat werden mit 60 ml Dimethylformamid übergossen. Man rührt die Mischung im Eisbad und gibt 8,27 g Benzylbromid zu. Nach 6 Stunden Rühren im Eisbad lässt man auf Raumtemperatur auftauen und lässt so noch 16 Stunden weiterrühren. Man giesst auf 500 ml Wasser und extrahiert zweimal mit je 100 ml Essigester. Die organischen Phasen werden viermal mit je 50 ml Wasser gewaschen. Man vereinigt die organischen Phasen, trocknet über Magnesiumsulfat filtriert und dampft das Filtrat am Rotationsverdampfer zur Trockene ein. Der Rückstand wird über 300 g Kieselgel chromatographiert. Als Eluent dient eine Mischung von Essigester und Ethanol im Verhältnis von 95/5. Man erhält so 3-Benzyloxy-2-(hydroxy-pyridin-3-yl-methyl)-pyran-4-on als gelben, spröden Schaum. $R_f$-Wert: 0,4 (Kieselgel 60, Essigester/Ethanol 9/1).

c) 3-Benzyloxy-2-[pyridin-3-yl-(tetrahydropyran-2-yloxy)-methyl]-pyran-4-on: 7,39 g 3-Benzyloxy-2-(hydroxy-pyridin-3-yl-methyl)-pyran-4-on werden in 75 ml Dichlormethan und 4,3 ml 3,4-Dihydro-2H-pyran aufgelöst. Man gibt 5 ml einer Lösung von HCI in Diethylether (5N) zu und lässt während 48 Stunden bei Raumtemperatur stehen. Man schüttelt einmal mit 50 ml gesättigter Natriumbicarbonatlösung und zweimal mit je 20 ml Wasser. Man trocknet die organische Phase über Magnesiumsulfat, filtriert und dampft zur Trockene ein. Als Rückstand erhält man 3-Benzyloxy-2-[pyridin-3-yl-(tetrahydropyran-2-yloxy)-methyl]-pyran-4-on als gelbes Harz. Diastereoisomerengemisch, $R_f$-Wert: 0,42 (Kieselgel 60, Essigester /Ethanol 9/1).

d) 3-Benzyloxy-1-(2-hydroxy-ethyl)-2-[pyridin-3-yl-(tetrahydropyran-2-yloxy)-methyl]-1*H*-pyridin-4-on: 4,3 g 3-Benzyloxy-2-[pyridin-3-yl-(tetrahydropyran-2-yloxy)-methyl]-pyran-4-on werden zusammen mit 5 ml Ethanolamin in 50 ml Ethanol während 18 Stunden unter Rückfluss gekocht, dann entfernt man das Ethanol am Rotationsverdampfer. Nach Aufarbeitung analog Beispiel 1 c erhält man 3-Benzyloxy-1-(2-hydroxy-ethyl)-2-[pyridin-3-yl-(tetrahydropyran-2-yloxy)-methyl]-1 *H*-pyridin-4-on als gelbes, zahes Harz. Diastereoisomerengemisch, $R_f$-Wert: 0,41 (Kieselgel 60, Dichlormethan/Ethanol 4/1).

e) 3-Benzyloxy-1-(2-hydroxy-ethyl)-2-(hydroxy-pyridin-3-yl-methyl)-1*H*-pyridin-4-on: 2,7 g 3-Benzyloxy-1-(2-hydroxy-ethyl)-2-[pyridin-3-yl-(tetrahydropyran-2-yloxy)-methyl]-1*H*-pyridin-4-on werden in einer Mischung aus 12 ml Ethanol und 12 ml 2N HCI während 3 Stunden unter Rückfluss gekocht. Man entfernt das Ethanol am Rotationsverdampfer und neutralisiert die zurückbleibende wässerige Lösung mit überschüssiger wässeriger Natriumbicarbonatlösung. Man extrahiert das ausfallende Produkt zweimal mit je 50 ml Essigester. Die organischen Phasen werden zweimal mit je 20 ml Wasser gewaschen. Man vereinigt die organischen Phasen, trocknet über Magnesiumsulfat filtriert und dampft das Filtrat am Rotationsverdampfer zur Trockene ein. Nach Umkristallisation aus Dichlormethan erhält man 3-Benzyloxy-1-(2-hydroxy-ethyl)-2-(hydroxy-pyridin-3-yl-methyl)-1*H*-pyridin-4-on als farblose Kristalle. Fp: 182-183°C.

Beispiel 7: 3-Hydroxy-2-(hydroxy-pyridin-3-yl-methyl)-1-methyl-1*H*-pyridin-4-on

1,34 g 3-Benzyloxy-2-(hydroxy-pyridin-3-yl-methyl)-1-methyl-1 *H*-pyridin-4-on werden in 30 ml Methanol über 0,13 g Palladiumkohle (5%) bei Normaldruck und Raumtemperatur hydriert bis zur Aufnahme von 1 mol $H_2$ pro mol Edukt. Nach Entfernen des Katalysators und Umkristallisation aus Methanol/Wasser erhält man 3-Hydroxy-2-(hydroxy-pyridin-3-yl-ethyl)-1-methyl-1*H*-pyridin-4-on als Monohydrat. Fp: 215-220°C.

Das Ausgangsmaterial kann zum Beispiel wie folgt hergestellt werden:

a) 3-Benzyloxy-1-methyl-2-[pyridin-3-yl-(tetrahydropyran-2-yloxy)-methyl]-1*H*-pyridin-4-on 2 g 3-Benzyloxy-2-[pyndin-3-yl-(tetrahydropyran-2-yloxy)-methyl]-pyran-4-on (Beispiel 6c) werden mit 13 ml Ethanol und 13 ml einer 30 proz Lösung von Methylamin in Ethanol versetzt. Man erwärmt eine Stunde lang auf 40°C und danach während 24 Stunden zum Rückfluss. Zum Aufarbeiten dampft man am Rotationsverdampfer zur Trockene ein Der Rückstand wird chromatographiert über 75 g Kieselgel. Als Eluent dient eine Mischung von Essigester und Ethanol im Verhältnis von 6/1 Man erhält so 3-Benzyloxy-1-methyl-2-[pyridin-3-yl-(tetrahydropyran-2-yloxy)-methyl]-1*H*-pyridin-4-on als gelben Schaum. Diastereoisomerengemisch, $R_f$-Wert: 0,09 (Kieselgel 60, Essigester/Ethanol 6/1).

b) 3-Benzyloxy-2-(hydroxy-pyridin-3-yl-methyl)-1-methyl-1*H*-pyndin-4-on 2,45 g 3-Benzyloxy-1-methyl-2-[pyridin-3-yl-(tetrahydropyran-2-yloxy)-methyl]-1 *H*-pyridin-4-on werden in einer Mischung aus 10 ml Ethanol und 10 ml HCl 2N während 20 Stunden bei Raumtemperatur stehen gelassen. Man entfernt das Ethanol am Rotationsverdampfer und neutralisiert die zurückbleibende wässerige Lösung mit überschüssiger wässeriger Natriumbicarbonatlösung. Man extrahiert das ausfallende Produkt zweimal mit je 50 ml Essigester. Die organischen Phasen werden zweimal mit je 20 ml Wasser gewaschen. Man vereinigt die organischen Phasen, trocknet über Magnesiumsulfat, filtriert und dampft das Filtrat am Rotationsverdampfer zur Trockene ein. Nach Umkristallisation aus Essigester erhält man 3-Benzyloxy-2-(hydroxy-pyridin-3-yl-methyl)-1-methyl-1*H*-pyridin-4-on. Hellbeige Kristalle, Fp: 169-174°C.

Beispiel 8: 4-[3-Hydroxy-2-(hydroxy-phenyl-methyl)-4-oxo-4*H*-pyridin-1-yl]-buttersäure

Aus 0,733 g 4-[3-Benzyloxy-2-(hydroxy-phenyl-methyl)-4-oxo-4*H*-pyridin-1-yl]-buttersäure erhält man durch katalytische Hydrierung analog Beispiel 1, nach Umkristallisation aus Methanol: 4-[3-Hydroxy-2-(hydroxy-phenyl-methyl)-4-oxo-4*H*-pyridin-1-yl]-buttersäure. Rötliche Kristalle Fp: 192-195°C.

Das Ausgangsmaterial kann zum Beispiel wie folgt hergestellt werden:

a) 4-{3-Benzyloxy-4-oxo-2-[phenyl-tetrahydropyran-2-yloxy)-methyl]-4*H*-pyridin-1-yl}-buttersäure: 2,5 g 3-Benzyloxy-2-[phenyl-(tetrahydropyran-2-yloxy)-methyl]-pyran-4-on (Beispiel 1 b) und 2,57 g 4-Aminobuttersäure [*AS-Nr.: 56-12-2*] werden in einer Mischung von 10 ml Tributylamin und 5 ml Ethylenglykol während 12 Stunden bei 120°C gerührt. Man giesst auf 100 ml Wasser und stellt mit verdünnter Salzsäure auf pH 5. Man extrahiert das ausfallende Produkt zweimal mit je 50 ml Essigester. Die organischen Phasen werden zweimal mit je 20 ml Wasser gewaschen. Man vereinigt die organischen Phasen, trocknet über Magnesiumsulfat filtriert und dampft das Filtrat am Rotationsverdampfer zur Trockene ein. Nach Umkristallisation aus Essigester erhält man 4-{3-Benzyloxy-4-oxo-2-[phenyl-tetrahydropyran-2-yloxy)-methyl]-4*H*-pyndin-1-yl}-buttersäure. Diastereoisomerengemisch, farblose Kristalle Fp: 205-210°C.

b) 4-[3-Benzyloxy-2-(hydroxy-phenyl-methyl)-4-oxo-4*H*-pyridin-1-yl]-buttersäure: 0,95 g 4-{3-Benzyloxy-4-oxo-2-[phenyl-tetrahydropyran-2-yloxy)-methyl]-4*H*-pyridin-1-yl}-buttersäure werden in einer Mischung von 10 ml Ethanol und 10 ml 2N HCl während 3 Stunden unter Rückfluss gekocht. Man versetzt mit 15 ml 2N NaOH und entfernt am Rotationsverdampfer das Ethanol. Die zurückbleibende Lösung wird mit 50 ml Wasser verdünnt und zweimal mit je 50 ml Essigester ausgezogen. Die Essigesterphasen werden verworfen. Die wässerige alkalische Phase wird mit 6 ml 2N HCl sauer gestellt und das ausfallende Produkt zweimal mit je 50 ml Essigester extrahiert. Die organischen Phasen werden zweimal mit je 20 ml Wasser gewaschen. Man vereinigt die organischen Phasen, trocknet über Magnesiumsulfat filtriert und dampft das Filtrat am Rotationsverdampfer zur Trockene ein. Nach Umkristallisation aus Methanol erhält man 4-[3-Benzyloxy-2-(hydroxy-phenyl-methyl)-4-oxo-4*H*-pyridin-1-yl]-buttersäure. Farblose Kristalle, Fp: 205-210°C.

Beispiel 9: 4-(2-Benzyl-3-hydroxy-4-oxo-4*H*-pyridin-1-yl)-buttersäure

1,2 g 4-[2-(Acetoxy-phenyl-methyl)-3-benzyloxy-4-oxo-4*H*-pyridin-1-yl]-buttersäure werden analog Beispiel 2 hydriert und man erhält nach Umkristallisation aus Ethanol 4-(2-Benzyl-3-hydroxy-4-oxo-4*H*-pyridin-1-yl)-buttersäure. Beige Kristalle, Fp: 170-172°C.

Das Ausgangsmaterial kann zum Beispiel wie folgt hergestellt werden:

a) 4-[2-(Acetoxy-phenyl-methyl)-3-benzyloxy-4-oxo-4*H*-pyridin-1-yl]-buttersäure: 1,28 g 4-[3-Benzyloxy-2-(hydroxy-phenyl-methyl)-4-oxo-4*H*-pyridin-1-yl]-buttersäure (Beispiel 8b) werden bei Raumtemperatur in 60 ml Dichlormethan suspendiert. Man gibt 1,1 ml Pyridin und 0,48 ml Acetanhydrid zu. Nach Zugabe von 0,05 g 4-Dimethylaminopyridin [*CAS-Nr.: 1122-58-3*] lässt man das Reaktionsgemisch 18 Stunden bei Raumtemperatur rühren. Zum Aufarbeiten wäscht man einmal mit 10 ml 2N HCl und dreimal mit 10 ml Wasser. Die organische Phase wird über Magnesiumsulfat getrocknet und filtriert. Das Filtrat wird am Rotationsverdampfer eingedampft. Man erhält 4-[2-(Acetoxy-phenyl-methyl)-3-benzyloxy-4-oxo-4*H*-pyridin-1-yl]-buttersäure. Beiger, amorpher Schaum. $R_f$-Wert 0,22 (Kieselgel 60, Dichlormethan / Ethanol 4/1).

Beispiel 10. 3-Hydroxy-1-[2-(2-hydroxy-ethoxy)-ethyl]-2-(hydroxy-phenyl-methyl)-1*H*-Pyridin-4-on

Aus 2,04 g 3-Benzyloxy-1-[2-(2-hydroxy-ethoxy)-ethyl]-2-(hydroxy-phenyl-methyl)-1H-pyridin-4-on erhalt man analog Beispiel 1: 3-Hydroxy-1-[2-(2-hydroxy-ethoxy)-ethyl]-2-(hydroxyphenyl-methyl)-1 *H*-pyridin-4-on. Farblose Knstalle Fp: 157-159°C.

Das Ausgangsmaterial kann zum Beispiel wie folgt hergestellt werden:

a) 3-Benzyloxy-1-[2-(2-hydroxy-ethoxy)-ethyl]-2-[phenyl-(tetrahydropyran-2-yloxy)-methyl]-1*H*-pyrindin-4-on: Aus 3-Benzyloxy-2-[phenyl-(tetrahydropyran-2-yloxy)-methyl]-pyran-4-on (Beispiel 1b) erhält man analog Beispiel 1c: 3-Benzyloxy-1-[2-(2-hydroxy-ethoxy)-ethyl]-2-[phenyl-(tetrahydropyran-2-yloxy)-methyl]-1 *H*-pyridin-4-on. Diastereoisomerengemisch, $R_f$-Wert 0,08 (Kieselgel 60, Essigester/Ethanol 6/1).

b) 3-Benzyloxy-1-[2-(2-hydroxy-ethoxy)-ethyl]-2-(hydroxy-phenyl-methyl)-1*H*-pyridin-4-on: Aus 2,8 g 3-Benzyloxy-1-[2-(2-hydroxy-ethoxy)-ethyl]-2-[phenyl-(tetrahydropyran-2-yloxy)-methyl]-1*H*-pyridin-4-on erhält man analog Beispiel 1 d: 3-Benzyloxy-1-[2-(2-hydroxy-ethoxy)-ethyl]-2-(hydroxy-phenyl-methyl)-1*H*-pyridin-4-on. Farblose Kristalle Fp: 151-152°C.

Beispiel 11: 3-Hydroxy-1-(2-hydroxy-ethyl)-2-[hydroxy-(4-hydroxy-3-methoxy-phenyl)-methyl]-1*H*-pyridin-4-on

1,1 g 3-Benzyloxy-2-[(4-benzyloxy-3-methoxy-phenyl)-hydroxy-methyl]-1-(2-hydroxy-ethyl)-1 *H*-pyridin-4-on werden in 50 ml Methanol über 0,2 g Palladiumkohle (5%) bei Normaldruck und Raumtemperatur hydriert bis zur Aufnahme von 2 mol $H_2$ pro mol Edukt. Nach Entfernen des Katalysators und Umkristallisation aus Ethanol erhält man 3-Hydroxy-1-(2-hydroxyethyl)-2-[hydroxy-(4-hydroxy-3-methoxy-phenyl)-methyl]-1*H*-pyridin-4-on. Fp: 179-190°C

Das Ausgangsmaterial kann zum Beispiel wie folgt hergestellt werden:

a) 2-[(4-Benzyloxy-3-methoxy-phenyl)-hydroxy-methyl]-3-hydroxy-pyran-4-on: Aus 3,36 g Pyromeconsäure und 7,27 g 3-Methoxy-4-benzyloxy-benzaldehyd erhalt man analog Beispiel 3a: 2-[(4-Benzyloxy-3-methoxy-phenyl)-hydroxy-methyl]-3-hydroxy-pyran-4-on. Fp: ab 142°C Kristallumwandlung, ab 190°C Zersetzung.

b) 3-Benzyloxy-2-[(4-benzyloxy-3-methoxy-phenyl)-hydroxy-methyl]-pyran-4-on: Aus 3,74 g 2-[(4-Benzyloxy-3-methoxy-phenyl)-hydroxy-methyl]-3-hydroxy-pyran-4-on erhalt man analog Beispiel 1 a: 3-Benzyloxy-2-[(4-benzyloxy-3-methoxy-phenyl)-hydroxy-methyl]-pyran-4-on. Fp: 116-117°C.

c) 3-Benzyloxy-2-[(4-benzyloxy-3-methoxy-phenyl)-(tetrahydropyran-2-yloxy)-methyl]-pyran-4-on: Aus 3-Benzyloxy-2-[(4-benzyloxy-3-methoxy-phenyl)-hydroxy-methyl]-pyran-4-on erhält man analog Beispiel 6c: 3-Benzyloxy-2-[(4-benzyloxy-3-methoxy-phenyl)-(tetrahydropyran-2-yloxy)-methyl]-pyran-4-on. Diastereoisomerengemisch, $R_f$-Wert 0,8 (Kieselgel 60, Essigester/Dichlormethan 1/1).

d) 3-Benzyloxy-2-[(4-benzyloxy-3-methoxy-phenyl)-(tetrahydropyran-2-yloxy)-methyl]-1-(2-hydroxy-ethyl)-1*H*-pyridin-4-on: Aus 3-Benzyloxy-2-[(4-benzyloxy-3-methoxy-phenyl)-(tetrahydropyran-2-yloxy)-methyl]-pyran-4-on erhält man analog Beispiel 1 c: 3-Benzyloxy-2-[(4-benzyloxy-3-methoxy-phenyl)-(tetrahydropyran-2-yloxy)-methyl]-1-(2-hydroxy-ethyl)-1*H*-pyridin-4-on. Diastereoisomerengemisch, $R_f$-Wert 0,18 (Kieselgel 60, Essigester/Ethanol

9/1).

e) 3-Benzyloxy-2-[(4-benzyloxy-3-methoxy-phenyl)-hydroxy-methyl]-1-(2-hydroxy-ethyl)-1*H*-pyridin-4-on: Aus 1,58 g 3-Benzyloxy-2-[(4-benzyloxy-3-methoxy-phenyl)-(tetrahydropyran-2-yloxy)-methyl]-1-(2-hydroxy-ethyl)-1*H*-pyridin-4-on erhält man analog Beispiel 6b nach Umkristallisation aus Essigester: 3-Benzyloxy-2-[(4-benzyloxy-3-methoxy-phenyl)-hydroxymethyl]-1-(2-hydroxy-ethyl)-1*H*-pyridin-4-on. Farblose Kristalle, Fp:172-173°C.

Beispiel 12: 3-Hydroxy-1-(2-hydroxy-ethyl)-6-hydroxymethyl-2-(hydroxy-phenyl-methyl)-1*H*-pyridin-4-on

Man hydriert 0,834 g 3-Benzyloxy-1-(2-hydroxy-ethyl)-6-hydroxymethyl-2-(hydroxy-phenylmethyl)-1 *H*-pyridin-4-on analog Beispiel 1 und erhält 3-Hydroxy-1-(2-hydroxy-ethyl)-6-hydroxymethyl-2-(hydroxy-phenyl-methyl)-1 *H*-pyridin-4-on als amorphen Schaum. $R_f$-Wert 0,15 (Kieselgel 60, Essigester/Ethanol 6/1).

Das Ausgangsmaterial kann zum Beispiel wie folgt hergestellt werden

a) 3-Hydroxy-6-hydroxymethyl-2-(hydroxy-phenyl-methyl)-pyran-4-on Aus 2,82 g Kojisaure [*CAS-Nr.: 501-30-4*] und 2,33 g Benzaldehyd erhalt man analog Beispiel 3a. 3-Hydroxy-6-hydroxymethyl-2-(hydroxy-phenyl-methyl)-pyran-4-on. Fp: 148-149,5°C.

b) 3-Benzyloxy-6-hydroxymethyl-2-(hydroxy-phenyl-methyl)-pyran-4-on. Aus 3-Hydroxy-6-hydroxymethyl-2-(hydroxy-phenyl-methyl)-pyran-4-on erhält man analog Beispiel 6b: 3-Benzyloxy-6-hydroxymethyl-2-(hydroxy-phenyl-methyl)-pyran-4-on. Gelbes Harz, $R_f$-Wert 0,57 (Kieselgel 60, Essigester/Ethanol 9/1).

c) 3-Benzyloxy-6-methoxymethoxymethyl-2-(methoxymethoxy-phenyl-methyl)-pyran-4-on: 7,0 g 3-Benzyloxy-6-hydroxymethyl-2-(hydroxy-phenyl-methyl)-pyran-4-on werden mit 15 ml Dimethoxymethan und 150 ml Dichlormethan aufgelöst. Man setzt 0,2 g *p*-Toluolsulfonsäure zu und kocht die Lösung über eine mit Molekularsieb (5 Å) gefüllte Extraktionshülse während 48 Stunden unter Rückfluss. Man lässt abkühlen und wäscht einmal mit 20 ml gesättigter Natriumbicarbonatlösung und zweimal mit je 20 ml Wasser. Man trocknet über Magnesiumsulfat, filtriert und dampft zur Trockene ein. Der Rückstand besteht aus ca. 80%igem 3-Benzyloxy-6-methoxymethoxymethyl-2-(methoxymethoxy-phenyl-methyl)-pyran-4-on. $R_f$-Wert 0,55 (Kieselgel 60, Essigester/Ethanol 98/2).

d) 3-Benzyloxy-1-(2-hydroxy-ethyl)-6-methoxymethoxymethyl-2-(methoxymethoxy-phenylmethyl)-1*H*-pyridin-4-on: Aus 3-Benzyloxy-6-methoxymethoxymethyl-2-(methoxymethoxyphenyl-methyl)-pyran-4-on erhält man analog Beispiel 1 c: 3-Benzyloxy-1-(2-hydroxy-ethyl)-6-methoxymethoxymethyl-2-(methoxymethoxy-phenyl-methyl)-1*H*-pyridin-4-on. Braunes Harz, $R_f$-Wert 0,36 (Kieselgel 60, Essigester/Ethanol 6/1).

e) 3-Benzyloxy-1-(2-hydroxy-ethyl)-6-hydroxymethyl-2-(hydroxy-phenyl-methyl)-1*H*-pyridin-4-on: Aus 2,1 g 3-Benzyloxy-1-(2-hydroxy-ethyl)-6-methoxymethoxymethyl-2-(methoxymethoxy-phenyl-methyl)-1*H* pyridin-4-on erhält man analog Beispiel 6e, nach Umkristallisation aus Dichlormethan: 3-Benzyloxy-1-(2-hydroxy-ethyl)-6-hydroxymethyl-2-(hydroxy-phenylmethyl)-1*H*-pyridin-4-on. Fp: ab 166°C Kristallumwandlung, dann 189-191°C.

Beispiel 13: 1-(2,3-Dihydroxy-propyl)-3-hydroxy-2-(hydroxy-phenyl-methyl)-1*H*-pyridin-4-on

Man hydriert 1,63 g 3-Benzyloxy-1-(2,3-dihydroxy-propyl)-2-(hydroxy-phenyl-methyl)-1 *H*-pyridin-4-on analog Beispiel 1 und erhalt 1-(2,3-Dihydroxy-propyl)-3-hydroxy-2-(hydroxyphenyl-methyl)-1 *H*-pyridin-4-on als farblosen, amorphen Schaum. Diastereoisomerengemisch, $R_f$-Wert 0,15 (Kieselgel 60, Dichlormethan/Ethanol 4/1).

Das Ausgangsmaterial kann zum Beispiel wie folgt hergestellt werden

a) 3-Benzyloxy-1-(2,3-dihydroxy-propyl)-2-[phenyl-(tetrahydropyran-2-yloxy)methyl]-1*H*-pyridin-4-on: 3,93 g 3-Benzyloxy-2-[phenyl-(tetrahydropyran-2-yloxy)-methyl]-pyran-4-on (Beispiel 1 b) und 3 g 3-Amino-1,2-propandiol [*CAS-Nr.: 616-30-8]* werden zusammen während 17 Stunden bei 110°C gerührt. Man versetzt die Schmelze mit 50 ml Wasser und 20 ml Essigester. Bei Raumtemperatur kristallisiert ein blassgelbes Produkt in feinen Nädelchen aus. Man filtriert und wäscht mit Wasser und Essigester. Nach dem Trocknen erhält man 3-Benzyloxy-1-(2,3-dihydroxy-propyl)-2-[phenyl-(tetrahydropyran-2-yloxy)methyl]-1*H*-pyridin-4-on als Diastereoisomerengemisch. Fp: 198-215°C. Das obige Filtrat wird im Scheidetrichter getrennt. Die organische Phase wird zweimal mit Wasser gewaschen und am Rotationsverdampfer zur Trockene eingedampft. Der Rückstand wird aus 20 ml Essigester umkristallisiert und man erhält nach dem Trocknen weiteres 3-Benzyloxy-1-(2,3-dihydroxy-propyl)-2-[phenyl-(tetrahydropyran-2-yloxy)methyl]-1 *H*-pyridin-4-on Gelbe würfelförmige Kristalle. Diastereoisomerengemisch. Fp: 190-206°C.

b) 3-Benzyloxy-1-(2,3-dihydroxy-propyl)-2-(hydroxy-phenyl-methyl)-1*H*-pyridin-4-on: Aus 2,1 g 3-Benzyloxy-1-(2,3-dihydroxy-propyl)-2-[phenyl-(tetrahydropyran-2-yloxy) methyl]- 1*H*-pyridin-4-on erhält man analog Beispiel 6e, nach Chromatographie: 3-Benzyloxy-1-(2,3-dihydroxy-propyl)-2-(hydroxy-phenyl-methyl)-1 *H*-pyridin-4-on. Farbloser Schaum, Diastereoisomerengemisch. $R_f$-Wert 0,22 (Kieselgel 60, Dichlormethan/Ethanol 4/1).

Beispiel 14: 2-Benzyl-1-(2,3-dihydroxy-propyl)-3-hydroxy-1*H*-pyridin-4-on

Aus 0,6 g 3-Benzyloxy-1-(2,3-dihydroxy-propyl)-2-(hydroxy-phenyl-methyl)-1*H*-pyridin-4-on (Beispiel 13b) erhält man analog Beispiel 2: 2-Benzyl-1-(2,3-dihydroxy-propyl)-3-hydroxy-1*H*-pyridin-4-on. Kristallisation aus Methanol, Fp: 185-186°C.

Beispiel 15. N-(2-Hydroxy-ethyl)-4-{hydroxy-[3-hydroxy-1-(2-hydroxy-ethyl)-4-oxo-1,4-dihydro-pyridin-2-yl]-methyl}-benzamid

Aus 1,18 g 4-{[3-Benzyloxy-1-(2-hydroxy-ethyl)-4-oxo-1,4-dihydro-pyridin-2-yl]-hydroxymethyl}-N-(2-hydroxy-ethyl)-benzamid Monohydrat erhält man analog Beispiel 1: N-(2-Hydroxy-ethyl)-4-{hydroxy-[3-hydroxy-1-(2-hydroxy-ethyl)-4-oxo-1,4-dihydro-pyridin-2-yl]-methyl}-benzamid. Rosafarbener, amorpher Schaum, $R_f$-Wert 0,05 (Kieselgel 60, Dichlormethan/Ethanol 4/1).

Das Ausgangsmaterial kann zum Beispiel wie folgt hergestellt werden:

a) 4-[Hydroxy-(3-hydroxy-4-oxo-4*H*-pyran-2-yl)-methyl]-benzoesäure: 4,48 Pyromeconsäure und 7,51 g 4-Carboxybenzaldehyd [*CAS-Nr.: 619-66-9*] werden in 8 ml Wasser, 43 ml 2N NaOH und 15 ml Methanol aufgelöst. Die braune Lösung hat pH 10 Man lässt bei Raumtemperatur 18 Stunden stehen. Man entfernt das Methanol am Rotationsverdampfer und neutralisiert die zurückbleibende wässerige Lösung mit 43 ml 2N HCl. Das ausfallende Produkt wird abfiltriert und getrocknet. Man erhält 4-[Hydroxy-(3-hydroxy-4-oxo-4*H*-pyran-2-yl)-methyl]-benzoesäure. Fp: ab 184°C unter Zersetzung.

b) 4-[(3-Benzyloxy-4-oxo-4*H*-pyran-2-yl)-hydroxy-methyl]benzoesäurebenzylester: 10,5 g 4-[Hydroxy-(3-hydroxy-4-oxo-4*H*-pyran-2-yl)-methyl]-benzoesäure und 22 g pulverisiertes Kaliumcarbonat werden in 120 ml Dimethylformamid bei Raumtemperatur gerührt. Man gibt 14 g Benzylbromid zu und rührt die Suspension 18 Stunden bei Raumtemperatur. Zum Aufarbeiten giesst man auf 500 ml Wasser und extrahiert zweimal mit je 200 ml Essigester. Die organischen Phasen werden viermal mit je 50 ml Wasser gewaschen, vereinigt und über Magnesiumsulfat getrocknet. Man filtnert vom Trockenmittel ab und dampft das Filtrat am Rotationsverdampfer ein. Nach Chromatographie erhält man 4-[(3-Benzyloxy-4-oxo-4*H*-pyran-2-yl)-hydroxy-methyl]benzoesäurebenzylester. Gelbes Oel, $R_f$-Wert 0,19 (Kieselgel 60, Hexan/Dichlormethan/Essigester 1/1/1).

c) 4-[(3-Benzyloxy-4-oxo-4*H*-pyran-2-yl)-(tetrahydropyran-2-yloxy)-methyl]-benzoesäurebenzylester: Aus 4-[(3-Benzyloxy-4-oxo-4H-pyran-2-yl)-hydroxy-methyl]-benzoesäurebenzylester erhält man analog Beispiel 6c: 4-[(3-Benzyloxy-4-oxo-4*H*-pyran-2-yl)-(tetrahydropyran-2-yloxy)-methyl]-benzoesäurebenzylester. Gelbes Harz, $R_f$-Wert 0,57 (Kieselgel 60, Hexan/Dichlormethan/Essigester 1/1/1).

d) 4-{[3-Benzyloxy-1(2-hydroxy-ethyl)-4-oxo-1,4-dihydro-pyridin-2-yl]-(tetrahydropyran-2-yl-oxy)-methyl}-N-(2-hydroxy-ethyl)-benzamid: Aus 4-[(3-Benzyloxy-4-oxo-4H-pyran-2-yl)-(tetrahydropyran-2-yloxy)-methyl]-benzoesaurebenzylester erhält man bei der Umsetzung analog Beispiel 1 c: 4-{[3-Benzyloxy-1 (2-hydroxy-ethyl)-4-oxo-1,4-dihydro-pyridin-2-yl]-(tetrahydropyran-2-yloxy)-methyl}-N-(2-hydroxy-ethyl)-benzamid als Diastereoisomerengemisch. Gelbes Harz, $R_f$-Wert 0,19 (Kieselgel 60, Dichlormethan/Ethanol 4/1).

e) 4-{[3-Benzyloxy-1-(2-hydroxy-ethyl)-4-oxo-1,4-dihydro-pyridin-2-yl]-hydroxy-methyl}-N-(2-hydroxy-ethyl)-benzamid: 2,25 g 4-{[3-Benzyloxy-1 (2-hydroxy-ethyl)-4-oxo-1,4-dihydro-pyridin-2-yl]-(tetrahydropyran-2-yloxy)-methyl}-N-(2-hydroxy-ethyl)-benzamid werden in 15 ml Methanol und 5,5 ml 2N HCl während 90 Minuten unter Rückfluss gekocht. Man entfernt das Methanol am Rotationsverdampfer und neutralisiert den Rückstand mit überschüssiger wässeriger Natriumbicarbonatlösung. Man extrahiert das ausfallende Produkt zweimal mit je 50 ml Essigester. Die organischen Phasen werden zweimal mit je 20 ml Wasser gewaschen. Man vereinigt die organischen Phasen, trocknet über Magnesiumsulfat filtriert und dampft das Filtrat am Rotationsverdampfer zur Trockene ein. Nach Umkristallisation aus Wasser erhält man 4-{ [3-Benzyloxy-1-(2-hydroxy-ethyl)-4-oxo-1,4-dihydro-pyridin-2-yl]-hydroxy-methyl}-N-(2-hydroxy-ethyl)-benzamid als Monohydrat. Fp: ab 108°C unter Zersetzung bis 227°C.

Beispiel 16: [3-Hydroxy-2-(hydroxy-phenyl-methyl)-4-oxo-4*H*-pyridin-1-yl]-essigsäure

Aus 1,02 g [3-Benzyloxy-2-(hydroxy-phenyl-methyl)-4-oxo-4*H*-pyridin-1-yl]-essigsäure-Natriumsalz-Tetrahydrat erhält man durch katalytische Hydrierung analog Beispiel 1, nach Umkristallisation aus Methanol: [3-Hydroxy-2-(hydroxy-phenyl-methyl)-4-oxo-4*H*-pyridin-1-yl]-essigsäure als Natriumsalz-Dihydrat. Hellbeige Kristalle. Fp: ab120°C Kristallwasserverlust, ab 300°C Zersetzung.

Das Ausgangsmaterial kann zum Beispiel wie folgt hergestellt werden:

a) {3-Benzyloxy-4-oxo-2-[phenyl-(tetrahydropyran-2-yloxy)-methyl]-4*H*-pyridin-1-yl}-essigsäureethylester: 2,5 g 3-Benzyloxy-2-[phenyl-(tetrahydropyran-2-yloxy)-methyl]-pyran-4-on (Beispiel 1 b) und 3,5 g Glycinethylester-hydrochlorid [*CAS-Nr.: 623-33-6*] werden in einer Mischung von 25 ml Tributylamin und 5 ml Ethylenglykol während 40 Stunden bei 110°C gerührt. Man versetzt mit 200 ml Wasser und stellt mit verdünnter Salzsaure auf pH 4 Man extrahiert zweimal mit je 100 ml Essigester. Die organischen Phasen werden dreimal mit je 50 ml Wasser gewaschen, über Magnesiumsulfat getrocknet, filtriert und eingedampft Nach Chromatographie erhält man {3-Benzyloxy-4-oxo-2-[phenyl-(tetrahydropyran-2-yloxy)-methyl]-4*H*-pyridin-1-yl}-essigsäureethylester. Gelbes Harz, $R_f$-Wert 0,25 (Kieselgel 60, Dichlormethan/Ethanol 9/1).

b) [3-Benzyloxy-2-(hydroxy-phenyl-methyl)-4-oxo-4*H*-pyridin-1-yl]-essigsäureethylester: Aus 1,3 g {3-Benzyloxy-4-oxo-2-[phenyl-(tetrahydropyran-2-yloxy)-methyl]-4H-pyndin-1-yl}-essigsäureethylester erhält man nach Behandlung mit 2N HCl und Ethanol und Chromatographie analog Beispiel 6e den [3-Benzyloxy-2-(hydroxy-phenyl-methyl)-4-oxo-4*H*-pyridin-1-yl]-essigsäureethylester als gelbliches Harz. $R_f$-Wert 0,4 (Kieselgel 60, Dichlormethan/ Ethanol 9/1).

c) [3-Benzyloxy-2-(hydroxy-phenyl-methyl)-4-oxo-4*H*-pyridin-1-yl]-essigsäure: 1,2 g [3-Benzyloxy-2-(hydroxy-phenyl-methyl)-4-oxo-4*H*-pyridin-1-yl]-essigsäureethylester werden bei Raumtemperatur mit 5 ml 2N NaOH und 1 ml Ethanol aufgelöst. Man lässt 20 Stunden bei Raumtemperatur stehen und kühlt dann eine Stunde im Eisbad ab Man filtriert ab und wäscht mit wenig Eiswasser. Nach Trocknen bei Raumtemperatur erhält man [3-Benzyloxy-2-(hydroxy-phenyl-methyl)-4-oxo-4*H*-pyridin-1-yl]-essigsäure als Natriumsalz-Tetrahydrat. Farblose Blättchen. Fp: nach Kristallumwandlung bei 85-95°C, 190-195°C.

Beispiel 17: 4-{2-[(4-Fluor-phenyl)-hydroxy-methyl]-3-hydroxy-4-oxo-4*H*-pyridin-1-yl}-buttersäure

Aus 1,9 g 4-{3-Benzyloxy-2-[(4-fluor-phenyl)-hydroxy-methyl]-4-oxo-4*H*-pyridin-1-yl}-buttersäure erhält man durch Hydrierung analog Beispiel 1 und Umkristallisation aus Methanol: 4-{2-[(4-Fluor-phenyl)-hydroxy-methyl]-3-hydroxy-4-oxo-4*H*-pyridin-1-yl}-buttersäure Blassrosa Kristalle, Fp: 177,5- 178°C.

Das Ausgangsmaterial kann zum Beispiel wie folgt hergestellt werden:

a) 4-{3-Benzyloxy-2-[(4-fluor-phenyl)-(tetrahydropyran-2-yloxy)-methyl]-4-oxo-4*H*-pyridin-1-yl}-buttersäure: Aus 4,1 g 3-Benzyloxy-2-[(4-fluorphenyl)-(tetrahydropyran-2-yloxy)-methyl]-pyran-4-on (Beispiel 3c) erhält man durch Umsetzung mit 4-Aminobuttersäure analog Beispiel 8a. 4-{3-Benzyloxy-2-[(4-fluor-phenyl)-(tetrahydropyran-2-yloxy)-methyl]-4-oxo-*4H*-pyridin-1-yl}-buttersaure als Diastereoisomerengemisch. Beige Kristalle, Fp: 187-190°C

b) 4-{3-Benzyloxy-2-[(4-fluor-phenyl)-hydroxy-methyl]-4-oxo-4*H*-pyridin-1-yl}-buttersaure Aus 1,0 g 4-{3-Benzyloxy-2-[(4-fluor-phenyl)-(tetrahydropyran-2-yloxy)-methyl]-4-oxo-4*H*-pyridin-1-yl}-buttersäure erhält man analog Beispiel 8b: 4-{3-Benzyloxy-2-[(4-fluor-phenyl)-hydroxy-methyl]-4-oxo-4*H*-pyridin-1-yl}-buttersaure. Farblose Kristalle. Fp 215-220°C

Beispiel 18: {2-[(4-Fluor-phenyl)-hydroxy-methyl]-3-hydroxy-4-oxo-4*H*-pyridin-1-yl}-essigsäure

Aus 1,47 g {3-Benzyloxy-2-[(4-fluor-phenyl)-hydroxy-methyl]-4-oxo-4*H*-pyridin-1-yl}-essigsäure erhält man durch Hydrierung analog Beispiel 1 und Umkristallisation aus Essigester: {2-[(4-Fluor-phenyl)-hydroxy-methyl]-3-hydroxy-4-oxo-4*H*-pyridin-1-yl}-essigsäure. Farblose Kristalle, Fp: 123-127°C.

Das Ausgangsmaterial kann zum Beispiel wie folgt hergestellt werden:

a) {3-Benzyloxy-2-[(4-fluor-phenyl)-(tetrahydropyran-2-yloxy)-methyl]-4-oxo-4*H*-pyridin-1-yl}-essigsäure: 5,54 g 3-Benzyloxy-2-[(4-fluorphenyl)-(tetrahydropyran-2-yloxy)-methyl]-pyran-4-on (Beispiel 3c) und 4,5 g Glycin werden in einer Mischung aus 55 ml Diethylenglykol und 30 ml Tri-*n*-butylamin während 48 Stunden bei 110°C gerührt.

Man verdünnt mit 100 ml Wasser und 75 ml 2N HCl. Man extrahiert dreimal mit je 100 ml Essigester. Die organischen Phasen werden dreimal mit je 50 ml Wasser gewaschen. Man vereinigt die organischen Phasen, trocknet über Magnesiumsulfat und filtriert. Man dampft das Filtrat am Rotationsverdampfer zur Trockene ein. Als Rückstand bleibt rohe {3-Benzyloxy-2-[(4-fluor-phenyl)-(tetrahydropyran-2-yloxy)-methyl]-4-oxo-4*H*-pyridin-1-yl}-essigsäure als Diastereoisomerengemisch. $R_f$-Wert 0,12 (Kieselgel 60, Dichlormethan/Ethanol 4/1).

b) {3-Benzyloxy-2-[(4-fluor-phenyl)-hydroxy-methyl]-4-oxo-4*H*-pyridin-1-yl}-essigsäure Aus 6,0 g roher {3-Benzyloxy-2-[(4-fluor-phenyl)-(tetrahydropyran-2-yloxy)-methyl]-4-oxo-4*H*-pyndin-1-yl}-essigsäure erhält man nach Behandlung mit verdünnter Salzsäure analog Beispiel 8b: {3-Benzyloxy-2-[(4-fluor-phenyl)-hydroxy-methyl]-4-oxo-4*H*-pyridin-1-yl}-essigsäure. Farblose Kristalle. Fp: 147-150°C.

Beispiel 19: N-{2-[3-Hydroxy-2-(hydroxy-phenyl-methyl)-4-oxo-4*H*-pyridin-1-yl]-ethyl}-acetamid

0,389 g N-{2-[3-Benzyloxy-2-(hydroxy-phenyl-methyl)-4-oxo-4*H*-pyridin-1-yl]-ethyl}-acetamid werden analog Beispiel 1 hydriert und nach Knstallisation aus Ether erhalt man N-{2-[3-Hydroxy-2-(hydroxy-phenyl-methyl)-4-oxo-4*H*-pyridin-1-yl]-ethyl}-acetamid. Beige Kristalle, Fp: 187,5-190°C

Das Ausgangsmaterial kann zum Beispiel wie folgt hergestellt werden:

a) 1-(2-Amino-ethyl)-3-benzyloxy-2-[phenyl-(tetrahydropyran-2-yloxy)-methyl]-1*H*-pyridin-4-on: 6,5 g 3-Benzyloxy-2-[phenyl-(tetrahydropyran-2-yloxy)-methyl]-pyran-4-on (Beispiel 1 b) werden zusammen mit 13 ml Ethylendiamin in 65 ml Ethanol während 8 Stunden unter Rückfluss gekocht. Nach Aufarbeitung analog Beispiel 1c erhält man 1-(2-Amino-ethyl)-3-benzyloxy-2-[phenyl-(tetrahydropyran-2-yloxy)-methyl]-1*H*-pyridin-4-on als Diastereoisomerengemisch. $R_f$-Wert 0,2 (Kieselgel 60, Dichlormethan/Ethanol 4/1).

b) N-(2-{3-Benzyloxy-4-oxo-2-[phenyl-(tetrahydropyran-2-yloxy)-methyl]-4-4*H*-pyridin-1-yl}-ethyl)-acetamid: 2,6 g Rohes 1-(2-Amino-ethyl)-3-benzyloxy-2-[phenyl-(tetrahydropyran-2-yloxy)-methyl]-1*H*-pyridin-4-on werden in 50 ml Dichlormethan gelost. Man setzt bei Raumtemperatur 3,5 ml Hünig-Base [*CAS-Nr.: 7087-68-5*] und anschliessend 0,85 ml Acetanhydrid zu. Man lasst die Lösung bei Raumtemperatur 18 Stunden stehen. Man wäscht einmal mit 10 ml 1N HCl und zweimal mit je 10 ml Wasser. Die organische Phase wird über Magnesiumsulfat getrocknet und filtriert. Man dampft das Filtrat am Rotationsverdampfer zur Trockene ein. Der Rückstand wird über Kieselgel chromatographiert. Als Eluent dient eine Mischung von Essigester und Ethanol im Verhältnis von 6/1). Man erhält N-(2-{3-Benzyloxy-4-oxo-2-[phenyl-(tetrahydropyran-2-yloxy)-methyl]-4-4*H*-pyridin-1-yl}-ethyl)-acetamid als braunes Harz $R_f$-Wert: 0,5 (Kieselgel 60, Dichlormethan/Ethanol 4/1).

c) N-{2-[3-Benzyloxy-2-(hyrdroxy-phenyl-methyl)-4-oxo-4*H*-pyridin-1-yl]-ethyl}-acetamid: Man löst 0,33 g N-(2-{3-Benzyloxy-4-oxo-2-[phenyl-(tetrahydropyran-2-yloxy)-methyl]-4-4*H*-pyridin-1-yl}-ethyl)-acetamid in 3,5 ml Methanol und setzt 0,69 ml 2N HCl zu Man erwärmt die Lösung während 18 Stunden auf 40°C. Man versetzt mit einem Ueberschuss an wässeriger Natriumbicarbonatlösung und extrahiert dreimal mit je 50 ml Essigester Man wäscht die Extrakte dreimal mit je 10 ml Wasser, vereinigt die organischen Phasen und trocknet über Magnesiumsulfat Man filtriert und dampft das Filtrat am Rotationsverdampfer zur Trockene ein. Der Rückstand wird aus Essigester kristallisiert Man erhalt N-{2-[3-Benzyloxy-2-(hydroxy-phenyl-methyl)-4-oxo-4*H*-pyridin-1-yl]-ethyl}-acetamid Beige Kristalle, Fp 180-182°C.

Beispiel 20: 1-(2-Dimethylamino-ethyl)-3-hydroxy-2-(hydroxy-phenyl-methyl)-1*H*-pyridin-4-on

1,97 g 3-Benzyloxy-1-(2-dimethylamino-ethyl)-2-(hydroxy-phenyl-methyl)-1*H*-pyridin-4-on werden hydriert analog Beispiel 1 und man erhält nach Kristallisation aus Methanol 1-(2-Dimethylamino-ethyl)-3-hydroxy-2-(hydroxy-phenyl-methyl)-1*H*-pyndin-4-on. Farblose Kristalle, Fp: 186-188°C.

Das Ausgangsmaterial kann zum Beispiel wie folgt hergestellt werden:

a) 3-Benzyloxy-1-(2-dimethylamino-ethyl)-2-[phenyl-(tetrahydropyran-2-yloxy)-methyl]-1*H*-pyridin-4-on: Aus 3-Benzyloxy-2-[phenyl-(tetrahydropyran-2-yloxy)-methyl]-pyran-4-on (Beispiel 1 b) erhält man durch Umsetzung mit 2-Dimethylamino-ethylamin [*CAS-Nr* · 1*08*-00-9] analog Beispiel 18a das 3-Benryloxy-1-(2-dimethylamino-ethyl)-2-[phenyl-(tetrahydropyran-2-yloxy)-methyl]-1*H*-pyridin-4-on als Diastereoisomerengemisch. Braunes Harz. $R_f$-Wert: 0,44 (Kieselgel 60, Dichlormethan/Ethanol 4/1).

b) 3-Benzyloxy-1-(2-dimethylamino-ethyl)-2-(hydroxy-phenyl-methyl)-1*H*-pyridin-4-on: Aus 5,2 g 3-Benzyloxy-1-(2-dimethylamino-ethyl)-2-[phenyl-(tetrahydropyran-2-yloxy)-methyl]-1 *H*-pyridin-4-on erhält man analog Beispiel 6e, nach Chromatographie: 3-Benzyloxy-1-(2-dimethylamino-ethyl)-2-(hydroxy-phenyl-methyl)-1 *H*-pyridin-4-on. Beiger, amorpher Schaum. $R_f$-Wert: 0,16 (Kieselgel 60, Dichlormethan/Ethanol 4/1).

Beispiel 21: 2-(Hydroxy-phenyl-methyl)-pyridin-3,4-diol

Aus 0,465 g 3-Benzyloxy-2-(hydroxy-phenyl-methyl)-pyridin-4-ol erhält man durch Hydrierung analog Beispiel 1: 2-(Hydroxy-phenyl-methyl)-pyridin-3,4-diol. Farblose Kristalle. Fp: ab 200°C unter Zersetzung.

Das Ausgangsmaterial kann zum Beispiel wie folgt hergestellt werden:

a) 3-Benzyloxy-2-[phenyl-(tetrahydropyran-2-yloxy)-methyl]-pyridin-4-ol 5 g 3-Benzyloxy-2-[phenyl-(tetrahydropyran-2-yloxy)-methyl]-pyran-4-on (Beispiel 1 b) werden bei Raumtemperatur mit 100 ml einer Losung von Ammoniak in Methanol (6 M) ubergossen und im verschlossenen Kolben bei Raumtemperatur während 6 Tagen stehen gelassen. Man dampft am Vakuum zur Trockene ein und versetzt den Rückstand mit 100 ml Ether. Die so entstandene Kristallsuspension wird abfiltnert, das Produkt mit Ether gewaschen und getrocknet. Man erhält 3-Benzyloxy-2-[phenyl-(tetrahydropyran-2-yloxy)-methyl]-pyridin-4-ol als Diastereoisomerengemisch. Gelbliche Kristalle, Fp: 192-192,5°C.

b) 3-Benzyloxy-2-(hydroxy-phenyl-methyl)-pyridin-4-ol: Aus 0,783 g 3,4 g 3-Benzyloxy-2-[phenyl-(tetrahydropyran-2-yloxy)-methyl]-pyridin-4-ol erhält man durch Behandlung analog Beispiel 1d: 3-Benzyloxy-2-(hydroxy-phenyl-methyl)-pyridin-4-ol. Farblose Kristalle, Fp: 215-218°C unter Zersetzung, Kristallumwandlung ab 205°C.

Beispiel 22: 2-[Hydroxy-(4-hydroxy-3-methoxy-phenyl)-methyl]-pyridin-3,4-diol

0,9 g 3-Benzyloxy-2-[(4-benzyloxy-3-methoxy-phenyl)-hydroxy-methyl]-pyridin-4-ol werden in 100 ml Methanol gelöst und in Gegenwart von 0,18 g Palladiumkohle (5%) bei Raumtemperatur bis zur Aufnahme von 2 mol $H_2$ pro mol Edukt hydriert. Man filtriert vom Katalysator ab und dampft das Filtrat ein. Nach Umkristallisation aus Methanol erhält man 2-[Hydroxy-(4-hydroxy-3-methoxy-phenyl)-methyl]-pyridin-3,4-diol. Farblose Kristalle, Fp: ab 210°C unter Zersetzung.

Das Ausgangsmaterial kann zum Beispiel wie folgt hergestellt werden:

a) 3-Benzyloxy-2-[(4-benzyloxy-3-methoxy-phenyl)-(tetrahydropyran-2-yloxy)-methyl]-pyridin-4-ol: 3-Benzyloxy-2-[(4-benzyloxy-3-methoxy-phenyl)-(tetrahydropyran-2-yloxy)-methyl]-pyran-4-on (Beispiel 11 c) wird analog Beispiel 21 a umgesetzt. Man erhält 3-Benzyloxy-2-[(4-benzyloxy-3-methoxy-phenyl)-(tetrahydropyran-2-yloxy)-methyl]-pyridin-4-ol als gelbes Harz. Diastereoisomerengemisch. $R_f$-Wert: 0,35 (Kieselgel 60, Essigester/Ethanol 9/1).

b) 3-Benzyloxy-2-[(4-benzyloxy-3-methoxy-phenyl)-hydroxy-methyl]-pyridin-4-ol. Aus 3-Benzyloxy-2-[(4-benzyloxy-3-methoxy-phenyl)-(tetrahydropyran-2-yloxy)-methyl]-pyridin-4-ol erhält man durch Behandlung analog Beispiel 1d: 3-Benzyloxy-2-[(4-benzyloxy-3-methoxyphenyl)-hydroxy-methyl]-pyridin-4-ol. Farblose Kristalle. Fp: 248-252°C.

Beispiel 23: 3-Hydroxy-1-methyl-2-(pyridin-3-carbonyl)-1*H*-pyndin-4-on

Durch Hydnerung von 1,35 g 3-Benzyloxy-1-methyl-2-(pyridin-3-carbonyl)-1*H*-pyridin-4-on analog Beispiel 1 erhält man nach Kristallisation aus Essigester: 3-Hydroxy-1-methyl-2-(pyridin-3-carbonyl)-1 *H*-pyridin-4-on. Gelbe Kristalle, Fp: 210-216°C.

Das Ausgangsmaterial kann zum Beispiel wie folgt hergestellt werden:

a) 3-Benzyloxy-1-methyl-2-(pyridin-3-carbonyl)-1*H*-pyridin4-on: Man löst 2,2 g 3-Benzyloxy-2-(hydroxy-pyridin-3-yl-methyl)-1-methyl-1 *H*-pyridin-4-on (Beispiel 7b) in 35 ml Dichlormethan und 7 ml Dimethylsulfoxid. Man gibt bei Raumtemperatur 4,9 ml Triethylamin zu und versetzt die Lösung abschliessend mit 4,33 g Pyridin-Schwefeltrioxid-Komplex [*CAS-Nr.: 26412-87-3*]. Man lässt die Lösung während 8 Tagen bei Raumtemperatur stehen, dann verdünnt man mit 50 ml Dichlormethan. Man schüttelt dreimal mit je 20 ml Wasser aus, trocknet die organische Phase über Magnesiumsulfat, filtriert und dampft am Rotationsverdampfer zur Trockene ein. Der Rückstand wird über Kieselgel chromatographiert. Als Eluent dient eine Mischung von Dichlormethan und Ethanol im Verhältnis von 6/1). Man erhält 3-Benzyloxy-1-methyl-2-(pyridin-3-carbonyl)-1 *H*-pyridin-4-on als gelbes Harz. $R_f$-Wert: 0,32 (Kieselgel 60, Dichlormethan/Ethanol 6/1).

Beispiel 24: 3-Hydroxy-2-[pyridin-3-yl-(acetoxy)-methyl]-1-methyl-1*H*-pyridin-4-on

2,7 g 3-Benzyloxy-1-methyl-2-[pyridin-3-yl-(acetoxy)-methyl-1*H*-pyridin-4-on werden in 50 ml Methanol gelöst und bei Raumtemperatur über 0,6 g Palladiumkohle (5%) bis zur Aufnahme von 1 mol $H_2$ pro mol Edukt hydriert. Man filtriert vom Katalysator ab und dampft das Filtrat am Rotationsverdampfer zur Trockene ein. Nach Umkristallisation aus Essigester erhält man 3-Hydroxy-2-[pyridin-3-yl-(acetoxy)-methyl]-1-methyl-1*H*-pyridin-4-on. Blass rosafarbene Kristalle, Fp:153-154°C.

Das Ausgangsmaterial kann zum Beispiel wie folgt hergestellt werden:

a) 3-Benzyloxy-1-methyl-2-[pyridin-3-yl-(acetoxy)-methyl-1*H*-pyridin-4-on: 3-Benzyloxy-2-(hydroxy-pyridin-3-yl-methyl)-1-methyl-1*H*-pyridin-4-on (Beispiel 7b) wird analog Beispiel 9a acetyliert. Man erhält 3-Benzyloxy-1-methyl-2-[pyridin-3-yl-(acetoxy)-methyl-1*H*-pyridin-4-on als gelbes, trübes Harz. $R_f$-Wert: 0,37 (Kieselgel 60, Dichlormethan/Ethanol 4/1).

Beispiel 25 3-Hydroxy-1-methyl-2-pyridin-3-ylmethyl-1*H*-pyridin-4-on

2,83 g 3-Benzyloxy-1-methyl-2-[pyridin-3-yl-(acetoxy)-methyl-1*H*-pyridin-4-on (Beispiel 24a) werden in 50 ml Methanol gelost und über 0,6 g Palladiumkohle (5%) zuerst bei Raumtemperatur bis zur Aufnahme von 1 mol $H_2$ pro mol Edukt hydriert Man erhöht die Temperatur auf 50 °C und hydriert weiter bis zur Aufnahme von einem weiteren mol $H_2$ pro mol Edukt. Man filtriert vom Katalysator ab und dampft das Filtrat am Rotationsverdampfer zur Trockene ein. Nach Umkristallisation aus Ethanol/Diethylether erhält man 3-Hydroxy-1-methyl-2-pyridin-3-ylmethyl-1 *H*-pyridin-4-on. Farblose Kristalle, Fp: 209-212°C, Kristallumwandlung ab 188°C.

Beispiel 26: 3-Hydroxy-1-(2-hydroxy-ethyl)-2-[hydroxy-(2-hydroxy-phenyl)-methyl]-1*H*-pyridin-4-on

Aus 1,84 g 3-Benzyloxy-2-[(2-benzyloxy-phenyl)-hydroxy-methyl]-1-(2-hydroxy-ethyl)-1*H*-pyridin-4-on erhält man analog Beispiel 11: 3-Hydroxy-1-(2-hydroxy-ethyl)-2-[hydroxy-(2-hydroxy-phenyl)-methyl]-1 *H*-pyridin-4-on. Farblose Kristalle, Fp: 196-197°C.

Das Ausgangsmaterial kann zum Beispiel wie folgt hergestellt werden:

a) 3-Hydroxy-2-[hydroxy-(2-hydroxy-phenyl)-methyl]-pyran-4-on: Aus 2,24 g Pyromeconsäure und 3,05 g Salicylaldehyd erhält man analog Beispiel 3a: 3-Hydroxy-2-[hydroxy-(2-hydroxy-phenyl)-methyl]-pyran-4-on. $R_f$-Wert: 0,45 (Kieselgel 60, Essigester/Ethanol 95/5).

b) 3-Benzyloxy-2-[(2-benzyloxy-phenyl)-hyrdroxy-methyl]-pyran-4-on: Aus 4,22 g 3-Hydroxy-2-[hydroxy-(2-hydroxy-phenyl)-methyl]-pyran-4-on erhält man analog Beispiel 1a: 3-Benzyloxy-2-[(2-benzyloxy-phenyl)-hydroxy-methyl]-pyran-4-on. Gelbe Kristalle, Fp: 144-145,6°C.

c) 3-Benzlrloxy-2-[(2-benzyloxy-phenyl)-(tetrahydropyran-2-yloxy)-methyl]-pyran-4-on: Aus 3-Benzyloxy-2-[(2-benzyloxy-phenyl)-hydroxy-methyl]-pyran-4-on erhält man analog Beispiel 1b: 3-Benzyloxy-2-[(2-benzyloxy-phenyl)-(tetrahydropyran-2-yloxy)-methyl]-pyran-4-on Gelbes, dickes Oel. Diastereoisomerengemisch. $R_f$-Werte: 0,48 und 0,56 (Kieselgel 60, Hexan/Dichlormethan/Essigester 1/1/1)

d) 3-Benzyl-oxy-2-[(2-benzyloxy-phenyl)-(tetrahydropyran-2-yloxy)-methyl]-1-(2-hydroxyethyl)-1*H*-pyridin-4-on: Aus 5,31 g 3-Benzyloxy-2-[(2-benzyloxy-phenyl)-(tetrahydropyran-2-yloxy)-methyl]-pyran-4-on erhalt man analog Beispiel 1c, nach Chromatographie 3-Benzyloxy-2-[(2-benzyloxy-phenyl)-(tetrahydropyran-2-yloxy)-methyl]-1-(2-hydroxy-ethyl)-1*H*-pyridin-4-on. Beiger Schaum. Diastereoisomerengemisch. $R_f$-Werte: 0,15 und 0,2 (Kieselgel 60, Essigester/Ethanol 9/1).

e) 3-Benzyloxy-2-[(2-benzyloxy-phenyl)-hydroxy-methyl]-1-(2-hydroxy-ethyl)-1*H*-pyridin-4-on: Aus 2,42 g 3-Benzyl-oxy-2-[(2-benzyloxy-phenyl)-(tetrahydropyran-2-yloxy)-methyl]-1-(2-hydroxy-ethyl)-1 *H*-pyridin-4-on erhält man analog Beispiel 6e, nach Chromatographie. 3-Benzyloxy-2-[(2-benzyloxy-phenyl)-hydroxy-methyl]-1-(2-hydroxy-ethyl)-1*H*-pyridin-4-on. Beiger Schaum. $R_f$-Wert: 0,17 (Kieselgel 60, Essigester/Ethanol 9/1).

Beispiel 27: 3-Hydroxy-2-(hydroxy-pyridin-2-yl-methyl)-1-methyl-1*H*-pyridin-4-on

Aus 1,88 g 3-Benzyloxy-2-(hydroxy-pyridin-2-yl-methyl)-1-methyl-1 *H*-pyridin-4-on erhält man analog Beispiel 7: 3-Hydroxy-2-(hydroxy-pyridin-2-yl-methyl)-1-methyl-1*H*-pyridin-4-on. Farblose Kristalle, Fp: 202-204°C.

Das Ausgangsmaterial kann zum Beispiel wie folgt hergestellt werden:

a) 2-(Hydroxy-pyridin-2-yl-methyl)-3-hydroxy-pyran-4-on: Aus 5,05 g Pyromeconsäure und 4,82 g Pyridin-2-carbaldehyd [*CAS-Nr.: 1121-60-4*] erhält man analog Beispiel 6a: 2-(Hydroxy-pyridin-2-yl-methyl)-3-hydroxy-pyran-4-on. Gelbe Kristalle, Fp: 161,2-164°C.

b) 3-Benzyloxy-2-(hydroxy-pyridin-2-yl-methyl)-pyran-4-on: Aus 2-(Hydroxy-pyridin-2-yl-methyl)-3-hydroxy-pyran-4-on erhält man analog Beispiel 6b: 3-Benzyloxy-2-(hydroxy-pyridin-2-yl-methyl)-pyran-4-on. Oranges Oel. $R_f$-Wert: 0,56 (Kieselgel 60, Essigester/Ethanol 9/1).

c) 3-Benzyloxy-2-[pyridin-2-yl-(tetrahyrdropyran-2-yloxy)-methyl]-pyran-4-on: Aus 12 g 3-Benzyloxy-2-(hydroxy-pyridin-2-yl-methyl)-pyran-4-on erhält man analog Beispiel 6c: 3-Benzyloxy-2-[pyridin-2-yl-(tetrahydropyran-2-yloxy)-methyl]-pyran-4-on als Diastereoisomerengemisch. Beige Kristalle, Fp: 107-121 °C.

d) 3-Benzyloxy-1-methyl-2-[pyridin-2-yl-(tetrahydropyran-2-yloxy)-methyl]-1*H*-pyridin-4-on: Aus 4,5 g 3-Benzyloxy-2-[pyridin-2-yl-(tetrahydropyran-2-yloxy)-methyl]-pyran-4-on erhalt man analog Beispiel 7a: 3-Benzyloxy-1-methyl-2-[pyridin-2-yl-(tetrahydropyran-2-yloxy)-methyl]-1*H*-pyridin-4-on. Rotes Harz. Diastereoisomerengemisch $R_f$-Wert 0,05 (Kieselgel 60, Essigester/Ethanol 9/1).

e) 3-Benzyloxy-2-(hydroxy-pyridin-2-yl-methyl)-1-methyl-1*H*-pyridin-4-on: Aus 2,5 g 3-Benzyloxy-1-methyl-2-[pyridin-2-yl-(tetrahydropyran-2-yloxy)-methyl]-1 *H*-pyridin-4-on erhält man analog Beispiel 7b: 3-Benzyloxy-2-(hydroxy-pyndin-2-yl-methyl)-1-methyl-1*H*-pyndin-4-on. Gelbes Harz. $R_f$-Wert: 0,52 (Kieselgel 60, Dichlormethan/Ethanol 4/1).

Beispiel 28: 3-Hydroxy-1-(2-hydroxy-ethyl)-2-(hydroxy-pyridin-2-yl-methyl)-1*H*-pyridin-4-on

Aus 0,877 g 3-Benzyloxy-1-(2-hydroxy-ethyl)-2-(hydroxy-pyridin-2-yl-methyl)-1*H*-pyridin-4-on erhält man analog Beispiel 6: 3-Hydroxy-1-(2-hydroxy-ethyl)-2-(hydroxy-pyridin-2-yl-methyl)-1*H*-pyridin-4-on. Farblose Kristalle, Fp: Zersetzung ab 185°C.

Das Ausgangsmaterial kann zum Beispiel wie folgt hergestellt werden:

a) 3-Benzyloxy-1-(2-hydroxy-ethyl)-2-[pyridin-2-yl-(tetrahydropyran-2-yloxy)-methyl]-1*H*-pyridin-4-on: Aus 4,4 g 3-Benzyloxy-2-[pyridin-2-yl-(tetrahydropyran-2-yloxy)-methyl]-pyran-4-on (Beispiel 27 c) erhält man analog Beispiel 6d: 3-Benzyloxy-1-(2-hydroxy-ethyl)-2-[pyridin-2-yl-(tetrahydropyran-2-yloxy)-methyl]-1 *H*-pyridin-4-on. Gelber Schaum. Diastereoisomerengemisch, $R_f$-Wert: 0,43 (Kieselgel 60, Dichlormethan/Ethanol 6/1).

b) 3-Benzyloxy-1-(2-hydroxy-ethyl)-2-(hydroxy-pyridin-2-yl-methyl)-1*H*-pyridin-4-on: Aus 1,4 g 3-Benzyloxy-1-(2-hydroxy-ethyl)-2-[pyridin-2-yl-(tetrahydropyran-2-yloxy)-methyl]-1*H*-pyridin-4-on erhält man analog Beispiel 6e: 3-Benzyloxy-1-(2-hydroxy-ethyl)-2-(hydroxy-pyridin-2-yl-methyl)-1 *H*-pyridin-4-on. Beige Kristalle. Fp: 173-176°C.

Beispiel 29: 2-(Benzo[1,3]dioxol-5-yl-hydroxy-methyl)-3-hydroxy-1-methyl-1*H*-pyridin-4-on

Durch Hydrierung von 1,19 g 2-(Benzo[1,3]dioxol-5-yl-hydroxy-methyl)-3-benzyloxy-1-methyl-1 *H*-pyridin-4-on analog Beispiel 1 erhält man 2-(Benzo[1,3]dioxol-5-yl-hydroxymethyl)-3-hydroxy-1-methyl-1*H*-pyridin-4-on. Hellbeige Kristalle Fp: 230-2312°C.

Das Ausgangsmaterial kann zum Beispiel wie folgt hergestellt werden.

a) 2-(Benzo[1,3]dioxol-5-yl-hydroxy-methyl)-3-hydroxy-pyran-4-on: Aus 5,04 g Pyromeconsäure und 7,11 g Piperonal [*CAS-Nr.: 120-57-0*] erhält man analog Beispiel 3a 2-(Benzo-[1,3]dioxol-5-yl-hydroxy-methyl)-3-hydroxy-pyran-4-on. Beige Kristalle. Fp: 158-160°C.

b) 2-(Benzo[1,3]dioxol-5-yl-hydroxy-methyl)-3-benzylon-pyran-4-on: Aus 6,74 g 2-(Benzo-[1,3]dioxol-5-yl-hydroxy-methyl)-3-hydroxy-pyran-4-on erhält man analog Beispiel 1a, nach Chromatographie: 2-(Benzo[1,3]dioxol-5-yl-hydroxy-methyl)-3-benzyloxy-pyran-4-on. Gelbes Harz. $R_f$-Wert: 0,19 (Kieselgel 60, Hexan/Essigester 1/1).

c) 2-(Benzo[1,3]dioxol-5-yl-(tetrahydropyran-2-yloxy)-methyl]-3-benzyloxy-pyran-4-on: Aus 7,1 g 2-(Benzo[1,3]dioxol-5-yl-hydroxy-methyl)-3-benzyloxy-pyran-4-on erhält man analog Beispiel 1b, nach Chromatographie:

2-[Benzo[1,3]dioxol-5-yl-(tetrahydropyran-2-yloxy)-methyl]-3-benzyloxy-pyran-4-on. Hellgelbes Oel. $R_f$-Wert: 0,47 (Kieselgel 60, Hexan/Essigester 1/1).

d) 2-[Benzo[1,3]dioxol-5-yl-(tetrahydropyran-2-yloxy)-methyl]-3-benzyloxy-1-methyl-1*H*-pyridin-4-on: Aus 2,4 g 2-[Benzo[1,3]dioxol-5-yl-(tetrahydropyran-2-yloxy)-methyl]-3-benzyloxypyran-4-on erhält man analog Beispiel 7a: 2-[Benzo[1,3]dioxol-5-yl-(tetrahydropyran-2-yl-oxy)-methyl]-3-benzyloxy-1-methyl-1 *H*-pyridin-4-on. Gelbe Kristalle. Diastereoisomerengemisch. Fp: 148,6-153°C.

e) 2-(Benzo[1,3]dioxol-5-yl-hydroxy-methyl)-3-benzyloxy-1-methyl-1*H*-pyridin-4-on: Aus 1,6 g 2-[Benzo[1,3]dioxol-5-yl-(tetrahydropyran-2-yloxy)-methyl]-3-benzyloxy-1-methyl-1*H*-pyridin-4-on erhält man analog Beispiel 7b: 2-(Benzo[1,3]dioxol-5-yl-hydroxy-methyl)-3-benzyloxy-1-methyl-1 *H*-pyridin-4-on. Hellbeige Kristalle, Fp: 207,5-209,3°C.

Beispiel 30: 2-(Benzo[1,3]dioxol-5-yl-hydroxy-methyl)-3-hydroxy-1-(2-hydroxy-ethyl)-1*H*-pyridin-4-on

Durch Hydrierung von 1,5 g 2-(Benzo[1,3]dioxol-5-yl-hydroxy-methyl)-3-benzyloxy-1-(2-hydroxy-ethyl)-1 *H*-pyridin-4-on analog Beispiel 1 erhält man 2-(Benzo[1,3]dioxol-5-yl-hydroxy-methyl)-3-hydroxy-1-(2-hydroxy-ethyl)-1 *H*-pyridin-4-on. Beige Kristalle, Fp: 195-196°C.

Das Ausgangsmaterial kann zum Beispiel wie folgt hergestellt werden.

a) 2-[Benzo[1,3]dioxol-5-yl-(tetrahydropyran-2-yloxy)-methyl]-3-benzyloxy-1-(2-hydroxyethyl)-1*H*-pyridin-4-on: Aus 2,4 g 2-[Benzo[1,3]dioxol-5-yl-(tetrahydropyran-2-yloxy)-methyl]-3-benzyloxy-pyran-4-on (Beispiel 29 c) erhält man analog Beispiel 1c: 2-[Benzo[1,3]dioxol-5-yl-(tetrahydropyran-2-yloxy)-methyl]-3-benzyloxy-1-(2-hydroxy-ethyl)-1*H*-pyndin-4-on als Diastereoisomerengemisch. Gelbes Harz. $R_f$-Wert: 0,17 (Kieselgel 60, Essigester/Ethanol 9/1).

b) 2-(Benzo-[1,3]dioxol-5-yl-hydroxy-methyl)-3-benzyloxy-1-(2-hydroxy-ethyl)-1*H*-pyridin-4-on: Aus 2,28 g 2-[Benzo[1,3]dioxol-5-yl-(tetrahydropyran-2-yloxy)-methyl]-3-benzyloxy-1-(2-hydroxy-ethyl)-1 *H*-pyridin-4-on erhält man analog Beispiel 1d: 2-(Benzo-[1,3]dioxol-5-yl-hydroxy-methyl)-3-benzyloxy-1-(2-hydroxy-ethyl)-1 *H*-pyridin-4-on. Gelbe Kristalle, Fp: 85-87 °C.

Beispiel 31: 3-Hydroxy-1-(2-hydroxy-ethyl)-2-[hydroxy-(4-nitro-phenyl)-methyl]-1*H*-pyridin-4-on-hydrochlorid

0,19 g 3-Benzyloxy-1-(2-hydroxy-ethyl)-2-[hydroxy-(4-nitro-phenyl)-methyl]-1*H*-pyridin-4-on werden mit 1,9 ml HCl conc. 37% übergossen. Man erhitzt die Mischung unter Rühren für 45 Minuten auf 100°C. Man setzt 20 ml Toluol zu und destilliert bei 50 °C Badtemperatur und 60 mbar Druck am Rotationsverdampfer ab. Den Zusatz von Toluol und das Abdestillieren wiederholt man viermal. Der kristalline Rückstand wird aus Ethanol umkristallisiert. Man erhält 3-Hydroxy-1-(2-hydroxy-ethyl)-2-[hydroxy-(4-nitro-phenyl)-methyl]-1*H*-pyridin-4-on-hydrochlorid. Braune Kristalle, Fp: ab 204°C unter Zersetzung.

Das Ausgangsmaterial kann zum Beispiel wie folgt hergestellt werden:

a) 3-Hydroxy-2-[hydroxy-(4-nitro-phenyl)-methyl]-pyran-4-on: Aus 16,8 g Pyromeconsäure und 22,7 g 4-Nitrobenzaldehyd erhält man analog Beispiel 3a: 3-Hydroxy-2-[hydroxy-(4-nitro-phenyl)-methyl]-pyran-4-on. Gelbe Kristalle, Fp: ab 176°C Zersetzung.

b) 3-Benzyloxy-2-[hydroxy-(4-nitro-phenyl)-methyl]-pyran-4-on: Aus 3-Hydroxy-2-[hydroxy-(4-nitro-phenyl)-methyl]pyran-4-on erhalt man analog Beispiel 1a, nach Umkristallisation aus Essigester: 3-Benzyloxy-2-[hydroxy-(4-nitro-phenyl)-methyl]-pyran-4-on. Gelbe Kristalle, Fp 167-168,5°C

c) 3-Benzyloxy-2-[(4-nitro-phenyl)-(tetrahydropyran-2-yloxy)-methyl]-pyran-4-on: Aus 3-Benzyloxy-2-[hydroxy-(4-nitro-phenyl)-methyl]pyran-4-on erhält man analog Beispiel 1b: 3-Benzyloxy-2-[(4-nitro-phenyl)-(tetrahydropyran-2-yloxy)-methyl]-pyran-4-on als Diastereoisomerengemisch. Gelbes Harz. $R_f$-Werte: 0,48 und 0,55 (Kieselgel 60, Hexan/Dichlormethan/Essigester 1/1/1).

d) 3-Benzyloxy-1-(2-hydroxy-ethyl)-2-[(4-nitro-phenyl)-(tetrahydropyran-2-yloxy)-methyl]-1*H*-pyridin-4-on: Aus 3-Benzyloxy-2-[(4-nitro-phenyl)-(tetrahydropyran-2-yloxy)-methyl]-pyran-4-on erhält man analog Beispiel 1 c 3-Benzyloxy-1-(2-hydroxy-ethyl)-2-[(4-nitro-phenyl)-(tetrahydropyran-2-yloxy)-methyl]-1 *H*-pyridin-4-on als Diastereoisomerengemisch. Gelbes Harz. $R_f$-Wert: 0,16 (Kieselgel 60, Essigester/Ethanol 9/1).

e) 3-Benzyloxy-1-(2-hydroxy-ethyl)-2-[hydroxy-(4-nitro-phenyl)-methyl]-1*H*-pyridin-4-on: Aus 0,51 g 3-Benzyloxy-1-(2-hydroxy-ethyl)-2-[(4-nitro-phenyl)-(tetrahydropyran-2-yloxy)-methyl]-1 *H*-pyridin-4-on erhält man analog Beispiel 1 d: 3-Benzyloxy-1-(2-hydroxy-ethyl)-2-[hydroxy-(4-nitro-phenyl)-methyl]-1 *H*-pyridin-4-on. Gelbes Harz. $R_f$-Wert: 0,2 (Kieselgel 60, Essigester/Ethanol 9/1).

Beispiel 32: 3-Hydroxy-1-(2-hydroxy-ethyl)-2-(hydroxy-*p*-tolyl-methyl)-1*H*-pyridin-4-on

1,9 g 3-Benzyloxy-1-(2-hydroxy-ethyl)-2-(hydroxy-*p*-tolyl-methyl)-1*H*-pyridin-4-on werden analog Beispiel 1 hydriert und man erhält 3-Hydroxy-1-(2-hydroxy-ethyl)-2-(hydroxy-*p*-tolylmethyl)-1 *H*-pyridin-4-on. Hellgelbe Kristalle, Fp: 205,5-207°C.

Das Ausgangsmaterial kann zum Beispiel wie folgt hergestellt werden:

a) 3-Hydroxy-2-(hydroxy-*p*-tolyl-methyl)-pyran-4-on: Aus 11,2 g Pyromeconsäure und 12,0 g *p*-Toluylaldehyd [*CAS-Nr.: 104-87-0*] erhält man analog Beispiel 3a: 3-Hydroxy-2-(hydroxy-*p*-tolyl-methyl)-pyran-4-on. Farblose Knstalle, Fp: 161,3-165,3°C.

b) 3-Benzyloxy-2-(hydroxy-*p*-tolyl-methyl)-pyran-4-on: Aus 16,1 g 3-Hydroxy-2-(hydroxy-*p*-tolyl-methyl)-pyran-4-on erhalt man analog Beispiel 1a, aus Essigester 3-Benzyloxy-2-(hydroxy-*p*-tolyl-methyl)-pyran-4-on Farblose Knstalle, Fp: 134,4-135,7°C

c) 3-Benzyloxy-2-[(tetrahydropyran-2-yloxy)-*p*-tolyl-methyl]-pyran-4-on: Aus 3-Benzyloxy-2-(hydroxy-*p*-tolyl-methyl)-pyran-4-on erhält man analog Beispiel 1b: 3-Benzyloxy-2-[(tetrahydropyran-2-yloxy)-*p*-tolyl-methyl]-pyran-4-on als Diastereoisomerengemisch. Gelbes Oel. $R_f$-Wert: 0,74 (Kieselgel 60, Dichlormethan/Essigester 1/1).

d) 3-Benzyloxy-1-(2-hydroxy-ethyl)-2-[(tetrahydropyran-2-yloxy)-*p*-tolyl methyl]-1*H*-pyridin-4-on: Aus 4,4 g 3-Benzyloxy-2-[(tetrahydropyran-2-yloxy)-*p*-tolyl-methyl]-pyran-4-on erhält man analog Beispiel 1c: 3-Benzyloxy-1-(2-hydroxy-ethyl)-2-[(tetrahydropyran-2-yloxy)-*p*-tolyl methyl]-1 *H*-pyridin-4-on als Diastereoisomerengemisch. Gelbes Oel. $R_f$-Wert: 0,22 (Kieselgel 60, Essigester/Ethanol 9/1).

e) 3-Benzyloxy-1-(2-hydroxy-ethyl)-2-(hydroxy-*p*-tolyl-methyl)-1*H*-pyridin-4-on: Aus 4,5 g 3-Benzyloxy-1-(2-hydroxy-ethyl)-2-[(tetrahydropyran-2-yloxy)-*p*-tolyl methyl]-1*H*-pyridin-4-on erhält man analog Beispiel 1d: 3-Benzyloxy-1-(2-hydroxy-ethyl)-2-(hydroxy-*p*-tolyl-methyl)-1 *H*-pyridin-4-on. Farblose Kristalle, Fp: 95-97°C.

Beispiel 33: 2-[(4-Brom-phenyl)-hydroxy-methyl]-3-hydroxy-1-(2-hydroxy-ethyl)-1*H*-pyridin-4-on

1,2 g 3-Benzyloxy-1-(2-hydroxy-ethyl)-2-[hydroxy-(4-brom-phenyl)-methyl]-1*H*-pyridin-4-on werden in 100 ml Methanol gelöst. Man setzt 0,2 ml 1,2-Dichlorbenzol zu und hydnert über 0,24 g Palladiumkohle bei Raumtemperatur und Normaldruck bis zur Aufnahme von 1 mol $H_2$ pro mol Edukt. Man filtriert vom Katalysator ab und dampft das Filtrat zur Trockene ein. Der Rückstand wird aus Ethanol umkristallisiert. Man erhält 2-[(4-Brom-phenyl)-hydroxymethyl]-3-hydroxy-1-(2-hydroxy-ethyl)-1*H*-pyridin-4-on. Farblose Kristalle, Fp. 189-193°C.

Das gleiche Produkt kann auch wie folgt hergestellt werden:

1,2 g 3-Benzyloxy-1-(2-hydroxy-ethyl)-2-[hydroxy-(4-brom-phenyl)-methyl]-1*H*-pyridin-4-on werden mit 12 ml HCI conc. 37% übergossen und unter Rühren während 10 Minuten auf 110°C erhitzt. Man kühlt ab und setzt 15 g Eis zu. Unter Rühren und Kühlen im Eisbad neutralisiert man mit Natronlauge bis pH 6,5 erreicht ist. Man setzt 15 ml Essigester zu und lasst bei Raumtemperatur während 18 Stunden ruhren. Man filtriert das ausgefallene Produkt ab und wäscht mit Wasser und Essigester. Nach Umknstallisation aus Ethanol erhält man 2-[(4-Brom-phenyl)-hydroxy-methyl]-3-hydroxy-1-(2-hydroxy-ethyl)-1*H*-pyridin-4-on.

Das Ausgangsmaterial kann zum Beispiel wie folgt hergestellt werden:

a) 3-Hydroxy-2-[hydroxy-(4-brom-phenyl)-methyl]-pyran-4-on: Aus 5,6 g Pyromeconsäure und 9,53 4-Brombenzaldehyd [*CAS-Nr.: 1122-91-4*] erhält man analog Beispiel 3a: 3-Hydroxy-2-[hydroxy-(4-brom-phenyl)-methyl]-pyran-4-on. Beige Kristalle, Fp: 143-145°C.

b) 3-Benzyloxy-2-[hydroxy-(4-brom-phenyl)-methyl]-pyran-4-on: Aus 14,0 g 3-Hydroxy-2-[hydroxy-(4-brom-phenyl)-methyl]pyran-4-on erhält man analog Beispiel 1a, nach Umkristallisation aus Essigester: 3-Benzyloxy-2-[hydroxy-(4-brom-phenyl)-methyl]-pyran-4-on. Farblose Kristalle, Fp: 148-149°C.

c) 3-Benzyloxy-2-[(4-brom-phenyl)-(tetrahydropyran-2-yloxy)-methy]-pyran-4-on: Aus 3-Benzyloxy-2-[hydroxy-(4-brom-phenyl)-methyl]pyran-4-on erhält man analog Beispiel 1b: 3-Benzyloxy-2-[(4-brom-phenyl)-(tetrahydropyran-2-yloxy)-methyl]-pyran-4-on als Diastereoisomerengemisch. Gelbes Harz. $R_f$-Wert: 0,47 (Kieselgel 60, Hexan/Dichlormethan/Essigester 1/1/1).

d) 3-Benzyloxy-1-(2-hydroxy-ethyl)-2-[(4-brom-phenyl)-(tetrahydropyran-2-yloxy)-methyl]-1*H*-pyridin-4-on: Aus 5,25 g 3-Benzyloxy-2-[(4-brom-phenyl)-(tetrahydropyran-2-yloxy)-methyl]-pyran-4-on erhält man analog Beispiel 1c: 3-Benzyloxy-1-(2-hydroxy-ethyl)-2-[(4-brom-phenyl)-(tetrahydropyran-2-yloxy)-methyl]-1 *H*-pyridin-4-on als Diastereoisomerengemisch. Gelber Schaum. $R_f$-Wert: 0,19 (Kieselgel 60, Essigester/Ethanol 9/1).

e) 3-Benzyloxy-1-(2-hydroxy-ethyl)-2-[hydroxy-(4-brom-phenyl)-methyl]-1*H*-pyridin-4-on: Aus 5,2 g 3-Benzyloxy-1-(2-hydroxy-ethyl)-2-[(4-brom-phenyl)-(tetrahydropyran-2-yloxy)-methyl]-1 *H*-pyridin-4-on erhält man analog Beispiel 1d: 3-Benzyloxy-1-(2-hydroxy-ethyl)-2-[hydroxy-(4-brom-phenyl)-methyl]-1 *H*-pyridin-4-on. Hellbeige Kristalle, Fp: 97-102°C

Beispiel 34: 2-[(4-Chlor-phenyl)-hydroxy-methyl]-3-hydroxy-1-(2-hydroxy-ethyl)-1*H*-pyridin-4-on

2,65 g 3-Benzyloxy-1-(2-hydroxy-ethyl)-2-[hydroxy-(4-chlor-phenyl)-methyl]-1*H*-pyridin-4-on werden mit 26 ml HCl conc 37% ubergossen und unter Rühren während 10 Minuten auf 110°C erhitzt. Man kühlt ab und setzt 100 g Eis zu. Unter Rühren und Kühlen im Eisbad neutralisiert man mit ca. 22 ml Natronlauge conc. bis pH 6,5 erreicht ist Man setzt 50 ml Essigester zu und lasst bei Raumtemperatur während 4 Stunden rühren. Man filtriert das ausgefallene Produkt ab und wäscht mit Wasser und Essigester. Man erhält 2-[(4-Chlorphenyl)-hydroxy-methyl]-3-hydroxy-1-(2-hydroxy-ethyl)-1*H*-pyridin-4-on. Fp: 184-186°C.

Das Ausgangsmaterial kann zum Beispiel wie folgt hergestellt werden:

a) 3-Hydroxy-2-[hydroxy-(4-chlor-phenyl)-methyl]-pyran-4-on: Aus 5,6 g Pyromeconsäure und 7,1 g 4-Chlorbenzaldehyd *[CAS-Nr.: 104-88-1]* erhält man analog Beispiel 3a: 3-Hydroxy-2-[hydroxy-(4-chlor-phenyl)-methyl]-pyran-4-on. Beige Kristalle, Fp: 144-146°C

b) 3-Benzyloxy-2-[hydroxy-(4-chlor-phenyl)-methyl]-pyran-4-on: Aus 11,4 g 3-Hydroxy-2-[hydroxy-(4-chlor-phenyl)-methyl]pyran-4-on erhält man analog Beispiel 1a, nach Umkristallisation aus Essigester: 3-Benzyloxy-2-[hydroxy-(4-chlor-phenyl)-methyl]-pyran-4-on. Farblose Kristalle, Fp: 142-143°C.

c) 3-Benzyloxy-2-[(4-chlor-phenyl)-(tetrahydropyran-2-yloxy)-methyl]-pyran-4-on: Aus 12,0g 3-Benzyloxy-2-[hydroxy-(4-chlor-phenyl)-methyl]pyran-4-on erhält man analog Beispiel 1b: 3-Benzyloxy-2-[(4-chlor-phenyl)-(tetrahydropyran-2-yloxy)-methyl]-pyran-4-on als Diastereoisomerengemisch. Gelbes Harz. $R_f$-Wert: 0,2 (Kieselgel 60, Hexan/Dichlormethan/Essigester 1/1/1).

d) 3-Benzyloxy-1-(2-hydroxy-ethyl)-2-[(4-chlor-phenyl)-(tetrahydropyran-2-yloxy)-methyl]-1*H*-pyridin-4-on: Aus 5,0 g 3-Benzyloxy-2-[(4-chlor-phenyl)-(tetrahydropyran-2-yloxy)-methyl]-pyran-4-on erhält man analog Beispiel 1c: 5,1 g 3-Benzyloxy-1-(2-hydroxy-ethyl)-2-[(4-chlorphenyl)-(tetrahydropyran-2-yloxy)-methyl]-1 *H*-pyridin-4-on als Diastereoisomerengemisch. Gelbe Kristalle, Fp: 157-167 °C. $R_f$-Wert: 0,25 (Kieselgel 60, Essigester/Ethanol 9/1)

e) 3-Benzyloxy-1-(2-hydroxy-ethyl)-2-[hydroxy-(4-chlor-phenyl)-methyl]-1*H*-pyridin-4-on Aus 5,1 g 3-Benzyloxy-1-(2-hydroxy-ethyl)-2-[(4-chlor-phenyl)-(tetrahydropyran-2-yloxy)-methyl]-1 *H*-pyridin-4-on erhalt man analog Beispiel 1 d: 3-Benzyloxy-1-(2-hydroxy-ethyl)-2-[hydroxy-(4-chlor-phenyl)-methyl]-1*H*-pyridin-4-on. Beige Kristalle. Fp: 87-92°C.

Beispiel 35: 1-(4-Chlor-phenyl)-9-hydroxy-3,4-dihydro-1*H*-pyrido[2,1-c][1,4]oxazin-8-on

Man versetzt 0,245 g 3-Benzyloxy-1-(2-hydroxy-ethyl)-2-[hydroxy-(4-chlor-phenyl)-methyl]-1 *H*-pyridin-4-on (Beispiel 34e) mit 2,5 ml Salzsäure conc. 37%. Man erhitzt die Lösung unter Rühren während 45 Minuten auf 110°C. Man kühlt ab und versetzt mit 5 g Eis. Man neutralisiert mit Natronlauge bis zum pH 6,5, dann setzt man 5 ml Essigester zu und lässt 4 Stunden im Eisbad rühren. Man filtriert und wäscht mit Wasser und Essigester. Nach dem Trocknen erhält man 1-(4-Chlor-phenyl)-9-hydroxy-3,4-dihydro-1*H*-pyrido[2,1-c][1,4]oxazin-8-on. Rosa Kristalle, Fp: 211-214°C.

Beispiel 36: 1-(4-Fluor-phenyl)-9-hydroxy-3,4-dihydro-1*H*-pyrido[2,1-c][1,4]oxazin-8-on

Aus 1,1 g 3-Benzyloxy-1-(2-hydroxy-ethyl)-2-[hydroxy-(4-fluor-phenyl)-methyl]-1*H*-pyridin-4-on (Beispiel 3e) erhält man analog Beispiel 35: 1-(4-Fluor-phenyl)-9-hydroxy-3,4-dihydro-1 *H*-pyrido[2,1-c][1,4]oxazin-8-on. Orange Kristalle, Fp: 221-223°C.

Beispiel 37: 1-(4-Brom-phenyl)-9-hydroxy-3,4-dihydro-1*H*-pyrido[2,1-c][1,4]oxazin-8-on

Aus 1,0 g 3-Benzyloxy-1-(2-hydroxy-ethyl)-2-[hydroxy-(4-brom-phenyl)-methyl]-1*H*-pyridin-4-on (Beispiel 33e) erhält man analog Beispiel 35: 1-(4-Brom-phenyl)-9-hydroxy-3,4-dihydro-1 *H*-pyrido[2,1-c][1,4]oxazin-8-on. Orange Kristalle, Fp: 200-212°C.

Beispiel 38: 6-[(4-Fluor-phenyl)-hydroxy-methyl]-7-hydroxy-3,4-dihydro-pyrido[2,1-c][1,4]oxazin-1,8-dion

0,266 g 5-Benzyloxy-6-[(4-fluoro-phenyl)-hydroxy-methyl]-1-(2-hydroxy-ethyl)-4-oxo-1,4-dihydro-pyridin-2-carbonsäure werden in 20 ml Methanol gelöst und bei Raumtemperatur über 0,05 g Palladiumkohle (5%) bis zur Aufnahme von 1 mol $H_2$ pro mol Edukt hydriert. Man filtriert vom Katalysator ab und dampft das Filtrat am Rotationsverdampfer zur Trockene ein. Nach Umkristallisation aus Methanol erhält man 6-[(4-Fluor-phenyl)-hydroxy-methyl]-7-hydroxy-3,4-dihydro-pyrido[2,1-c][1,4]oxazin-1,8-dion als farblose Kristalle. Fp:221-225°C.

Das Ausgangsmaterial kann zum Beispiel wie folgt hergestellt werden:

a) 6-[(4-Fluoro-phenyl)-hydroxy-methyl]-5-hydroxy-4-oxo-4*H*-pyran-2-carbonsaure Man lost 7,8 g 5-Hydroxy-4-oxo-4*H*-pyran-2-carbonsäure [*CAS-Nr.. 499-78-5*] in 15 ml Wasser und 47,5 ml 2N NaOH auf. Man gibt 6,2 g 4-Fluorbenzaldehyd zu und versetzt noch mit 20 ml Methanol. Man lässt 48 Stunden bei Raumtemperatur rühren. Man entfernt am Rotationsverdampfer das Methanol und neutralisiert die zurückbleibende wassenge Lösung mit 47,5 ml 2N HCl. Die entstehende Kristallsuspension wird in einem Eisbad eine Stunde gerührt, dann filtriert man das Produkt ab und wäscht mit Wasser. Nach Trocken erhält man: 10,9 g 6-[(4-Fluoro-phenyl)-hydroxy-methyl]-5-hydroxy-4-oxo-4*H*-pyran-2-carbonsäure. $R_f$-Wert: 0,27 (Kieselgel 60, Dichlormethan/Methanol/Wasser/Eisessig 75/27/5/0,5.

b) 5-Benzyloxy-6-[(4-fluoro-phenyl)-hydroxy-methyl]-4-oxo-4*H*-pyran-2-carbonsäurebenzylester: Aus 6-[(4-Fluoro-phenyl)-hydroxy-methyl]-5-hydroxy-4-oxo-4H-pyran-2-carbonsäure erhält man analog Beispiel 15b, nach Chromatographie: 5-Benzyloxy-6-[(4-fluoro-phenyl)-hydroxy-methyl]-4-oxo-4*H*-pyran-2-carbonsäurebenzylester. $R_f$-Wert: 0,2 (Kieselgel 60, Hexan/Essigester 2/1).

c) 5-Benzyloxy-6-[(4-fluoro-phenyl)-(tetrahydropyran-2-yloxy)-methyl]-4-oxo-4*H*-pyran-2-carbonsäurebenzylester: Aus 5-Benzyloxy-6-[(4-fluoro-phenyl)-hydroxy-methyl]-4-oxo-4*H*-pyran-2-carbonsäurebenzylester erhält man analog Beispiel 6c: 5-Benzyloxy-6-[(4-fluorophenyl)-(tetrahydropyran-2-yloxy)-methyl]-4-oxo-4*H*-pyran-2-carbonsäurebenzylester. Gelbes Oel. $R_f$-Wert: 0,33 (Kieselgel 60, Hexan/Essigester 4/1).

d) 5-Benzyloxy-6-[(4-fluoro-phenyl)-(tetrahydropyran-2-yloxy)-methyl]-1-(2-hydroxy-ethyl-4-oxo-1,4-dihydro-pyridin-2-carbonsäure-(2-hydroxy-ethyl)-amid: 7,2 g 5-Benzyloxy-6-[(4-fluoro-phenyl)-(tetrahydropyran-2-yloxy)-methyl]-4-oxo-4*H*-pyran-2-carbonsäurebenzylester werden in 70 ml Ethanol und 7 ml Ethanolamin während 48 Stunden unter Rückfluss gekocht. Man entfernt am Rotationsverdampfer das Lösungsmittel. Der Rückstand wird in 100 ml Essigester aufgenommen und dreimal mit je 30 ml Wasser gewaschen. Die organische Phase wird über Magnesiumsulfat getrocknet, filtriert und am Rotationsverdampfer zur Trockene eingedampft. Nach Chromatographie über Kieselgel erhält man 5-Benzyloxy-6-[(4-fluoro-phenyl)-(tetrahydropyran-2-yloxy)-methyl]-1-(2-hydroxy-ethyl-4-oxo-1,4-dihydropyridin-2-carbonsäure-(2-hydroxy-ethyl)-amid. $R_f$-Wert: 0,1 (Kieselgel 60, Dichlormethan/Ethanol 4/1)

e) 5-Benzyloxy-6-[(4-fluoro-phenyl)-hydroxy-methyl]-1-(2-hydroxy-ethyl)-4-oxo-1,4-dihydropyridin-2-carbonsäure: Man löst 1 g 5-Benzyloxy-6-[(4-fluoro-phenyl)-(tetrahydropyran-2-yloxy)-methyl]-1-(2-hydroxy-ethyl-4-oxo-1,4-dihydro-pyridin-2-carbonsäure-(2-hydroxy-ethyl)-amid in einer Mischung von 5 ml 2N HCl und 5 ml Ethanol und kocht wahrend 3 Stunden unter Rückfluss. Man kühlt auf Raumtemperatur ab und versetzt mit 7,5 ml 2N NaOH. Man entfernt am Rotationsverdampfer das Ethanol und stellt die zurückbleibende wasserige Lösung mit 2N HCl auf pH 4. Die ausfallenden Kristalle werden abfiltnert und getrocknet. Man erhält 5-Benzyloxy-6-[(4-fluoro-phenyl)-hydroxy-methyl]-1-(2-hydroxy-ethyl)-4-oxo-1,4-dihydro-pyridin-2-carbonsäure. Beige Kristalle. $R_f$-Wert: 0,26 (Kieselgel 60, Dichlormethan/Methanol/Wasser/Eisessig 75/27/5/0,5).

Beispiel 39: 9-Hydroxy-1-phenyl-3,4-dihydro-1*H*-pyrido[2,1-c][1,4]oxazin-8-on

Aus 1,49 g eines ca. äquimolaren Gemisches aus 3-Benzyloxy-1-(2-hydroxy-ethyl)-2-(hydroxy-phenyl-methyl)-1 *H*-pyridin-4-on (Beispiel 1 d) und 3-Hydroxy-1-(2-hydroxy-ethyl)-2-(hydroxy-phenyl-methyl)-1 *H*-pyridin-4-on (Beispiel 1) erhält man analog Beispiel 35: 9-Hydroxy-1-phenyl-3,4-dihydro-1*H*-pyrido[2,1-c][1,4]oxazin-8-on. Beige Kristalle, Fp: 229-234 °C.

Beispiel 40: 9-Hydroxy-1-p-tolyl-3,4-dihydro-1*H*-pyrido[2,1-c][1,4]oxazin-8-on

Aus 0,46 g 3-Benzyloxy-1-(2-hydroxy-ethyl)-2-(hydroxy-*p*-tolyl-methyl)-1*H*-pyridin-4-on (Beispiel 32e) erhält man analog Beispiel 35: 9-Hydroxy-1-p-tolyl-3,4-dihydro-1*H*-pyrido[2,1-c]-[1,4]oxazin-8-on. Rötliche Kristalle, Fp: 210-218°C unter Zersetzung. Kristallumwandlung von Stäbchen in Nädelchen ab 200°C.

Beispiel 41: 4-[2-(4-fluorbenzyl)-3-hydroxy-4-oxo-4H-pyridin-1-yl]-buttersäure-ethylester

2,1 g 4-{2-[Acetoxy-(4-fluor-phenyl)-methyl]-3-benzyloxy-4-oxo-4*H*-pyridin-1-yl}-buttersäureethylester werden in 50 ml Tetrahydrofuran über 0,5 g Palladiumkohle (5%) bei Normaldruck bei einer Temperatur von 50°C hydriert bis 2 mol $H_2$ pro mol Edukt aufgenommen worden sind. Man filtriert vom Katalysator ab und dampft das Filtrat am Rotationsverdampfer ein. Nach Umkristallisation aus Essigester erhält man 4-[2-(4-fluorbenzyl)-3-hydroxy-4-oxo-4H-pyndin-1-yl]-buttersaure-ethylester. Leicht rotliche Kristalle Fp· 137-141 °C.

Das Ausgangsmaterial kann zum Beispiel wie folgt hergestellt werden:

a) 4-{3-Benzyloxy-2-[(4-fluor-phenyl)-hydroxy-methyl]-4-oxo-4*H*-pyridin-1-yl}-buttersaureethylester: 2,15 g 4-{3-Benzyloxy-2-[(4-fluor-phenyl)-hydroxy-methyl]-4-oxo-4*H*-pyridin-1-yl}-buttersäure (Beispiel 17b) werden in 100 ml Ethanol absolut gelöst. Man setzt 2 ml Chlorwasserstoff in Ether (5N) zu und kocht die Lösung während 5 Stunden unter Rückfluss. Man dampft am Rotationsverdampfer ein und versetzt den Rückstand mit ca. 50 ml Toluol. Man dampft wieder zur Trockene ein und versetzt den Rückstand mit 20 ml Essigester. Die ausfallenden Kristalle werden abfiltriert, mit Essigester gewaschen und getrocknet. Man erhält 4-{3-Benzyloxy-2-[(4-fluor-phenyl)-hydroxy-methyl]-4-oxo-4*H*-pyridin-1-yl}-buttersäureethylester als Hydrochlorid. Farblose Kristalle, Fp: 136-138°C.

b) 4-{2-[Acetoxy-(4-fluor-phenyl)-methyl]-3-benzyloxy-4-oxo-4*H*-pyridin-1-yl}-buttersäureethylester: Aus 4-{3-Benzyloxy-2-[(4-fluor-phenyl)-hydroxy-methyl]-4-oxo-4*H*-pyridin-1-yl}-buttersäureethylester erhält man analog Beispiel 9a: 4-{2-[Acetoxy-(4-fluor-phenyl)-methyl]-3-benzyloxy-4-oxo-4*H*-pyridin-1-yl}-buttersäureethylester. Leicht trübes Harz. $R_f$-Wert: 0,12 (Kieselgel 60, Dichlormethan/Essigester 1/1)

Beispiel 42: 2-Benzyl-3-hydroxy-1-(2-acetoxy-ethyl)-1*H*-pyridin-4-on

2,2 g Essigsäure-[1-(2-acetoxy-ethyl)-3-benzyloxy-4-oxo-1,4-dihydropyndin-2-yl-methyl]-phenyl-methylester werden in 50 ml Tetrahydrofuran über 0,5 g Palladiumkohle (5%) bei Normaldruck bei einer Temperatur von 50°C hydriert bis 2 mol $H_2$ pro mol Edukt aufgenommen worden sind. Man filtriert vom Katalysator ab und dampft das Filtrat am Rotationsverdampfer ein. Nach Umkristallisation aus Tetrahydrofuran erhält man 2-Benzyl-3-hydroxy-1-(2-acetoxy-ethyl)-1 *H*-pyridin-4-on. Farblose Kristalle, Fp: 139,7-140,7°C.

Das Ausgangsmaterial kann zum Beispiel wie folgt hergestellt werden:

a) Essigsäure-[1-(2-acetoxy-ethyl)-3-benzyloxy-4-oxo-1,4-dihydropyridin-2-yl-methyl]-phenyl-methylester: 1,76 g 3-Benzyloxy-1-(2-hydroxy-ethyl)-2-(hydroxy-phenyl-methyl)-1*H*-pyridin-4-on (Beispiel 1d) werden in 20 ml Dichlormethan suspendiert und mit 1,5 ml Triethylamin versetzt. Man gibt 1,04 ml Acetanhydrid und anschliessend noch 20 mg 4-Dimethylaminopyndin zu Man lasst bei Raumtemperatur rühren Nach 5 Stunden verdünnt man die Reaktionslosung mit 100 ml Essigester Man wascht viermal mit je 20 ml Wasser und einmal mit 10 ml gesättigter Solelösung. Die organische Phase wird über Magnesiumsulfat getrocknet, filtriert und eingedampft. Man erhalt Essigsaure-[1-(2-acetoxy-ethyl)-3-benzyloxy-4-oxo-1,4-dihydropyridin-2-yl-methyl]-phenyl-methylester als Rohprodukt. Trübes Harz. $R_f$-Wert: 0,5 (Kieselgel 60, Essigester/Ethanol 9/1).

Beispiel 43: Essigsäure-[1-(2-acetoxy-ethyl)-3-hydroxy-4-oxo-1,4-dihydropyridin-2-yl-methyl]-phenyl-methylester

1,62 g Essigsaure-[1-(2-acetoxy-ethyl)-3-benzyloxy-4-oxo-1,4-dihydropyridin-2-yl-methyl]-phenyl-methylester werden in 100 ml Tetrahydrofuran über 200 mg Palladiumkohle (5%) bei Normaldruck bei Raumtemperatur hydriert bis 1 mol $H_2$ pro mol Edukt aufgenommen worden sind. Man filtriert vom Katalysator ab und dampft das Filtrat am

Rotationsverdampfer ein. Man erhält: Essigsäure-[1-(2-acetoxy-ethyl)-3-hydroxy-4-oxo-1,4-dihydropyndin-2-yl-methyl]-phenyl-methylester. Farblose Kristalle, Fp: 154-156 °C

Das Ausgangsmaterial kann zum Beispiel wie folgt hergestellt werden:

a) Essigsäure-[1-(2-acetoxy-ethyl)-3-benzyloxy-4-oxo-1,4-dihydropyridin-2-yl-methyl]-phenyl-methylester: 17.4 g 3-Benzyloxy-1-(2-hydroxy-ethyl)-2-(hydroxy-phenyl-methyl)-1 *H*-pyridin-4-on (Beispiel 1d) werden bei Raumtemperatur in 200 ml Dichlormethan suspendiert. Man gibt 33 ml Triethylamin und 18.7 ml Acetanhydrid zu. Nach Zugabe von 0.05 g 4-Dimethylaminopyridin [*CAS-Nr.: 1122-58-3*] lässt man das Reaktionsgemisch 23 Stunden bei Raumtemperatur rühren. Man setzt 5 ml Ethanol zu und lässt 30 Minuten bei Raumtemperatur rühren. Zum Aufarbeiten wäscht man dreimal mit je 50 ml Wasser. Die organische Phase wird über Magnesiumsulfat getrocknet und filtriert. Das Filtrat wird am Rotationsverdampfer eingedampft. Man erhält Essigsäure-[1-(2-acetoxy-ethyl)-3-benzyloxy-4-oxo-1,4-dihydropyridin-2-yl-methyl]-phenyl-methylester. Oranges Harz. $R_f$-Wert 0.125 (Kieselgel 60, Dichlormethan/Essigester 1/1).

In analoger Weise wie in den Beispielen 1 bis 43 kann man auch die nachstehend aufgeführten Verbindungen herstellen:

Beispiel 44: 4-{2-[(4-Fluor-phenyl)-hydroxy-methyl]-3-hydroxy-4-oxo-4*H*-pyridin-1-yl}-buttersäureethylester

Beispiel 45: 4-[3-Hydroxy-2-(hydroxy-phenyl-methyl)-4-oxo-4*H*-pyridin-1-yl]-buttersaureethylester

Beispiel 46: 4-(2-Benzyl-3-hydroxy-4-oxo-4*H*-pyridin-1-yl)-buttersaureethylester

Beispiel 47: 3,9-Dihydroxy-1-phenyl-3,4-dihydro-1*H*-pyrido[2,1-c][1,4]oxazin-8-on

Beispiel 48: 1-(4-Fluorophenyl)-3,9-dihydroxy-3,4-dihydro-1*H*-pyrido[2,1-c][1,4]oxazin-8-on

Beispiel 49: 2-[(2-Fluorphenyl)-hydroxy-methyl]-3-hydroxy-1-(2-hydroxy-ethyl)-1*H*-pyridin-4-on

1,5 g 2-[(2-Fluorphenyl)-hydroxy-methyl]-3-benzyloxy-1-(2-hydroxy-ethyl)-*1H*-pyridin-4-on werden analog Beispiel 1 hydriert und man erhält nach Umkristallisieren aus Ethanol 2-[(2-Fluorphenyl)-hydroxy-methyl]-3-hydroxy-1-(2-hydroxy-ethyl)-*1H*-pyndin-4-on als farblose Kristalle Fp: 177-178 °C.

Das Ausgangsmaterial kann zum Beispiel wie folgt hergestellt werden:

a) 2-[(2-Fluorphenyl)-hydroxy-methyl]-3-hydroxy-pyran-4-on: Aus 5,6 g Pyromeconsaure und 6,33 g 2-Fluorbenzaldehyd *[CAS-Nr.: 446-52-6*] erhält man analog Beispiel 3a 2-[(2-Fluorphenyl)-hydroxy-methyl]-3-hydroxy-pyran-4-on als farblose Kristalle Fp:122,5-124 °C.

b) 2-[(2-Fluorphenyl)-hydroxy-methyl]-3-benzyloxy-pyran-4-on: Aus 10,9 g 2-[(2-Fluorphenyl)-hydroxy-methyl]-3-hydroxy-pyran-4-on erhält man durch Umsetzung mit Benzylbromid in N,N-Dimethylformamid und Kaliumcarbonat analog Beispiel 1a 2-[(2-Fluorphenyl)-hydroxy-methyl]-3-benzyloxy-pyran-4-on. Farblose Kristalle, Fp: 116-116,5 °C

c) 3-Benzyloxy-2-[(2-fluorohenyl)-(tetrahydro-pyran-2-yloxy)-methyl]-pyran-4-on: Aus 13,2 g 2-[(2-Fluorphenyl)-hydroxy-methyl]-3-benzyloxy-pyran-4-on erhalt man durch Umsetzung mit 3,4-Dihydro-2*H*-pyran analog Beispiel 1b rohes 3-Benzyloxy-2-[(2-fluorphenyl)-(tetrahydro-pyran-2-yloxy)-methyl]-pyran-4-on als Diastereomerengemisch, $R_f$- Wert 0,55 (Kieselgel 60, Hexan/Dichlormethan/Essigester1/1/1).

d) 3-Benzyloxy-1-(2-hydroxy-ethyl)-2-[(2-fluorphenyl)-(tetrahydro-pyran-2-yloxy)-methyl]-*1H*-pyridin-4-on: 5,66 g 3-Benzyloxy-2-[(2-fluorphenyl)-(tetrahydro-pyran-2-yloxy)-methyl]-pyran-4-on werden in 50 ml Ethanol und 10 ml Ethanolamin während 24 Stunden am Ruckfluss gekocht. Nach Aufarbeitung analog Beispiel 1c erhält man 3-Benzyloxy-1-(2-hydroxy-ethyl)-2-[(2-fluorphenyl)-(tetrahydro-pyran-2-yloxy)-methyl]-*1H*-pyridin-4-on als als gelbe halbknstalline Masse. $R_f$-Wert: 0,2 (Kieselgel 60, Essigester/Ethanol 9/1)

e) 2-[(2-Fluorphenyl)-hydroxy-methyl]-3-benzyloxy-1-(2-hydroxy-ethyl)-*1H*-pyridin-4-on: 6,3 g 3-Benzyloxy-1-(2-hydroxy-ethyl)-2-[(2-fluorphenyl)-(tetrahydro-pyran-2-yloxy)-methyl]-*1H*-pyridin-4-on werden in 65 ml Ethanol und 13 ml Salzsäure 2N während 3 Stunden am Rückfluss gekocht. Zum Aufarbeiten befreit man am Rotationsverdampfer vom Ethanol. Der Rückstand wird mit 50 ml Wasser verdünnt und mit 50 ml Essigester überschichtet. Unter Rühren gibt man jetzt 50 ml gesättigte Natriumbicarbonatlösung zu. Das ausfallende Produkt wird abfiltriert und mit Wasser und Essigester gewaschen. Nach dem Trocknen erhält man 2-[(2-Fluorphenyl)-hydroxy-methyl]-3-benzyloxy-1-(2-hydroxy-ethyl)-*1H*-pyridin-4-on als farblose Kristalle. Fp: 164-166 °C.

Beispiel 50: 2-[(3-Fluorphenyl)-hydroxy-methyl]-3-hydroxy-1-(2-hydroxy-ethyl)-1*H*-pyridin-4-on

1,2 g 2-[(3-Fluorphenyl)-hydroxy-methyl]-3-benzyloxy-1-(2-hydroxy-ethyl)-*1H*-pyndin-4-on werden analog Beispiel 1 hydriert und man erhält 2-[(3-Fluorphenyl)-hydroxy-methyl]-3-hydroxy-1-(2-hydroxy-ethyl)-*1H*-pyndin-4-on als farblose Kristalle Fp: 194,2-196,2 °C.

Das Ausgangsmaterial kann zum Beispiel wie folgt hergestellt werden

a) 2-[(3-Fluorphenyl)-hydroxy-methyl]-3-hydroxy-pyran-4-on: Aus 5,6 g Pyromeconsäure und 6,33 g 3-Fluorbenzaldehyd [*CAS-Nr.: 456-48-4*] erhält man analog Beispiel 3a 2-[(3-Fluorphenyl)-hydroxy-methyl]-3-hydroxy-pyran-4-on als farblose Kristalle. Fp: 149-150,5 °C.

b) 2-[(3-Fluorphenyl)-hydroxy-methyl]-3-benzyloxy-pyran-4-on: Aus 11,2 g 2-[(3-Fluorphenyl)-hydroxy-methyl]-3-hydroxy-pyran-4-on erhält man durch Umsetzung mit Benzylbromid in N,N-Dimethylformamid und Kaliumcarbonat analog Beispiel 1a 2-[(3-Fluorphenyl)-hydroxy-methyl]-3-benzyloxy-pyran-4-on. Farblose Knstalle, Fp 95-96 °C.

c) 3-Benzyloxy-2-[(3-fluorphenyl)-(tetrahydro-pyran-2-yloxy)-methyl]-pyran-4-on Aus 11,3 g 2-[(3-Fluorphenyl)-hydroxy-methyl]-3-benzyloxy-pyran-4-on erhält man durch Umsetzung mit 3,4-Dihydro-*2H*-pyran analog Beispiel 1 b rohes 3-Benzyloxy-2-[(3-fluorphenyl)-(tetrahydro-pyran-2-yloxy)-methyl]-pyran-4-on als Diastereomerengemisch $R_f$- Wert 0,5 (Kieselgel 60, Hexan/Dichlormethan/Essigester1/1/1).

d) 3-Benzylogy-1-(2-hydroxy-ethyl)-2-[(3-fluorphenyl)-(tetrahydro-pyran-2-yloxy)-methyl]-*1H*-pyridin-4-on: 5,0 g 3-Benzyloxy-2-[(3-fluorphenyl)-(tetrahydro-pyran-2-yloxy)-methyl]-pyran-4-on werden in 50 ml Ethanol und 10 ml Ethanolamin während 24 Stunden am Rückfluss gekocht. Nach Aufarbeitung analog Beispiel 1c erhält man 3-Benzyloxy-1-(2-hydroxy-ethyl)-2-[(3-fluorphenyl)-(tetrahydro-pyran-2-yloxy)-methyl]-*1H*-pyridin-4-on als als gelbes Harz. $R_f$ -Wert: 0,22 (Kieselgel 60, Essigester/Ethanol 9/1).

e) 2-[(3-Fluorphenyl)-hydroxy-methyl]-3-benzyloxy-1-(2-hydroxy-ethyl)-*1H*-pyridin-4-on: Aus 5,3 g 3-Benzyloxy-1-(2-hydroxy-ethyl)-2-[(3-fluorphenyl)-(tetrahydro-pyran-2-yloxy)-methyl]-*1H*-pyridin-4-on erhält man analog Beispiel 49e 2-[(3-Fluorphenyl)-hydroxy-methyl]-3-benzyloxy-1-(2-hydroxy-ethyl)-*1H*-pyridin-4-on als farblose Kristalle. Fp: 179-182 °C. Kristallumwandlung ab 80 °C.

Beispiel 51: 5-Hydroxy1-(2-hydroxy-ethyl)-6-(hydroxy-phenyl-methyl)-4-oxo-1,4-dihydropyridin-2-carbonsäure

Beispiel 52: 5-Hydroxy1-(2-hydroxy-ethyl)-6-(hydroxy-phenyl-methyl)-4-oxo-1,4-dihydro-pyridin-2-carbonsäure-(2-hydroxyethyl)-amid

Beispiel 53: 2-[(4-Fluor-phenyl)-hydroxy-methyl]-3-hydroxy-1-(2-hydroxy-ethyl)-6-hydroxymethyl-1*H*-pyridin-4-on

Beispiel 54: Essigsäure 1-(2-acetoxy-ethyl)-6-[acetoxy-(4-fluor-phenyl)-methyl]-5-hydroxy-4-oxo-1,4-dihydro-pyridin-2-ylmethylester

Beispiel 55: 3-Hydroxy-1-(2-hydroxy-ethyl)-2-(hydroxy-naphth-2-yl-methyl)-1*H*-pyridin-4-on

1,5 g 2-(Hydroxy-naphth-2-yl-methyl)-3-benzyloxy-1-(2-hydroxy-ethyl)-*1H*-pyridin-4-on werden analog Beispiel 1 hydriert und man erhält: 3-Hydroxy-1-(2-hydroxy-ethyl)-2-(hydroxy-naphth-2-yl-methyl)-*1H*-pyridin-4-on als orange Kristalle Fp: ab 207 °C langsame Zersetzung.

Das Ausgangsmaterial kann zum Beispiel wie folgt hergestellt werden

a) 2-(Hydroxy-naphthalen-2-yl-methyl)-3-hydroxy-pyran-4-on: Aus 5,6 g Pyromeconsäure und 8,05 g 2-Naphthaldehyd [*CAS-Nr.:* 66-99-9] erhalt man analog Beispiel 3a 2-(Hydroxy-naphth-2-yl-methyl)-3-hydroxy-pyran-4-on als farblose Kristalle Fp:140-144 °C.

b) 2-(Hydroxy-naphth-2-yl-methyl)-3-benzyloxy-pyran-4-on: Aus 12,9 g 2-(Hydroxy-naphth-2-yl-methyl)-3-hydroxy-pyran-4-on erhält man durch Umsetzung mit Benzylbromid in N,N-Dimethylformamid und Kaliumcarbonat analog Beispiel 1 a 2-(Hydroxy-naphth-2-yl-methyl)-3-benzyloxy-pyran-4-on. beige Kristalle, Fp: 159-160 °C.

c) 3-Benzyloxy-2-[naphth-2-yl-(tetrahydro-pyran-2-yloxy)-methyl]-pyran-4-on: Aus 12,9 g 2-(Hydroxy-naphth-2-yl-methyl)-3-benzyloxy-pyran-4-on erhält man durch Umsetzung mit 3,4-Dihydro-*2H*-pyran analog Beispiel 1b rohes 3-Benzyloxy-2-[naphth-2-yl-(tetrahydro-pyran-2-yloxy)-methyl]-pyran-4-on als Diastereomerengemisch $R_f$- Wert: 0,52 (Kieselgel 60, Hexan/Dichlormethan/Essig-ester1/1 /1).

d) 3-Benzyloxy-1-(2-hydroxy-ethyl)-2-[naphth-2-yl-(tetrahydro-pyran-2-yloxy)-methyl]-1*H*-pyridin-4-on: 5,7 g 3-Benzyloxy-2-[naphth-2-yl-(tetrahydro-pyran-2-yloxy)-methyl]-pyran-4-on werden in 60 ml Ethanol und 6 ml Ethanolamin während 24 Stunden am Rückfluss gekocht. Nach Aufarbeitung analog Beispiel 1c erhält man rohes 3-Benzyloxy-1-(2-hydroxy-ethyl)-2-[naphth-2-yl-(tetrahydro-pyran-2-yloxy)-methyl]-*1H*-pyridin-4-on als als gelben, harten Schaum. $R_f$-Wert: 0,25 (Kieselgel 60, Essigester/Ethanol 9/1).

e) 2-[Naphth-2-yl-hydroxy-methyl]-3-benzyloxy-1-(2-hydroxy-ethyl)-*1H*-pyridin-4-on Aus 5,9 g 3-Benzyloxy-1-(2-hydroxy-ethyl)-2-[naphth-2-yl-(tetrahydro-pyran-2-yloxy)-methyl]-*1H*-pyridin-4-on werden in einer Mischung aus 60 ml Ethanol und 12 ml Salzsaure 2N wahrend 3 Stunden am Rückfluss gekocht Man entfernt das Ethanol am Rotationsverdampfer und neutralisiert die zuruckbleibende wassrige Losung mit überschüssiger wässeriger Natnumbicarbonatlosung Man extrahiert das ausfallende Produkt zwei Mal mit je 50 ml Essigester Die organischen Phasen werden zweimal mit je 20 ml Wasser und einmal mit 20 ml Sole gewaschen. Man vereinigt die organischen Phasen, trocknet über Magnesiumsulfat, filtriert und dampft das Filtrat am Rotationsverdampfer zur Trockene ein. Nach Umkristallisation aus Essigester erhalt man 2-[Naphth-2-yl-hydroxy-methyl]-3-benzyloxy-1-(2-hydroxy-ethyl)-*1H*-pyridin-4-on mit 1/3 mol/mol Essigester als Kristalllösungsmittel farblose Kristalle. Fp: 110,5-111,5 °C.

Beispiel 56: 4-[3-Hydroxy-2-(hydroxy-naphth-2-yl-methyl)--4-oxo-4*H*-pyridin-1-yl]-buttersäure

Beispiel 57: 4-[3-Hyrdroxy-2-(hydroxy-naphth-2-yl-methyl)--4-oxo-4*H*-pyridin-1-yl]-buttersäureethylester

Beispiel 58: 4-(3-Hydroxy-naphth-2-ylmethyl-4-oxo-4*H*-pyridin-1-yl)-buttersäureethylester

Beispiel 59: 4-{Hydroxy-[3-hydroxy-1-(2-hydroxy-ethyl)-4-oxo-1,4-dihydro-pyridin-2-yl]-methyl}-benzamid

0,7 g 4-{[3-Benzyloxy-1-(2-hydroxy-ethyl)-4-oxo-1,4-dihydro-pyridin-2-yl]-hydroxy-methyl}-benzamid werden in 20 ml Methanol über 0,07 g Palladiumkohle (5%) bei Normaldruck und Raumtemperatur hydriert. Nach Abfiltrieren des Katalysators wird das Produkt aus Methanol umkristallisiert. Man erhält 4-{Hydroxy-[3-hydroxy-1-(2-hydroxy-ethyl)-4-oxo-1,4-dihydropyridin-2-yl]-methyl}-benzamid als farblose Kristalle. Fp: 215,4-216,5 °C

Das Ausgangsmaterial kann zum Beispiel wie folgt hergestellt werden

a) 4-[Hydroxy-(3-hydroxy-4-oxo-4H-pyran-2-yl)-methyl]-benzonitril: Aus 11,2 g Pyromeconsäure und 13,1 g 4-Cyanbenzaldehyd [*CAS-Nr.: 105-07-7*] erhält man analog Beispiel 3a 4-[Hydroxy-(3-hydroxy-4-oxo-4H-pyran-2-yl)-methyl]-benzonitril als farblose Kristalle, Fp: 164-167 °C.

b) 4-[(3-Benzyloxy-4-oxo--4H-pyran-2-yl)-hydroxy-methyl]-benzonitril: Aus 23,3 g 4-[Hydroxy-(3-hydroxy-4-oxo-4H-pyran-2-yl)-methyl]-benzonitril erhält man durch Umsetzung mit Benzylbromid in N,N-Dimethylformamid und Kaliumcarbonat analog Beispiel 1a: 4-[(3-Benzyloxy-4-oxo--4H-pyran-2-yl)-hydroxy-methyl]-benzonitril. Hellgelbe Kristalle, Fp 134,9-137,8 °C.

c) 4-[(3-Benzyloxy-4-oxo--4H-pyran-2-yl)-(tetrahydro-pyran-2-yloxyl-methyl]-benzonitril: Aus 26,8 g 4-[(3-Benzyloxy-4-oxo--4H-pyran-2-yl)-hydroxy-methyl]-benzonitril erhält man durch Umsetzung mit 3,4-Dihydro-*2H*-pyran analog Beispiel 1b und nachfolgender Chromatographie 4-[(3-Benzyloxy-4-oxo-4H-pyran-2-yl)-(tetrahydro-pyran-2-yloxy)-methyl]-benzonitril als Diastereomerengemisch $R_f$- Wert 0,42 (Kieselgel 60, Hexan/Essigester1/1).

d) 4-[[3-Benzyloxy-1-(2-hydroxy-ethyl)-4-oxo-1,4-dihydro-pyridin-2-yl]-(tetrahydro-pyran-2-yloxy)-methyl]-benzamid: 4,17 g 4-[(3-Benzyloxy-4-oxo--4H-pyran-2-yl)-(tetrahydro-pyran-2-yloxy)-methyl]-benzonitril werden in 40 ml Ethanol und 4,2 ml Ethanolamin während 140 Stunden am Rückfluss gekocht. Nach Aufarbeitung analog Beispiel 1c und Chromatographie erhält man 4-[[3-Benzyloxy-1-(2-hydroxy-ethyl)-4-oxo-1,4-dihydro-pyridin-2-yl]-(tetrahydro-pyran-2-yloxy)-methyl]-benzamid als gelbes Harz. $R_f$-Wert: 0,4 (Kieselgel 60, Dichlormethan/Ethanol 4/1).

e) 4-{[3-Benzyloxy-1-(2-hydroxy-ethyl)-4-oxo-1,4-dihydro-pyridin-2-yl]-hydroxy-methyl}-benzamid: 1,7 g 4-[[3-Benzyloxy-1-(2-hydroxy-ethyl)-4-oxo-1,4-dihydro-pyridin-2-yl]-(tetrahydro-pyran-2-yloxy)-methyl]-benzamid werden in 5 ml Methanol und 3,7 ml Salzsäure 2N während 90 Minuten am Rückfluss gekocht. Zum Aufarbeiten befreit

man am Rotationsverdampfer vom Methanol. Der Rückstand wird mit 50 ml Wasser verdünnt, mit 20 ml Essigester überschichtet und mit 3,7 ml Natronlauge 2N neutralisiert. Das ausfallende Produkt wird abfiltriert und mit Wasser und Essigester gewaschen. Nach dem Trocknen erhält man 4-{[3-Benzyloxy-1-(2-hydroxy-ethyl)-4-oxo-1,4-dihydro-pyridin-2-yl]-hydroxy-methyl}-benzamid als farblose Kristalle. Fp· 132-137 °C

Beispiel 60: 4-{Hydroxy-[3-hydroxy-1-(2-hydroxy-ethyl)-4-oxo-1,4-dihydro-pyridin-2-yl]-methyl}-benzonitril

Beispiel 61: 3-Hydroxy-1-(2-hydroxy-ethyl)-2-(4-methyl-benzyl)-1*H*-pyridin-4-on

1,18 g Essigsäure 2-[3-hydroxy-2-(4-methyl-benzyl)-4-oxo-4H-pyndin-1-yl]-ethylester werden bei Raumtemperatur in 3 ml Methanol gelost und mit 3 ml Natronlauge 2N versetzt. Man rührt die Losung wahrend 18 Stunden bei Raumtemperatur, dann neutralisiert man mit 3 ml Salzsäure 2N. Man entfernt am Rotationsverdampfer das Methanol und filtnert das ausfallende Produkt ab. Nach dem Trocknen erhält man: 3-Hydroxy-1-(2-hydroxy-ethyl)-2-(4-methyl-benzyl)-*1H*-pyridin-4-on als farblose Kristalle, Fp: 217-219 °C.

Das Ausgangsmaterial kann zum Beispiel wie folgt hergestellt werden:

a) Essigsäure-[1-(2-acetoxy-ethyl)-3-benzyloxy-4-oxo-1,4-dihydropyridin-2-yl]-p-tolyl-methylester: 3,46 g 3-Benzyloxy-1-(2-hydroxy-ethyl)-2-(hydroxy-p-tolyl-methyl)-*1H*-pyridin-4-on (Beispiel 32e) werden bei Raumtemperatur in 35 ml Dichlormethan suspendiert. Man gibt 5,7 ml Triethylamin und 2,35 ml Acetanhydrid zu. Nach Zugabe von 0,05 g 4-Dimethyl-aminopyridin [*CAS-Nr.: 1122-58-3*] lässt man das Reaktionsgemisch 18 Stunden bei Raumtemperatur rühren. Man gibt 2 ml Ethanol zu und lasst 30 Minuten bei Raumtemperatur rühren. Zum Aufarbeiten wäscht man einmal mit 10 ml Salzsäure 2N und dreimal mit 10 ml Wasser. Die wässrigen Phasen werden einmal mit 20 ml Dichlormethan rückextrahiert. Die vereinigten organischen Phasen werden über Magnesiumsulfat getrocknet und filtriert. Das Filtrat wird am Rotationsverdampferor eingedampft. Man erhält rohen Essigsäure-[1-(2-acetoxy-ethyl)-3-benzyloxy-4-oxo-1,4-dihydropyridin-2-yl]-p-tolyl-methylester als beiges Harz. $R_f$-Wert 0,7 auf Kieselgel 60 im Laufmittel Dichlormethan / Ethanol 4/1).

b) Essigsäure-2-[3-hydroxy-2-(4-methyl-benzyl)-4-oxo-4H-pyridin-1-yl]-ethylester: 4,1 g Essigsäure[1-(2-acetoxy-ethyl)-3-benzyloxy-4-oxo-1,4-dihydropyridin-2-yl]-*p*-tolyl-methylester werden in 100 ml Tetrahydrofuran über 0,4 g Palladiumkohle (5%) bei 50°C unter Normaldruck hydriert bis 2 Mol $H_2$ pro Mol Edukt aufgenommen worden sind. Nach Abfiltrieren des Katalysators und einengen des Filtrats erhält man Essigsäure-2-[3-hydroxy-2-(4-methylbenzyl)-4-oxo-4H-pyridin-1-yl]-ethylester farblose Knstalle, Fp: 205-206,3 °C.

Beispiel 62: 4-[3-Hydroxy-2-(hydroxy-*p*-tolyl-methyl)-4-oxo-4*H*-pyridin-1-yl]-buttersäure

Beispiel 63. 4-[3-Hydroxy-2-(hydroxy-*p*-tolyl-methyl)-4-oxo-4*H*-pyridin-1-yl]-buttersäureethylester

Beispiel 64: 4-[3-Hydroxy-2-(4-methyl-benzyl)-4-oxo-4*H*-pyridin-1-yl]-buttersäureethylester

Beispiel 65: *(+)*-3-Hydroxy-1-(2-hydroxy-ethyl)-2-(hydroxy-phenyl-methyl)-*1H*-pyridin-4-on

0,278 g *(+)*-3-Benzyloxy-1-(2-hydroxy-ethyl)-2-(hydroxy-phenyl-methyl)-*1H*-pyridin-4-on werden analog Beispiel 1 hydnert und man erhält (+)-3-Hydroxy-1-(2-hydroxy-ethyl)-2-(hydroxy-phenyl-methyl)-*1H*-pyridin-4-on farblose Kristalle Fp: 169-170°C, $[\alpha]_D$ = +358° (c = 1% in Methanol).

Das Ausgangsmaterial kann zum Beispiel wie folgt hergestellt werden

a) *(+)*-Essigsäure [1-(2-acetoxy-ethyl)-3-benzyloxy-4-oxo-1,4-dihydropyridin-2-yl-methyl]-phenyl-methylester: Racemischer Essigsäure-[1-(2-acetoxy-ethyl)-3-benzyloxy-4-oxo-1,4-dihydropyridin-2-yl-methyl]-phenyl-methylester (Beispiel 43a) wird über eine Säule mit chiralem Trägermaterial wie zum Beispiel Chiralcel OD chromatographiert. Als Eluent wird Hexan/Ethanol im Verhältnis 8/2 verwendet. Als erste unpolare Fraktion eluiert man *(+)*-Essigsäure-[1-(2-acetoxy-ethyl)-3-benzyloxy-4-oxo-1,4-dihydropyridin-2-yl-methyl]-phenyl-methylester. Gelbes Harz, $[\alpha]_D$= +104° (c = 1 % in Methanol).

b) *(+)*-3-Benzyloxy-1-(2-hydroxy-ethyl)-2-(hydroxy-phenyl-methyl)-1*H*-pyridin-4-on 0,5 g *(+)*-Essigsäure-[1-(2-acetoxy-ethyl)-3-benzyloxy-4-oxo-1,4-dihydropyridin-2-yl-methyl]-phenyl-methylester werden mit 5 ml einer 6 M Lösung von Ammoniak in Methanol aufgelöst und 5 Stunden bei Raumtemperatur gerührt. Man dampft am Rotationsverdampfer zurTrokkene ein und verrührt den Rückstand mit Aether Nach Filtration und Trocknen erhält man (+)-3-Benzyloxy-1-(2-hydroxy-ethyl)-2-(hydroxy-phenyl-methyl)-*1H*-pyridin-4-on als farblose Kristalle Fp:

222-224,6 °C, $[\alpha]_D$= +277° (c = 1 % in Methanol).

Beispiel 66: *(-)*-3-Hydroxy-1-(2-hydroxy-ethyl)-2-(hydroxy-phenyl-methyl)-*1H*-pyridin-4-on

5,98 g *(-)*-3-Benzyloxy-1-(2-hydroxy-ethyl)-2-(hydroxy-phenyl-methyl) -*1H*-pyridin-4-on werden analog Beispiel 1 hydriert und man erhält *(-)*-3-Hydroxy-1-(2-hydroxy-ethyl)-2-(hydroxyphenyl-methyl)-*1H*-pyridin-4-on farblose Kristalle Fp: 168-170 °C, $[\alpha]_D$ = -352° (c = 1% in Methanol).

Das Ausgangsmaterial kann zum Beispiel wie folgt hergestellt werden:

a) *(-)*-Essigsäure-[1-(2-acetoxy-ethyl)-3-benzyloxy-4-oxo-1,4-dihydropyridin-2-yl-methyl]-phenyl-methylester: Aus racemischem Essigsäure-[1-(2-acetoxy-ethyl)-3-benzyloxy-4-oxo-1,4-dihydropyridin-2-yl-methyl]-phenyl-methylester wie in Beispiel 65a. Die polarere Fraktion ergibt *(-)*-Essigsäure [1-(2-acetoxy-ethyl)-3-benzyloxy-4-oxo-1,4-dihydropyridin-2-yl-methyl]-phenyl-methylester. Gelbes Harz, $[\alpha]_D$ = -100,5° (c = 1 % in Methanol).

b) *(-)*-3-Benzyloxy-1-(2-hydroxy-ethyl)-2-(hydroxy-phenyl-methyl)-*1H*-pyridin-4-on: Aus 1,6 g *(-)*-Essigsäure [1-(2-acetoxy-ethyl)-3-benzyloxy-4-oxo-1,4-dihydropyridin-2-yl-methyl]-phenyl-methylester erhält man analog Beispiel 65b *(-)*-3-Benzyloxy-1-(2-hydroxy-ethyl)-2-(hydroxy-phenyl-methyl)-*1H*-pyridin-4-on als farblose Kristalle Fp: 223-225,5 °C, $[\alpha]_D$ = -272° (c = 1% in Methanol).

Beispiel 67: Essigsäure-2-[2-(4--fluor-benzoyl)-3-hydroxy-4-oxo-4H-pyridin-1-yl]-ethylester

Beispiel 68: 2-[(4-Fluorphenyl)-hydroxy-methyl]-3-hydroxy-1-methyl-*1H*-pyridin-4-on

Beispiel 69: 2-(4-Fluorbenzyl)-3-hydroxy-1-methyl-*1H*-pyridin-4-on

Beispiel 70: 2-(4Fluor-benzoyl)-3-hydroxy-1-(2-hydroxy-ethyl)-*1H*-pyridin-4-on

Aus 3-Benzyloxy-2-(4-fluor-benzoyl)-1-(2-hydroxy-ethyl)-*1H*-pyridin-4-on erhält man bei Behandlung mit Salzsäure conc. analog Beispiel 34 2-(4Fluor-benzoyl)-3-hydroxy-1-(2-hydroxy-ethyl)-*1H*-pyridin-4-on. Fp: 209-216 °C

Das Ausgangsmaterial kann zum Beispiel wie folgt hergestellt werden:

a) 3-Benzyloxy-2-(4-fluor-benzoyl)-1-(2-hydroxy-ethyl)-*1H*-pyridin-4-on: 3,69 g 2-[(4-Fluorphenyl)-hydroxy-methyl]-3-benzyloxy-1-(2-hydroxy-ethyl)-*1H*-pyridin-4-on werden in 95 ml Acetonitril gelöst und mit 7,85 g Natriumpercarbonat [*CAS-Nr: 15630-89-4],* 0,37 g Pyridiniumdichromat [*CAS-Nr.: 20039-37-6*] und 0,4 g Aliquat 336 [*CAS-Nr.: 5137-55-3*] versetzt. Man erhitzt während 6 Stunden unter kräftigem Rühren zum Rückfluss, dann dampft man die Suspension zur Trockene ein. Man versetzt den Rückstand mit Wasser und extrahiert das Produkt mit Essigester. Nach Umkristallisation aus Essigester erhalt man 3-Benzyloxy-2-(4-fluor-benzoyl)-1-(2-hydroxy-ethyl)-*1H*-pyridin-4-on Fp: 182-182,6° C

Beispiel 71: Essigsäure-2-[3-hydroxy-2-(4-fluorbenzyl)-4-oxo-4H-pyridin-1-yl]-ethylester

6,2 g Essigsäure-[1-(2-acetoxy-ethyl)-3-benzyloxy-4-oxo-1,4-dihydropyridin-2-yl]-(4-fluorphenyl)-methylester werden analog Beispiel 61 b hydriert und man erhält Essigsäure-2-[3-hydroxy-2-(4-fluorbenzyl)-4-oxo-4H-pyridin-1-yl]-ethylester als farblose Kristalle, Fp 118-119 °C.

Das Ausgangsmaterial kann zum Beispiel wie folgt hergestellt werden

a) Essigsäure-[1-(2-acetoxy-ethyl)-3-benzyloxy-4-oxo-1,4-dihydropyridin-2-yl]-(4-fluorphenyl)-methylester: Aus 4,7 g 2-[(4-Fluorphenyl)-hydroxy-methyl]-3-benzyloxy-1-(2-hydroxy-ethyl)-*1H*-pyridin-4-on (Beispiel 3e) erhält man analog Beispiel 61a Essigsäure-[1-(2-acetoxy-ethyl)-3-benzyloxy-4-oxo-1,4-dihydropyridin-2-yl]-(4-fluorphenyl)-methylester als farbloses Harz. $R_f$ -Wert: 0,48 (Kieselgel 60, Essigester/Ethanol 9/1).

Beispiel 72: 3-Hydroxy-1-(2-hydroxy-ethyl)-2-(4-fluorbenzyl)-*1H*-pyridin-4-on

2,0 g Essigsäure-2-[3-hydroxy-2-(4-fluorbenzyl)-4-oxo-4H-pyridin-1-yl]-ethylester werden in 20 ml Methanol gelöst und mit 5 ml einer 6 M Lösung von Ammoniak in Methanol versetzt. Man rührt 18 Stunden bei Raumtemperatur, dabei fällt das Produkt als dicker Kristallbrei aus. Man filtriert und wäscht mit Methanol. Nach dem Trocknen erhält man 3-Hydroxy-1-(2-hydroxy-ethyl)-2-(4-fluorbenzyl)-*1H*-pyridin-4-on als farblose Kristalle Fp: 225-231 °C, zers. ab 190 °C.

Beispiel 73: 2-(4-Fluor-benzoyl)-pyridin-3,4-diol

0,88 g (3-Benzyloxy-4-hydroxy-pyridin-2-yl-)-(4-fluorphenyl)-methanon werden analog Beispiel 1 hydriert und man erhält nach Umkristallisation 2-(4-Fluor-benzoyl)-pyridin-3,4-diol als rötliche Kristalle. Fp: 249-251 °C unter Zersetzung.

Das Ausgangsmatenal kann zum Beispiel wie folgt hergestellt werden

a) 3-Benzyloxy-2-[(4-fluorphenyl)-(tetrahydro-pyran-2-yloxy)-methyl]-pyridin-4-ol: Aus 3-Benzyloxy-2-[(4-fluorphenyl)-(tetrahydro-pyran-2-yloxy)-methyl]-pyran-4-on (Beispiel 3c) erhalt man durch Umsetzung mit Ammoniak analog Beispiel 21 a 3-Benzyloxy-2-[(4-fluorphenyl)-(tetrahydro-pyran-2-yloxy)-methyl]-pyridin-4-ol. Harz $R_f$ -Wert. 0,62 (Kieselgel 60, Dichlormethan/Ethanol 4/1).

b) 3-Benzyloxy-2-[hydroxy-(4-fluorphenyl)-methyl]-pyridin-4-ol Aus 3-Benzyloxy-2-[(4-fluorphenyl)l-(tetrahydro-pyran-2-yloxy)-methyl]-pyridin-4-ol erhält man bei Behandlung analog Beispiel 1d 3-Benzyloxy-2-[hydroxy-(4-fluorphenyl)-methyl]-pyridin-4-ol. Farblose Kristalle, Fp: 208-213 °C unter Zersetzung.

c) (3-Benzyloxy-4-hydroxy-pyridin-2-yl-)-(4-fluorphenyl)-methanon: Aus 1,62 g 3-Benzyloxy-2-[hydroxy-(4-fluorphenyl)-methyl]-pyridin-4-ol erhalt man analog Beispiel 70a, nach Chromatographie (3-Benzyloxy-4-hydroxy-pyridin-2-yl-)-(4-fluorphenyl)-methanon als grünliches Harz, $R_f$ -Wert: 0,53 (Kieselgel 60, Essigester/Ethanol 6/1).

Beispiel 74: [2-(4-Fluorbenzyl)-3-hydroxy-4-oxo-4H-pyridin-1-yl]-essigsäure

Rötliche Kristalle, Fp:233-235 °C zers. ab 215 °C

Beispiel 75: [2-(4-Fluorbenzyl)-3-hydroxy-4-oxo--4H-oyridin-1-yl-]-essigsäure-ethylester

Beispiel 76: 2-(4-Fluorbenzyl)-pyridin-3,4-diol:

Gelbliche Kristalle, Fp: 236-240 °C Kristallumwandlung ab 180 °C, langsame Zersetzung ab 215 °C.

Beispiel 77: 2-(4-Fluorbenzyl)--3-hydroxy-1-(2-morpholin-4-yl-ethyl)-*1H*-pyridin-4-on

Farblose Kristalle, Fp: 160,3-161,2 °C.

Beispiel 78: 4-[2-(4-Fluorbenzyl)--3-hydroxy-4-oxo-4H-pyridin-1-ylmethyl]-benzoesaure

Beispiel 79: Essigsäure-2-[3-hydroxy-2-(4-methyl-benzyl)-4-oxo-4H-pyridin-1-yl]-ethylester

Farblose Kristalle, Fp: 205-206,3 °C. Entspricht Beispiel 61 b

Beispiel 80: 3-Hydroxy-1-(2-hydroxy-ethyl)-2-(4-chlorbenzyl)-*1H*-pyridin-4-on:

Beige Kristalle, Fp:222-224°C, unter Zersetzung.

Beispiel 81: Essigsäure 2-[3-hydroxy-2-(4-chlorbenzyl)-4-oxo-4H-pyridin-1-yl]-ethylester

Beispiel 82. 3-Hydroxy-1-(2-hydroxy-ethyl)-2-(4-brombenzyl)-*1H*-pyridin-4-on

Beispiel 83: Essigsäure-[1-(2-acetoxy-ethyl)-3-hydroxy-4-oxo-1,4-dihydropyridin-2-yl-]-(2-fluorphenyl)-methylester

Aus 2,15 g Essigsäure-[1-(2-acetoxy-ethyl)-3-benzyloxy-4-oxo-1,4-dihydropyridin-2-yl-]-(2-fluorphenyl)-methylester erhält man durch Hydrierung analog Beispiel 1 Essigsäure-[1-(2-acetoxy-ethyl)-3-hydroxy-4-oxo-1,4-dihydropyridin-2-yl-]-(2-fluorphenyl)-methylester. Farblose Kristalle, Fp: 173-174,5 °C.

Das Ausgangsmaterial kann zum Beispiel wie folgt hergestellt werden:

a) Essigsäure-[1-(2-acetoxy-ethyl)-3-benzyloxy-4-oxo-1,4-dihydropyridin-2-yl-]-(2-fluorphenyl)-methylester: Aus 1,71 g 2-[(2-Fluorphenyl)-hydroxy-methyl]-3-benzyloxy-1-(2-hydroxy-ethyl)-*1H*-pyridin-4-on (Beispiel 49e) erhält man durch Acetylierung analog Beispiel 61 a rohen Essigsäure-[1-(2-acetoxy-ethyl)-3-benzyloxy-4-oxo-1,4-dihydropyridin-2-yl-]-(2-fluorphenyl)-methylester als beiges Harz. $R_f$ -Wert: 0,27 (Kieselgel 60, Essigester/Ethanol 9/1)

Beispiel 84: Essigsäure-2-[3-hydroxy-2-(3-fluorbenzyl)-4-oxo-4H-pyridin-1-yl]-ethylester

1,95 g Essigsäure-[1-(2-acetoxy-ethyl)-3-benzyloxy-4-oxo-1,4-dihydropyridin-2-yl]-(3-fluorphenyl)-methylester werden analog Beispiel 61 b hydriert und man erhält Essigsäure-2-[3-hydroxy-2-(3-fluorbenzyl)-4-oxo-4H-pyridin-1-yl]-ethylester als farblose Kristalle, Fp: 147,9-149,3 °C.

Das Ausgangsmaterial kann zum Beispiel wie folgt hergestellt werden:

a) Essigsäure-[1-(2-acetoxy-ethyl)-3-benzyloxy-4-oxo-1,4-dihydropyridin-2-yl]-(3-fluorphenyl)-methylester: Aus 1,67 g 2-[(3-Fluorphenyl)-hydroxy-methyl]-3-benzyloxy-1-(2-hydroxy-ethyl)-*1H*-pyridin-4-on (Beispiel 49e) erhält man durch Acetylierung analog Beispiel 61 a rohen Essigsäure-[1-(2-acetoxy-ethyl)-3-benzyloxy-4-oxo-1,4-dihydropyridin-2-yl-]-(3-fluorphenyl)-methylester $R_f$ -Wert: 0,37 (Kieselgel 60, Essigester/Ethanol 9/1).

Beispiel 85: 3-Hydroxy-1-(2-hydroxy-ethyl)-2-(3-fluorbenzyl)-*1H*-pyridin-4-on

Aus 0,702 g Essigsaure 2-[3-hydroxy-2-(3-fluorbenzyl)-4-oxo-4H-pyridin-1-yl]-ethylester (Beispiel 84) erhält man analog Beispiel 72 3-Hydroxy-1-(2-hydroxy-ethyl)-2-(3-fluorbenzyl)-*1H*-pyridin-4-on als farblose Kristalle, Fp: 235-242 °C, langsame Zersetzung ab 204 °C.

Beispiel 86: 3-Hydroxy-1-(2-hydroxy-ethyl)-2-(hydroxy-thiophen-2-yl-methyl)-*1H*-pyridin-4-on

Aus 0,952 g 3-Benzyloxy-1-(2-hydroxy-ethyl)-2-(hydroxy-thiophen-2-yl-methyl)-*1H*-pyridin-4-on erhält man durch Hydrierung analog Beispiel 1, nach Chromatographie: 3-Hydroxy-1-(2-hydroxy-ethyl)-2-(hydroxy-thiophen-2-yl-methyl)-*1H*-pyridin-4-on. Fp: 186,9-187,3 °C.

Das Ausgangsmaterial kann zum Beispiel wie folgt hergestellt werden.

a) 3-Hydroxy-2-(hydroxy-thiophen-2-yl-methyl)-pyran-4-on: Aus 5,6 g Pyromeconsaure und 5,6 g Thiophen-2-carbaldehyd [*CAS-Nr.: 98-03-3*] erhält man analog Beispiel 3a 3-Hydroxy-2-(hydroxy-thiophen-2-yl-methyl)-pyran-4-on als gelbliche Kristalle, Fp: ab 144 °C unter Zersetzung.

b) 3-Benzyloxy-2-(hydroxy-thiophen-2-yl-methyl)-pyran-4-on: Aus 4,39 g 3-Hydroxy-2-(hydroxy-thiophen-2-yl-methyl-pyran-4-on erhält man durch Umsetzung mit Benzylbromid in N,N-Dimethylformamid und Kaliumcarbonat analog Beispiel 1 a 3-Benzyloxy-2-(hydroxy-thiophen-2-yl-methyl)-pyran-4-on. Gelbliche Kristalle, Fp: 114,8-117 °C.

c) 3-Benzyloxy-2-[(tetrahydro-pyran-2-yloxy)-thiophen-2-yl-methyl]-pyran-4-on: Aus 4,3 g 3-Benzyloxy-2-(hydroxy-thiophen-2-yl-methyl)-pyran-4-on erhält man durch Umsetzung mit 3,4-Dihydro-*2H*-pyran analog Beispiel 1 b, nach Chromatographie 3-Benzyloxy-2-[(tetrahydro-pyran-2-yloxy)-thiophen-2-yl-methyl]-pyran-4-on als Diastereomerengemisch $R_f$- Wert 0,28 (Kieselgel 60, Hexan/Essigester 2/1).

d) 3-Benzyloxy-1-(2-hydroxy-ethyl)-2-[(tetrahydro-pyran-2-yloxy)-thiophen-2-yl-methyl]-*1H*-pyridin-4-on: Aus 4,0 g 3-Benzyloxy-2-[(tetrahydro-pyran-2-yloxy)-thiophen-2-yl-methyl]-pyran-4-on erhält man durch Umsetzung mit Ethanolamin analog Beispiel 1c, nach Chromatographie 3-Benzyloxy-1-(2-hydroxy-ethyl)-2-[(tetrahydro-pyran-2-yloxy)-thiophen-2-yl-methyl]-*1H*-pyridin-4-on als als braunes Harz. $R_f$ -Wert: 0,24 (Kieselgel 60, Essigester/ Ethanol 9/1).

e) 3-Benzyloxy-1-(2-hydroxy-ethyl)-2-(hydroxy-thiophen-2-yl-methyl)-*1H*-pyridin-4-on 1,88 g 3-Benzyloxy-1-(2-hydroxy-ethyl)-2-[(tetrahydro-pyran-2-yloxy)-thiophen-2-yl-methyl]-*1H*-pyridin-4-on werden in 7 ml Methanol und 4,25 ml Salzsäure 2N wahrend 90 Minuten am Rückfluss gekocht. Zum Aufarbeiten befreit man am Rotationsverdampfer vom Methanol. Der Rückstand wird mit 20 ml Wasser verdünnt und mit 20 ml Essigester überschichtet. Unter Rühren gibt man jetzt 10 ml gesättigte Natriumbicarbonatlösung zu. Das ausfallende Produkt wird abfiltriert und mit Wasser und Essigester gewaschen. Nach dem Trocknen erhält man 3-Benzyloxy-1-(2-hydroxy-ethyl)-2-(hydroxy-thiophen-2-yl-methyl)-*1H*-pyndin-4-on als farblose Kristalle. Fp: 193,8-196 °C.

Beispiel 87: 9-Hydroxy-1-(4-methylthio-phenyl)-3,4-dihydro-*1H*-pyrido[2,1-c][1,4]oxazin-8-on-hydrochlorid

5,99 g 3-Benzyloxy-1-(2-hydroxy-ethyl)-2-[hydroxy-(4-methylthio-phenyl)-methyl]-*1H*-pyridin-4-on werden in 60 ml Salzsäure 6N während 45 Minuten am Rückfluss gekocht. Man kühlt auf Raumtemperatur ab und überschichtet

mit 10 ml Essigester. Unter kräftigem Rühren neutralisiert man mit verdünnter Natronlauge bis das Produkt kristallin ausfällt. Man lässt über Nacht im Kühlschrank stehen und filtriert dann ab. Die Kristalle werden mit wenig kaltem Alkohol gewaschen. Nach dem Trocknen erhält man 9-Hydroxy-1-(4-methylthiophenyl)-3,4-dihydro-*1H*-pyrido[2,1-c][1,4] oxazin-8-on-hydrochlorid. Fp: 235-238 °C

Das Ausgangsmaterial kann zum Beispiel wie folgt hergestellt werden:

a) 3-Hydroxy-2-[hydroxy-(4-methylthio-phenyl)-methyl]-pyran-4-on: Aus 9,3 g Pyromeconsäure und 14 g 4-(Methylthio)-benzaldehyd [*CAS-Nr.: 3446-89-7*] erhalt man analog Beispiel 3a 3-Hydroxy-2-[hydroxy-(4-methylsulfanyl-phenyl)-methyl]-pyran-4-on als beige Kristalle.

b) 3-Benzyloxy-2-[hydroxy-(4-methylthio-phenyl)-methyl]-pyran-4-on: Aus 16,6 g 3-Hydroxy-2-[hydroxy-(4-methylthio-phenyl)-methyl]-pyran-4-on erhält man durch Umsetzung mit Benzylbromid in N,N-Dimethylformamid und Kaliumcarbonat analog Beispiel 1a 3-Benzyloxy-2-[hydroxy-(4-methylthio-phenyl)-methyl]-pyran-4-on. Gelbliche Kristalle, Fp· 82-82,5 °C

c) 3-Benzyloxy-2-[(4-methylthio-phenyl)-(tetrahydro-pyran-2-yloxy)-methyl]-pyran-4-on: Aus 16,7 g 3-Benzyloxy-2-[hydroxy-(4-methylthio-phenyl)-methyl]-pyran-4-on erhält man durch Umsetzung mit 3,4-Dihydro-*2H*-pyran analog Beispiel 1b rohes 3-Benzyloxy-2-[(4-methylthio-phenyl)-(tetrahydro-pyran-2-yloxy)-methyl]-pyran-4-on als Diastereomerengemisch. $R_f$-Wert 0,65 (Kieselgel 60, Hexan/Dichlormethan/Essigester 1/1/1)

d) 3-Benzyloxy-1-(2-hydroxy-ethyl)-2-[(4-methylthio-phenyl)-(tetrahydro-pyran-2-yloxy)-methyl]-1*H*-pyridin-4-on: Aus 11,3 g 3-Benzyloxy-2-[(4-methylthio-phenyl)-(tetrahydro-pyran-2-yloxy)-methyl]-pyran-4-on erhält man durch Umsetzung mit Ethanolamin analog Beispiel 1c, nach Chromatographie 3-Benzyloxy-1-(2-hydroxy-ethyl)-2-[(4-methylthio-phenyl)-(tetrahydro-pyran-2-yloxy)-methyl]-*1H*-pyridin-4-on. Gelber Schaum. $R_f$ -Wert: 0,16 (Kieselgel 60, Essigester/Ethanol 9/1).

e) 3-Benzyloxy-1-(2-hydroxy-ethyl)-2-[hydroxy-(4-methylthio-phenyl)-methyl]-*1H*-pyridin-4-on: Aus 7,8 g 3-Benzyloxy-1-(2-hydroxy-ethyl)-2-[(4-methylthio-phenyl)-(tetrahydro-pyran-2-yloxy)-methyl]-*1H*-pyridin-4-on erhält man analog Beispiel 1d 3-Benzyloxy-1-(2-hydroxyethyl)-2-[hydroxy-(4-methylthio-phenyl)-methyl]-*1H*-pyridin-4-on. Fp: 120-125° C.

Beispiel 88: 9-Hydroxy-3-methoxy-1-phenyl-3,4-dihydro-*1H*-pyrido[2,1-c][1,4]oxazin-8-on

Aus 1,3 g 9-Benzyloxy-3-methoxy-1-phenyl-3,4-dihydro-1*H*-pyrido[2,1-c][1,4]oxazin-8-on erhält man durch Hydrierung analog Beispiel 1 9-Hydroxy-3-methoxy-1-phenyl-3,4-dihydro-*1H*-pyrido[2,1-c][1,4]oxazin-8-on. Farblose Kristalle Fp: 201,7-203,5 °C.

Das Ausgangsmaterial kann zum Beispiel wie folgt hergestellt werden:

a) 3-Benzyloxy-1-(2,2-dimethoxy-ethyl)-2-[phenyl-(tetrahydro-pyran-2-yloxy)-methyl]-*1H*-pyridin-4-on: 4,04 g 3-Benzyloxy-2-[phenyl-(tetrahydro-pyran-2-yloxy)-methyl]-pyran-4-on (Beispiel 1c) werden in 40 ml Ethanol und 4 ml 2-Aminoacetaldehyd-dimethylacetal [*CAS-Nr.: 22483-09-6*] während 45 Stunden am Rückfluss gekocht. Zum Aufarbeiten entfernt man das Ethanol am Rotationsverdampfer. Der Rückstand wird in 200 ml Essigester aufgenommen und dreimal mit je 50 ml Wasser gewaschen. Die organische Phase wird über Magnesiumsulfat getrocknet, filtriert und am Rotationsverdampfer zur Trockene eingedampft. Man erhält, nach Chromatographie 3-Benzyloxy-1-(2,2-dimethoxy-ethyl)-2-[phenyl-(tetrahydro-pyran-2-yloxy)-methyl]-*1H*-pyridin-4-on als Diastereomerengemisch. Oranges Harz, $R_f$-Wert: 0,45 (Kieselgel 60, Essigester/Ethanol 9/1).

b) 9-Benzyloxy-3-methoxy-1-phenyl-3,4-dihydro-*1H*-pyrido[2,1-c][1,4]oxazin-8-on 2,13 g 3-Benzyloxy-1-(2,2-dimethoxy-ethyl)-2-[phenyl-(tetrahydro-pyran-2-yloxy)-methyl]-*1H*-pyridin-4-on werden in 5 ml Methanol und 8,8 ml Salzsäure 2N während 2 Stunden am Rückfluss gekocht. Man destilliert am Rotationsverdampfer des Methanol ab, verdünnt den Rückstand mit Wasser und neutralisiert mit 8,8 ml Natronlauge 2N. Man extrahiert mit Essigester und wäscht die Extrakte zweimal mit Wasser. Die Extrakte werden getrocknet, filtriert und eingedampft. Nach Chromatographie erhält man: 1,3 g 9-Benzyloxy-3-methoxy-1-phenyl-3,4-dihydro-*1H*-pyrido[2,1-c][1,4]oxazin-8-on als Diastereomerengemisch. Gelbes Harz $R_f$-Wert: 0,45 (Kieselgel 60, Essigester/Ethanol 9/1).

Beispiel 89: 9-Hydroxy-1-phenyl-*1H*-pyrido[2,1-c][1,4]oxazin-3,8-dion

Beispiel 90: 9-Hydroxy-1-thiophen-2-yl-3,4-dihydro-*1H*-pyrido[2,1-c][1,4]oxazin-8-on

Aus 3-Benzyloxy-1-(2-hydroxy-ethyl)-2-(hydroxy-thiophen-2-yl-methyl)-*1H*-pyndin-4-on erhält man analog Beispiel 35 9-Hydroxy-1-thiophen-2-yl-3,4-dihydro-*1H*-pyrido[2,1-c][1,4]-oxazin-8-on. Beige Kristalle Fp: 226-229 °C.

Beispiel 91: 2-(Furan-2-yl-hydroxy-methyl)-3-hydroxy-1-(2-hydroxy-ethyl-*1H*-pyridin-4-on

Farblose Kristalle Fp: 180-82 °C

Beispiel 92: 2-(Furan-2-yl-hydroxy-methyl)-3-hydroxy-1-methyl-*1H*-pyridin-4-on

Beispiel 93: 1-Ethyl-3-hydroxy-2-(hydroxy-phenyl-methyl)-*1H*-pyridin-4-on

Farblose Kristalle Fp: 209-216 °C

Beispiel 94: 2-Benzyl-1-ethyl-3-hydroxy-*1H*-pyridin-4-on

Farblose Kristalle Fp: 179-180 °C

Beispiel 95: N-(2-Hydroxy-ethyl)-2-[3-hydroxy-2-(hydroxy-phenyl-methyl)-4-oxo-4H-pyridin-1-yl]-acetamid

Farblose Knstalle Fp:177-179 °C

Beispiel 96: 1-Ethyl-2-[(4-fluorphenyl)-hydroxy-methyl]-3-hydroxy-*1H*-pyridin-4-on

Farblose Kristalle Fp: 206-209 °C.

Beispiel 97: 1-Ethyl-2-(4-fluorbenzyl)-3-hydroxy-*1H*-pyridin-4-on

Farblose Kristalle Fp: 170-175 °C

Beispiel 98: [2-(4-Fluorbenzyl)-3-hydroxy-4-oxo-4H-pyridin-1-yl]-essigsäure methylester

Farblose Kristalle Fp: 208-212 °C

Beispiel 99: 3-Hydroxy-2-(hydroxy-p-tolyl-methyl)-1-methyl-*1H*-pyridin-4-on

Beispiel 100: 3-Hydroxy-1-methyl-2-(4-methylbenzyl)-*1H*-pyridin-4-on

Beispiel 101: 2-[(4-Chlorphenyl)-hydroxy-methyl]-3-hydroxy-1-methyl-*1H*-pyridin-4-on

Beispiel 102: 2-(4-Chlorbenzyl)-3-hydroxy-1-methyl-*1H*-pyridin-4-on

Beispiele A bis D: Pharmazeutische Präparate

Unter dem Ausdruck "Wirkstoff" ist nachstehend eine Verbindung der Formel in freier Form oder in Form eines pharmazeutisch verwendbaren Salzes, zu verstehen, insbesondere eine solche Verbindung, die als Produkt in einen der vorstehenden Beispiele beschrieben ist.

Beispiel A: Tabletten, enthaltend je 200 mg Wirkstoff, können wie folgt hergestellt werden:

| Zusammensetzung (10000 Tabletten) | |
|---|---|
| Wirkstoff | 2000,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert Nach dem Trocknen mischt man den Rest der Kartoffelstärke, das Magnesiumstearat, den Talk und das Siliciumdioxid zu und presst das Gemisch zu Tabletten von je 295,0 mg Gewicht und 200 mg Wirkstoffgehalt, die gewünschtenfalls mit Teilkerben zu feineren Anpassung der Dosierung versehen sein können.

Beispiel B: Lacktabletten, enthaltend je 400 mg Wirkstoff, können wie folgt hergestellt werden.

Zusammensetzung (1000 Tabletten)

| | |
|---|---|
| Wirkstoff | 400,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten verpresst und diese mit einer Lösung der Hydroxypropylmethylcellulose und des Schellacks in Dichlormethan lackiert; Endgewicht der Lacktablette: 583 mg.

Beispiel C: Gelatinesteckkapseln, enthaltend 500 mg Wirkstoff, können z. B. folgendermassen hergestellt werden:

Zusammensetzung (für 1000 Kapseln):

| | |
|---|---|
| Wirkstoff | 500,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. dann wird zunächst die Lactose durch ein Sieb mit einer Maschenweite von 0,6 mm und dann die mikrokristalline Zellulose durch ein Sieb mit einer Maschenweite von

0,9 mm hinzugesiebt. daraufhin wird erneut 10 Minuten innig gemischt. Zuletzt wird das Magnesiumstearat durch ein Sieb mit einer Maschenweite von 0,8 mm hinzugesiebt. Nach 3-minütigem weiteren Mischen werden je 790 mg der erhaltenen Formulierung in Gelatinesteckkapseln passender Grösse abgefüllt.

Beispiel D: Orales Suspensionspulver, enthaltend 300 mg Wirkstoff, kann beispielsweise wie folgt hergestellt werden

Zusammensetzung (1 Applikation):

| | |
|---|---|
| Wirkstoff | 300 mg |
| Hydroxypropylcellulose (Klucel HF) | 50 mg |
| Weinsäure | 100 mg |
| Natriumlaurylsulfat | 100 mg |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird die mikrokristalline Zellulose durch ein Sieb mit einer Maschenweite von 0,9 mm hinzugesiebt. Daraufhin wird erneut 10 Minuten innig gemischt. Zuletzt wird die Weinsäure durch ein Sieb mit einer Maschenweite von 0,8 mm hinzugesiebt. Nach 3-minütigem weiteren Mischen wird die Mischung in ein Gefäss mit mindestens 10 ml Fassungsvermögen gefullt Zum Gebrauch wird mit Wasser auf 10 ml aufgefüllt und die Mischung kräftig geschüttelt.

## Patentansprüche

1. Verbindung der Formel I

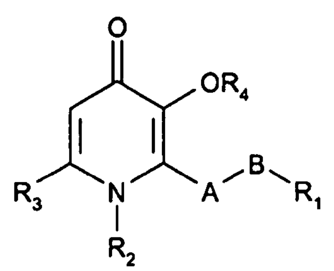

(I)

worin

$R_1$ Wasserstoff, Halogen, Niederalkyl, Niederalkoxy, unsubstituiertes Niederalkoxycarbonyl, Amino, Niederalkylamino, Diniederalkylamino, Aminocarbonyl, N-Niederalkylaminocarbonyl, N,N-Diniederalkylaminocarbonyl, Carboxyl, unsubstituiertes Niederalkylsulfonyl, Aminosulfonyl, Cyano, Hydroxy, Nitro, Tetrazolyl, oder Niederalkylendioxy, das mit der Gruppe B ein heterocyclisches sauerstoffhaltiges Ringsystem bildet, worin der heterocyclische Ring ein gesättigter oder ungesättigter Ring mit fünf bis und mit sieben Ringgliedern darstellt, von denen mindestens eines Sauerstoff ist und in welchem gegebenenfalls ein oder mehrere weitere Heteroatome vorhanden sind, wobei der Ring unsubstituiert oder mit Niederalkyl, Niederalkoxy oder Hydroxy substituiert sein kann und ein oder mehrere Ringkohlenstoffatome zu Carbonyl oxidiert sein können;

$R_2$ Wasserstoff, unsubstituiertes oder mit Halogen, Hydroxy oder Trifluoromethyl substituiertes Niederalkyl, Niederalkylenhydroxy, Niederalkylen-niederalkoxy, unsubstituiertes Niederalkylencarboxy, unsubstituiertes Niederalkylencarbonylniederalkoxy, Niederalkylenamin, N-Niederalkanoylniederalkylenamin, unsubstituiertes Niederalkanoyloxyniederalkylen oder Formylniederalkylen;

$R_3$ Wasserstoff, Niederalkyl, Carboxyl, Niederalkanoyloxyniederalkylen, Aminocarbonyl, N-Niederalkylaminocarbonyl oder unsubstituiertes N,N-Diniederalkylaminocarbonyl;

$R_4$ Wasserstoff;

A unsubstituiertes oder mit Niederalkyl, Halogen oder Hydroxy substituiertes Methylen, Carbonyl oder

zusammen mit $R_2$ und den mit ihnen verbunden Atomen einen sauerstoffhaltigen heterocyclischen Ring bestehend aus einem gesättigten oder ungesättigten Ring mit fünf bis und mit sieben Ringgliedern, von denen mindestens eines Sauerstoff ist und in welchem gegebenenfalls ein oder mehrere weitere Heteroatome vorhanden sind, wobei der Ring unsubstituiert oder mit Niederalkyl, Niederalkoxy oder Hydroxy substituiert sein kann und ein oder mehrere Ringkohlenstoffatome zu Carbonyl oxidiert sein können; und

B einfach oder mehrfach substituiertes oder unsubstituiertes Phenyl oder Naphthyl, wobei die Subsitutenten aus Niederalkyl, Niederalkoxy, Hydroxy, Nitro, Halogen, Trifluoromethyl, Carboxy, Amino und Cyano ausgewählt werden, oder einfach oder mehrfach substituiertes oder unsubstituiertes Heteroaryl, wobei Heteroaryl ein aromatischer Rest mit 5 bis und mit 7 Ringatomen darstellt, worin mindestens eines der Ringatome ein Heteroatom ist, und die Substituenten aus Niederalkyl, Halogen und Trifluoromethyl ausgewählt werden;

bedeuten: und deren Stereoisomere, Tautomere und Salze, wobei das Präfix "Nieder" einen Rest bis und mit 7 Kohlenstoffatomen bezeichnet und, falls nicht anders angegeben, Niederalkyl sowie Niederalkylen für sich oder als Bestandteil anderer Gruppen alternativ substituiert sein können mit Halogen, Hydroxy oder Trifluoromethyl.

**2.** Verbindung nach Anspruch 1, worin

$R_1$ Wasserstoff, Halogen, Niederalkyl, unsubstituiertes Niederalkoxycarbonyl, Amino, unsubstituiertes Niederalkylamino, unsubstituiertes Diniederalkylamino, Aminocarbonyl, N-Niederalkylaminocarbonyl, N,N-Diniederalkylaminocarbonyl, Carboxyl, Cyano, Hydroxy, Nitro oder Niederalkylendioxy, das mit der Gruppe B ein heterocyclisches sauerstoffhaltiges Ringsystem bildet;

$R_2$ Wasserstoff, unsubstituiertes oder mit Halogen, Hydroxy oder Trifluoromethyl substituiertes Niederalkyl, Niederalkylenhydroxy, Niederalkylen-niederalkoxy, unsubstituiertes Niederalkylencarboxy, unsubstituiertes Niederalkylencarbonylniederalkoxy, Niederalkylenamin oder N-Niederalkanoylniederalkylenamin;

$R_3$ Wasserstoff, Niederalkyl, Carboxyl, Niederalkanoyloxyniederalkylen oder N-Niederalkylaminocarbonyl;

$R_4$ Wasserstoff;

A unsubstituiertes oder mit Niederalkyl, Halogen oder Hydroxy substituiertes Methylen, Carbonyl oder zusammen mit $R_2$ und den mit ihnen verbunden Atomen einen sauerstoffhaltigen heterocyclischen Ring; und

B einfach oder mehrfach substituiertes oder unsubstituiertes Phenyl oder Naphthyl oder einfach oder mehrfach substituiertes oder unsubstituiertes Heteroaryl;

bedeuten: und deren Stereoisomere, Tautomere und pharmazeutisch verträgliche Salze, wobei, falls nicht anders angegeben, Niederalkyl sowie Niederalkylen für sich oder als Bestandteil anderer Gruppen alternativ substituiert sein können mit Halogen, Hydroxy oder Trifluoromethyl.

**3.** Verbindung nach Anspruch 1, worin

$R_1$ Wasserstoff, Halogen, Niederalkyl, Niederalkoxy, Cyano, Hydroxy oder Nitro;

$R_2$ unsubstituiertes oder mit Halogen, Hydroxy oder Trifluoromethyl substituiertes Niederalkyl, Niederalkylenhydroxy oder Niederalkylenamin;

$R_3$ und $R_4$ Wasserstoff;

A unsubstituiertes oder mit Niederalkyl, Halogen oder Hydroxy substituiertes Methylen, Carbonyl oder zusammen mit $R_2$ und den mit ihnen verbundenen Atomen einen unsubstituierten sauerstoffhaltigen heterocyclischen Ring; und

B einfach substituiertes oder unsubstituiertes Phenyl oder Naphthyl oder einfach substituiertes oder unsubstituiertes Heteroaryl;

bedeuten; und deren Stereoisomere, Tautomere und pharmazeutisch verträgliche Salze, wobei, falls nicht anders angegeben, Niederalkyl sowie Niederalkylen für sich oder als Bestandteil anderer Gruppen alternativ substituiert sein können mit Halogen, Hydroxy oder Trifluoromethyl.

**4.** Verbindung nach Anspruch 1, worin

$R_1$ Wasserstoff, Halogen oder unsubstituiertes Niederalkyl;

$R_2$ unsubstituiertes oder mit Halogen, Hydroxy oder Trifluoromethyl substituiertes Niederalkyl oder Nie-

deralkylenhydroxy;

| | |
|---|---|
| $R_3$ und $R_4$ | Wasserstoff; |
| A | unsubstituiertes oder mit Niederalkyl, Halogen oder Hydroxy substituiertes Methylen oder zusammen mit $R_2$ und den mit ihnen verbundenen Atomen einen unsubstituierten sauerstoffhaltigen heterocyclischen Ring; und |
| B | einfach substituiertes oder unsubstituiertes Phenyl oder Naphthyl oder einfach substituiertes oder unsubstituiertes Heteroaryl; |
| bedeuten; | und deren Stereoisomere, Tautomere und pharmazeutisch verträgliche Salze, wobei, falls nicht anders angegeben, Niederalkyl sowie Niederalkylen für sich oder als Bestandteil anderer Gruppen alternativ substituiert sein können mit Halogen, Hydroxy oder Trifluoromethyl. |

**5.** Eine Verbindung nach Anspruch 1 aus der Gruppe bestehend aus
3-Hydroxy-1-(2-hydroxy-ethyl)-2-(hydroxy-phenyl-methyl)-1*H*-pyridin-4-on;
3-Hydroxy-1-(2-hydroxy-ethyl)-2-(4-methyl-benzyl)-1*H*-pyridin-4-on;
3-Hydroxy-1-(2-hydroxy-ethyl)-2-(hydroxy-4-fluorphenyl-methyl)-1 *H*-pyridin-4-on;
3-Hydroxy-1-(2-hydroxy-ethyl)-2-(4-fluorbenzyl)-*1H*-pyridin-4-on;
3-Hydroxy-1-(2-hydroxy-ethyl)-2-(hydroxy-4-chlorphenyl-methyl)-1*H*-pyridin-4-on; und
3-Hydroxy-1-(2-hydroxy-ethyl)-2-(4-chlorbenzyl)-*1H*-pyridin-4-on;
und deren Stereoisomeren, Tautomeren und pharmazeutisch verträglichen Salzen.

**6.** Eine Verbindung aus der Gruppe bestehend aus
3-Hydroxy-2-[pyridin-3-yl-(acetoxy)-methyl]-1-methyl-1 *H*-pyridin-4-on;
Essigsäure-[1-(2-acetoxy-ethyl)-3-hydroxy-4-oxo-1,4-dihydropyridin-2-yl-methyl]-phenylmethylester;
Essigsäure 1-(2-acetoxy-ethyl)-6-[acetoxy-(4-fluor-phenyl)-methyl]-5-hydroxy-4-oxo-1,4-dihydro-pyridin-2-ylmethylester;
2-(4-Fluorbenzyl)--3-hydroxy-1-(2-morphohn-4-yl-ethyl)-*1H*-pyridin-4-on;
4-[2-(4-Fluorbenzyl)--3-hydroxy-4-oxo-4H-pyridin-1-ylmethyl]-benzoesäure;
Essigsäure-[1-(2-acetoxy-ethyl)-3-hydroxy-4-oxo-1,4-dihydropyridin-2-yl-]-(2-fluorphenyl)-methylester; und
N-(2-Hydroxy-ethyl)-2-[3-hydroxy-2-(hydroxy-phenyl-methyl)-4-oxo-4H-pyridin-1-yl]-acetamid;
und deren Stereoisomeren, Tautomeren und pharmazeutisch verträglichen Salzen.

**7.** Pharmazeutische Präparate enthaltend eine Verbindung gemäss einem der Ansprüche 1 bis 6 und mindestens ein pharmazeutisch verwendbares Trägermaterial.

**8.** Eine Verbindung gemäss einem der Ansprüche 1 bis 6 zur Anwendung in einem Verfahren zur therapeutischen Behandlung des tierischen oder menschlichen Körpers.

**9.** Eine Verbindung gemäss einem der Ansprüche 1 bis 6 zur Verwendung bei der Behandlung von Krankheiten, die einen Eisenüberschuss im menschlichen oder tierischen Körper verursachen oder durch ihn verursacht werden.

**10.** Verwendung einer Verbindung gemäss einem der Ansprüche 1 bis 6 zur Herstellung pharmazeutischer Präparate.

**11.** Verwendung einer Verbindung gemäss einem der Ansprüche 1 bis 6 zur Herstellung pharmazeutischer Präparate zur Behandlung eines Eisenüberschusses im menschlichen oder tierischen Körper.

**12.** Verfahren zur Herstellung einer Verbindung nach Formel I gemäss Anspruch 1, Stereoisomeren, Tautomeren und pharmazeutisch verträglicher Salze davon, **dadurch gekennzeichnet, dass** man eine Verbindung der Formel II

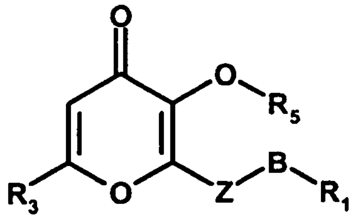

(II)

worin $R_1$, $R_3$, und B wie unter Formel I definiert sind, Z unsubsituiertes oder mit Niederalkyl, Halogen oder Hydroxy

substituiertes Methylen, wobei das Präfix "Nieder" wie in Anspruch 1 definiert ist und wobei gegebenfalls Substituenten in geschützter Form vorliegen können, oder Carbonyl bedeutet und $R_5$ gleich $R_4$ wie unter Formel I definiert ist oder gegebenenfalls eine geeignete Schutzgruppe darstellt, mit einer Verbindung der Formel III

$$H_2N\text{-}R_2 \qquad \textbf{(III)} \qquad \text{(III)}$$

worin $R_2$ wie unter Formel I definiert ist, umsetzt zu einer Verbindung der Formel IV

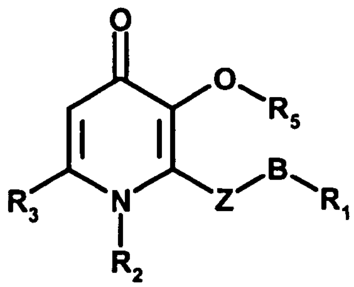

**(IV)**

worin $R_1$, $R_2$, $R_3$, $R_5$, B und Z wie unter Formeln I und II definiert sind, und diese Verbindung

a) wenn erforderlich, durch gleichzeitige Abspaltung einer Schutzgruppe $R_5$ und einer gegebenfalls an der Gruppe Z vorhanden Schutzgruppe in eine Verbindung der Formel I umwandelt, und, wenn erwünscht, in eine andere Verbindung der Formel umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, oder

b) wenn erforderlich, nach Abspaltung einer Schutzgruppe $R_5$ oder einer gegebenfalls an der Gruppe Z vorhanden Schutzgruppe zunächst in eine andere geschützte Form einer Verbindung der Formel I umwandelt und, wenn erwünscht, in eine geschützte Form einer anderen Verbindung der Formel I umwandelt, und anschliessend durch Abspaltung der verbleibenden Schutzgruppen in eine Verbindung der Formel I umwandelt, und, wenn erwünscht, in eine andere Verbindung der Formel umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

## Claims

**1.** Compound of formula I

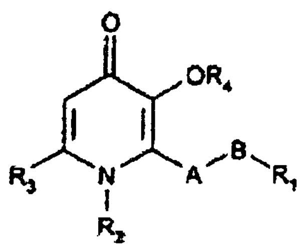

(I)

wherein

$R_1$ signifies hydrogen, halogen, lower alkyl, lower alkoxy, unsubstituted lower alkoxycarbonyl, amino, lower alkylamino, di-lower-alkylamino, aminocarbonyl, N-lower-alkylaminocarbonyl, N,N-di-lower-alkylaminocarbonyl, carboxyl, unsubstituted lower alkylsulphonyl, aminosulphonyl, cyano, hydroxy, nitro, tetrazolyl, or lower alkylenedioxy, which with group B forms a heterocyclic, oxygen-containing ring system, in which the heterocyclic ring is a saturated or unsaturated ring with five up to and including seven ring members, at least one of which is oxygen and in which one or more further hetero atoms are optionally present, whereby the ring may be unsubstituted or substituted by lower alkyl, lower alkoxy or hydroxy, and one or more ring carbon atoms may be oxidised to carbonyl;

$R_2$ signifies hydrogen, unsubstituted lower alkyl or lower alkyl substituted by halogen, hydroxy or trifluoromethyl, lower alkylenehydroxy, lower alkylene-lower-alkoxy, unsubstituted lower alkylenecarboxy, unsubstituted lower alkylenecarbonyl-lower-alkoxy, lower alkyleneamine, N-loweralkanoyl-lower-alkylene-amine, unsubstituted lower alkanoyloxy-lower-alkylene or formyl-lower-alkylene;

$R_3$ signifies hydrogen, lower alkyl, carboxyl, lower alkanoyloxy-lower-alkylene, aminocarbonyl, N-lower-alkylaminocarbonyl or unsubstituted N,N-di-lower-alkylaminocarbonyl;

$R_4$ signifies hydrogen;

A unsubstituted methylene or methylene substituted by lower alkyl, halogen or hydroxy, carbonyl or, together with $R_2$ and the atoms linked thereto, forms an oxygen-containing, heterocyclic ring consisting of a saturated or unsaturated ring with five up to and including seven ring members, at least one of which is oxygen and in which one or more further hetero atoms are optionally present, whereby the nng may be unsubstituted or substituted by lower alkyl, lower alkoxy or hydroxy, and one or more ring carbon atoms may be oxidised to carbonyl; and

B signifies phenyl or napthyl which are either unsubstituted or substituted once or many times, whereby the substituents are selected from lower alkyl, lower alkoxy, hydroxy, nitro, halogen, tnfluoromethyl, carboxy, ammo and cyano, or it signifies heteroaryl which is either unsubstituted or substituted once or many times, whereby heteroaryl is an aromatic radical with five up to and including seven ring atoms, with at least one of the ring atoms being a hetero atom, and the substituents may be selected from lower alkyl, halogen and trifluoromethyl;

and the stereoisomers, tautomers and salts thereof, whereby the prefix "lower" indicates a radical with up to and including 7 carbon atoms, and if not otherwise stated, lower alkyl and lower alkylene themselves or as a constituents of other groups may be alternatively substituted by halogen, hydroxy or trifluoromethyl.

**2.** Compounds according to claim 1, wherein

$R_1$ hydrogen, halogen, lower alkyl, unsubstituted lower alkoxycarbonyl, amino, unsubstituted lower alkylamino, unsubstituted di-lower-alkylarnino, aminocarbonyl, N-lower-alkylaminocarbonyl, N,N-di-lower-alkylaminocarbonyl, carboxyl, cyano, hydroxy, nitro or lower alkylenedioxy, which with group B forms a heterocyclic oxygen-containing ring system;

$R_2$ signifies hydrogen, lower alkyl which is either unsubstituted or substituted by halogen, hydroxy or trifluoromethyl, lower alkylenehydroxy, lower-alkylene-lower-alkoxy, unsubstituted lower alkylenecarboxy, unsubstituted lower alkylenecarbonyl-lower-alkoxy, lower alkylene-amine or N-lower-alkanoyl-lower-alkylene-amine;

$R_3$ signifies hydrogen, lower alkyl, carboxyl, lower alkanoyloxy-lower-alkylene or N-lower-alkylaminocarbonyl;

$R_4$ signifies hydrogen;

A signifies unsubstituted methylene or methylene substituted by lower alkyl, halogen or hydroxy, carbonyl or, together with $R_2$ and the atoms linked thereto, forms an oxygen-containing, heterocyclic ring; and

B signifies phenyl or napthyl which are either unsubstituted or substituted once or many times, or heteroaryl which is either unsubstituted or substituted once or many times;

and the stereoisomers, tautomers and pharmaceutically acceptable salts, whereby if not otherwise stated, lower alkyl and lower alkylene themselves or as a constituents of other groups may be alternatively substituted by halogen, hydroxy or trifluoromethyl.

**3.** Compounds according to claim 1, wherein

$R_1$ signifies hydrogen, halogen, lower alkyl, lower alkoxy, cyano, hydroxy or nitro;

$R_2$ signifies lower alkyl which is either unsubstituted or substituted by halogen, hydroxy or trifluoromethyl, lower alkylenehydroxy, or lower-alkylene-amine;

$R_3$ and $R_4$ signify hydrogen;

A signifies unsubstituted methylene or methylene substituted by lower alkyl, halogen or hydroxy, carbonyl or, together with $R_2$ and the atoms linked thereto, forms an unsubstituted, oxygen-containing, heterocyclic ring; and

B signifies once-substituted or unsubstituted phenyl or naphthyl or once-substituted or unsubstituted heteroaryl;

and the stereoisomers, tautomers and pharmaceutically acceptable salts, whereby if not otherwise stated, lower alkyl and lower alkylene themselves or as a constituents of other groups may be alternatively substituted by halogen, hydroxy or trifluoromethyl

**4.** Compound according to claim 1, wherein

$R_1$ signifies hydrogen, halogen or unsubstituted lower alkyl;
$R_2$ signifies lower alkyl which is either unsubstituted or substituted by halogen, hydroxy or trifluoromethyl, or lower alkylenehydroxy;
$R_3$ and $R_4$ signify hydrogen;
A signifies unsubstituted methylene or methylene substituted by lower alkyl, halogen or hydroxy, or, together with $R_2$ and the atoms linked thereto, forms an unsubstituted, oxygen-containing, heterocyclic ring; and
B signifies once-substituted or unsubstituted phenyl or naphthyl or once-substituted or unsubstituted heteroaryl;

and the stereoisomers, tautomers and pharmaceutically acceptable salts, whereby if not otherwise stated, lower alkyl and lower alkylene themselves or as a constituents of other groups may be alternatively substituted by halogen, hydroxy or trifluoromethyl.

**5.** A compound according to claim 1 from the group consisting of
3-hydroxy-1-(2-hydroxyethyl)-2-hydroxyphenylmethyl)-1*H*-pyridin-4-one;
3-hydroxy-1-(2-hydroxyethyl)-2-(4-methylbenzyl)-1*H*-pyridin-4-one;
3-hydroxy-1-(2-hydroxyethyl)-2-(hydroxy-4-fluorophenylmethyl)-1*H*-pyridin-4-one;
3-hydroxy-1-(2-hydroxyethyl)-2-(4-fluorobenzyl)-1*H*-pyridin-4-one;
3-hydroxy-1-(2-hydroxyethyl)-2-(hydroxy-4-chlorophenylmethyl)-1*H*-pyridin-4-one;
3-hydroxy-1-(2-hydroxyethyl)-2-(4-chlorobenzyl)-1*H*-pyridin-4-one;
and the stereoisomers, tautomers and pharmaceutically acceptable salts thereof.

**6.** A compound from the group consisting of
3-hydroxy-2-[pyridin-3-yl(acetoxy)-methyl]-1-methyl-1*H*-pyridin-4-one;
acetic acid-[1-(2-acetoxyethyl)-3-hydroxy-4-oxo-1,4-dihydropyridin-2-yl-methyl]-phenylmethylester;
acetic acid-[1-(2-acetoxyethyl)-6-[acetoxy-(4-fluorophenyl)-methyl]-5-hydroxy-4-oxo-1,4-dihydropyridin-2-ylmethylester;
2-(4-fluorobenzyl)-3-hydroxy-1-(2-morpholin-4-yl-ethyl)-1*H*-pyridin-4-one;
4-[2-(4-fluorobenzyl)-3-hydroxy-4-oxo-4H-pyridin-1-ylrnethyl]benzoic acid;
acetic acid-[1-(2-acetoxyethyl)-3-hydroxy-4-oxo-1,4-dihydropyndin-2-yl]-(2-fluorophenyl)-methylester; and
N-(2-hydroxyethyl)-2-[3-hydroxy-2-(hydroxyphenylmethyl)-4-oxo-4H-pyridin-1-yl]-acetamide;
and the stereoisomers, tautomers and pharmaceutically acceptable salts thereof.

**7.** Pharmaceutical preparations containing a compound according to one of claims 1 to 6 and at least one pharmaceutically employable carrier material.

**8.** A compound according to one of claims 1 to 6 for use in a process for the therapeutic treatment of an animal or human body.

**9.** A compound according to one of claims 1 to 6 for use in the treatment of pathological conditions which cause or are caused by an iron excess in the human or animal body.

**10.** Use of a compound according to one of claims 1 to 6 in the production of pharmaceutical preparations.

**11.** Use of a compound according to one of claims 1 to 6 in the production of pharmaceutical preparations for the treatment of an excess of iron in the human or animal body.

**12.** Process for the production of a compound of formula I according to claim 1, stereoisomers, tautomers and pharmaceutically acceptable salts thereof, whereby a compound of formula II

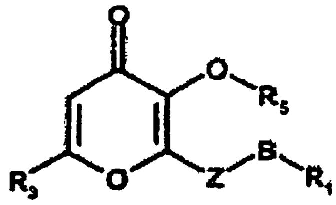

(II)

wherein $R_1$, $R_3$ and B are defined under formula I, Z signifies unsubstituted methylene or methylene substituted by lower alkyl, halogen or hydroxy, whereby the prefix "lower" is defined as in claim 1, and whereby substituents may be optionally present in protected form, or it signifies carbonyl, and $R_5$ has the same definition as $R_4$ under formula I, or optionally represents an appropriate protecting group,
is reacted with a compound of formula III

$$H_2N\text{-}R_2 \qquad \textbf{(III)} \qquad\qquad \text{(III)}$$

wherein $R_2$ is defined under formula I, to form a compound of formula IV

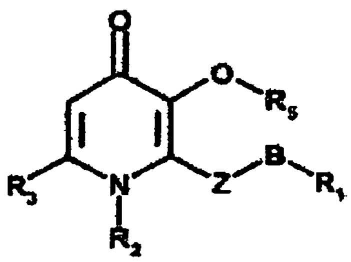

(IV)

wherein $R_1$, $R_2$, $R_3$, $R_5$, B and Z are defined as under formulae I and II, and this compound

a) is converted into a compound of formula I, if necessary by simultaneously cleaving a protecting group $R_5$ and a protecting group optionally present at the group Z, and if desired, into a different compound of formula I, and/or if desired, a resulting salt is converted into the free compound or into a different salt, and/or, if desired, a resulting free compound of formula I with salt-forming properties is converted into a salt, or
b) if necessary, after cleaving a protecting group $R_5$ or a protecting group optionally present at the group Z, is converted first of all into a different protected form of a compound of formula I, and, if desired, converted into a protected form of a different compound of formula I, and is subsequently converted into a compound of formula I by cleaving the remaining protecting groups, and if desired, converted into a different compound of formula I, and/or if desired, a resulting salt is converted into the free compound or into a different salt, and/or, if desired, a resulting free compound of formula I with salt-forming properties is converted into a salt.

**Revendications**

**1.** Composé de formule I

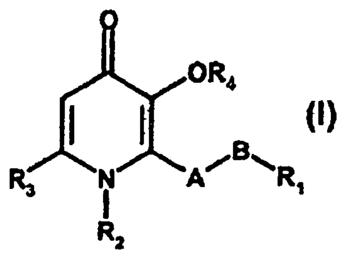

(I)

dans laquelle

$R_1$ est un atome d'hydrogène ou d'halogène, un groupe alkyle inférieur, alcoxy inférieur, (alcoxy inférieur) carbonyle non substitué, amino, (alkyle inférieur)amino, di(alkyle inférieur)amino, aminocarbonyle, N-(alkyle inférieur)aminocarbonyle, N,N-di(alkyle inférieur)aminocarbonyle, carboxyle, (alkyle inférieur)sulfonyle non substitué, aminosulfonyle, cyano, hydroxy, nitro, tétrazolyle ou (alkylène inférieur)dioxy, qui avec le groupe B forme un système cyclique hétérocyclique oxygéné, où le noyau hétérocyclique représente un noyau saturé ou insaturé ayant de 5 à 7 chaînons, limites comprises, dont au moins l'un est un atome d'oxygène, et où éventuellement sont présents un ou plusieurs autres hétéroatomes, le noyau pouvant être non substitué ou substitué par des substituants allcyle inférieur, alcoxy inférieur ou hydroxy, et un ou plusieurs atomes de carbone cyclique pouvant être oxydés en carbonyle ;
$R_2$ est un atome d'hydrogène ou un groupe alkyle inférieur, (alkylène inférieur)-hydroxy, (alkylène inférieur) (alcoxy inférieur) non substitué ou substitué par des substituants halogéno, hydroxy ou trifluorométhyle, un groupe (alkylène inférieur)carboxy non substitué, (alkylène inférieur)carbonyl(alcoxy inférieur) non substitué, (alkylène inférieur)-amine, N-(alcanoyle inférieur)(alkylène inférieur)amine, (alcanoyle inférieur)oxy(alkylène inférieur) ou formyl(alkylène inférieur) non substitué ;
$R_3$ est un atome d'hydrogène ou un groupe alkyle inférieur, carboxyle, (alcanoyle inférieur)oxy(alkylène inférieur), aminocarbonyle, N-(alkyle inférieur)aminocarbonyle ou N,N-di(alkyle inférieur)aminocarbonyle non substitué ;
$R_4$ est un atome d'hydrogène ;
A est un radical méthylène non substitué ou substitué par des substituants alkyle inférieur, halogéno ou hydroxy, un groupe carbonyle, ou encore forme avec $R_2$ et les atomes qui y sont liés un anneau hétérocyclique oxygéné constitué d'un cycle saturé ou insaturé ayant de 5 à 7 chaînons, limites comprises, dont au moins l'un est l'oxygène, et dans lequel éventuellement sont présents un ou plusieurs autres hétéroatomes, le cycle pouvant être non substitué ou substitué par des substituants allcyle inférieur, alcoxy inférieur ou hydroxy, et un ou plusieurs atomes de carbone cycliques pouvant être oxydés en carbonyle ; et
B est un groupe phényle ou naphtyle mono- ou poly-substitué ou non substitué, les substituants étant choisis parmi les groupes alkyle inférieur, alcoxy inférieur, hydroxy, nitro, halogéno, trifluorométhyle, carboxy, amino et cyano, ou encore un groupe hétéroaryle mono- ou poly-substitué ou non substitué, le radical hétéroaryle étant un radical aromatique ayant 5 à 7 atomes cycliques, limites comprises, au moins l'un des atomes cycliques étant un hétéroatome, et les substituants étant choisis parmi les groupes alkyle inférieur, halogéno et trifluorométhyle ;

et leurs stéréoisomères, tautomères et sels, l'adjectif "inférieur" désignant un radical ayant jusqu'à 7 atomes de carbone et, sauf mention contraire, les groupes alkyle inférieur et alkylène inférieur peuvent, en soi ou en tant que constituant d'autres groupes, être comme alternative substitués par des substituants halogéno, hydroxy ou trifluorométhyle.

**2.** Composé selon la revendication 1, dans lequel :

$R_1$ est un atome d'hydrogène ou d'halogène ou un groupe alkyle inférieur, (alcoxy inférieur)carbonyle non substitué, amino, (alkyle inférieur)amino non substitué, di(alkyle inférieur)amino non substitué, aminocarbonyle, N-(alkyle inférieur)aminocarbonyle, N,N-di(akyle inférieur)aminocarbonyle, carboxyle, cyano, hydroxy, nitro ou (alkylène inférieur)dioxy, qui avec le groupe B forme un système cyclique hétérocyclique oxygéné ;
$R_2$ est un atome d'hydrogène ou un groupe alkyle inférieur, (alkylène inférieur)-hydroxy, (alkylène inférieur) (alcoxy inférieur) non substitué ou substitué par des substituants halogéno, hydroxy ou trifluorométhyle, un groupe (alkylène inférieur)carboxy non substitué, (alkylène inférieur)carbonyl(alcoxy inférieur) non substitué, (alkylène inférieur)-amine, ou N-(alcanoyle inférieur)(alkylène inférieur)amine ;

$R_3$ est un atome d'hydrogène ou un groupe alkyle inférieur, carboxyle, (alcanoyle inférieur)oxy(alkylène inférieur) ou N-(alkyle inférieur)aminocarbonyle ;
$R_4$ est un atome d'hydrogène ;
A est un radical méthylène non substitué ou substitué par des substituants alkyle inférieur, halogéno ou hydroxy, un groupe carbonyle, ou encore forme avec $R_2$ et les atomes qui y sont liés un anneau hétérocyclique oxygéné ; et
B est un groupe phényle ou naphtyle mono- ou poly-substitué ou non substitué, ou un groupe hétéroaryle mono- ou poly-substitué ou non substitué ;

et leurs stéréoisomères, tautomères et sels compatibles d'un point de vue pharmaceutique, où, sauf mention contraire, les groupes alkyle inférieur et alkylène inférieur peuvent, en soi ou aussi en tant que constituant d'autres groupes, être substitués par des substituants halogéno, hydroxy ou trifluorométhyle.

**3.** Composé selon la revendication 1, dans lequel :

$R_1$ est un atome d'hydrogène ou d'halogène ou un groupe alkyle inférieur, alcoxy inférieur, cyano, hydroxy ou nitro ;
$R_2$ est un radical alkyle inférieur, (alkylène inférieur)-hydroxy ou (alkylène inférieur)-amine non substitué ou substitué par des substituants halogéno, hydroxy ou trifluorométhyle ;
$R_3$ et $R_4$ sont des atomes d'hydrogène ;
A est un radical méthylène non substitué ou substitué par des substituants alkyle inférieur, halogéno ou hydroxy, un groupe carbonyle, ou forme avec $R_2$ et les atomes qui y sont liés un anneau hétérocyclique oxygéné non substitué ; et
B est un groupe phényle ou naphtyle monosubstitué ou non substitué, ou hétéroaryle monosubstitué ou non substitué ;

et leurs stéréoisomères, tautomères et sels compatibles d'un point de vue pharmaceutique, où, sauf mention contraire, les groupes alkyle inférieur et alkylène inférieur peuvent, en soi ou aussi en tant que constituant d'autres groupes, être substitués par des substituants halogéno, hydroxy ou trifluorométhyle.

**4.** Composé selon la revendication 1, dans lequel :

$R_1$ est un atome d'hydrogène ou d'halogène ou un groupe alkyle inférieur non substitué ;
$R_2$ est un groupe alkyle inférieur ou (alkylène inférieur)-hydroxy non substitué ou substitué par des substituants halogéno, hydroxy ou trifluorométhyle ;
$R_3$ et $R_4$ sont des atomes d'hydrogène ;
A est un radical méthylène non substitué ou substitué par des substituants allcyle inférieur, halogéno ou hydroxy, ou forme avec $R_2$ et les atomes qui y sont liés un anneau hétérocyclique oxygéné non substitué ; et
B est un groupe phényle ou naphtyle monosubstitué ou non substitué ou un groupe hétéroaryle monosubstitué ou non substitué ;

et leurs stéréoisomères, tautomères et sels compatibles d'un point de vue pharmaceutique, où, sauf mention contraire, les groupes alkyle inférieur et allcylène inférieur peuvent, en soi comme en tant que constituant d'autres groupes, être substitués par des substituants halogéno, hydroxy ou trifluorométhyle.

**5.** Composé selon la revendication 1, choisi dans le groupe consistant en les composés suivants :

3-hydroxy-1-(2-hydroxy-éthyl)-2-(hydroxy-phényl-méthyl)-1H-pyridin-4-one ;
3-hydroxy-1-(2-hydroxy-éthyl)-2-(4-méthyl-benzyl)-1H-pyridin-4-one ;
3-hydroxy-1-(2-hydroxy-éthyl)-2-(hydroxy-4-fluorophényl-méthyl)-1H-pyridin-4-one ;
3-hydroxy-1-(2-hydroxy-éthyl)-2-(4-fluorobenzyl)-1H-pyridin-4-one ;
3-bydroxy-1-(2-bydroxy-éthyl)-2-(hydroxy-4-chloropbényl-méthyl)-1H-pyridin-4-one ; et
3-hydroxy-1-(2-hydroxy-éthyl)-2-(4-chlorobenzyl)-1H-pyridin-4-one ;

et leurs stéréoisomères, tautomères et sels compatibles d'un point de vue pharmaceutique.

**6.** Composé choisi dans le groupe consistant en les composés suivants :

3-hydroxy-2-[pyridin-3-yl-(acétoxy)-méthyl]-1H-pyridin-4-one ;
ester [1-(2-acétoxy-éthyl)-3-hydroxy-4-oxo-1,4-dihydropyridin-2-yl-méthyl]-phényl-méthylique de l'acide acétique ;
ester 1-(2-acétoxy-éthyl)-6-[acétoxy-(4-fluoro-phényl)-méthyl]-5-hydroxy-4-oxo-1,4-dihydro-pyridin-2-yl-méthylique de l'acide acétique ;
2-(4-fluorobenzyl)-3-hydroxy-1-(2-morpholin-4-yl-éthyl)-1H-pyridin-4-one ;
acide 4-[2-(4-fluorobenzyl)--3-hydroxy-4-oxo-4H-pyridin-1-ylméthyl]-benzoïque ;
ester [1-(2-acétoxy-éthyl)-3-hydroxy-4-oxo-1,4-dihydropyridin-2-yl-]-(2-fluorophényl)-méthylique de l'acide acétique ; et
N-(2-hydroxy-éthyl)-2-[3-hydroxy-2-(hydroxy-phényl-méthyl)-4-oxo-4H-pyridin-1-yl]-acétamide ;

et leurs stéréoisomères, tautomères et sels compatibles d'un point de vue pharmaceutique.

**7.** Préparations pharmaceutiques contenant un composé selon l'une des revendications 1 à 6 et au moins un matériau excipient utilisable d'un point de vue pharmaceutique.

**8.** Composé selon l'une des revendications 1 à 6, pour utilisation dans un procédé destiné au traitement thérapeutique du corps animal ou humain.

**9.** Composé selon l'une des revendications 1 à 6 pour utilisation lors du traitement de maladies qui provoquent un excès de fer dans le corps humain ou animal, ou qui sont provoquées par cet excès.

**10.** Utilisation d'un composé selon l'une des revendications 1 à 6 pour préparer des préparations pharmaceutiques.

**11.** Utilisation d'un composé selon l'une des revendications 1 à 6 pour préparer des préparations pharmaceutiques destinées au traitement d'un excès de fer dans le corps humain ou animal.

**12.** Procédé de préparation d'un composé de formule I selon la revendication 1, de stéréoisomères, de tautomères et de sels compatibles d'un point de vue pharmaceutique de ce composé, **caractérisé en ce qu'**on fait réagir un composé de formule II

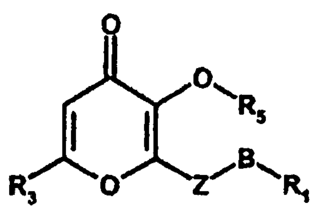

(II)

dans laquelle $R_1$, $R_3$ et B sont tels que définis à propos de la formule I, Z est un radical méthylène non substitué ou substitué par des substituants alkyle inférieur, halogéno ou hydroxy, l'adjectif "inférieur" étant tel que défini dans la revendication 1, et où les substituants peuvent éventuellement se présenter sous forme protégée, ou encore est un groupe carbonyle, et $R_5$ est égal à $R_4$ tel que défini à propos de la formule I, ou représente éventuellement un groupe protecteur approprié, avec un composé de formule III

$$H_2N\text{-}R_2 \qquad (III)$$

dans laquelle $R_2$ est tel que défini à propos de la formule I, pour obtenir un composé de formule IV

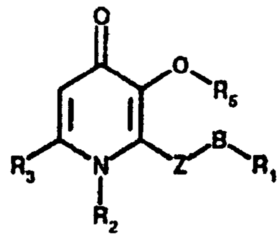

(IV)

dans laquelle $R_1$, $R_2$, $R_3$, $R_5$, B et Z sont tels que définis à propos des formules I et II, et,

a) si nécessaire, on convertit ce composé par dissociation simultanée d'un groupe protecteur $R_5$ et d'un groupe protecteur éventuellement présent sur le groupe Z pour obtenir un composé de formule I et, si on le souhaite, on le convertit en un autre composé de formule I, et/ou, si on le souhaite, on convertit un sel ainsi obtenu en son composé libre ou en un autre sel, et/ou, si on le souhaite, on convertit un composé libre ainsi obtenu de formule I présentant des propriétés de salification en un sel, ou bien
b) si nécessaire, on convertit ce composé, après dissociation d'un groupe protecteur $R_5$ ou d'un groupe protecteur éventuellement présent sur le groupe Z, en une autre forme protégée d'un composé de formule I et, si on le souhaite, on le convertit en une forme protégée d'un autre composé de formule I, puis, par dissociation des groupes protecteurs restants, on le convertit en un composé de formule I et, si on le souhaite, on le convertit en un autre composé de formule I et/ou, si on le souhaite, on convertit un sel ainsi obtenu en son composé libre ou en un autre sel et/ou, si on le souhaite, on convertit en un sel un composé libre ainsi obtenu de formule I présentant des propriétés de salification.